# EUROPEAN PATENT APPLICATION

(11) **EP 4 574 827 A1**
(43) Date of publication of application: **25.06.2025**
(21) Application number: 23881984.1
(22) Date of filing: 27.10.2023
(51) Int. Cl.: C07D 487/04, A61P 25/00, A61K 31/397, A61K 31/40, A61K 31/495

(54) **NITROGEN-CONTAINING HETEROCYCLIC COMPOUNDS AND PHARMACEUTICALLY ACCEPTABLE SALTS THEREOF, PREPARATION METHOD THEREFOR, AND THE USE THEREOF**

(30) Priority: 28.10.2022 CN 202211339268; 26.12.2022 CN 202211686868; 08.02.2023 CN 202310099794; 01.03.2023 CN 202310186197; 17.03.2023 CN 202310263463; 16.06.2023 CN 202310719553; 23.10.2023 CN 202311378324
(71) Applicant: Neushen Therapeutics (Shanghai) Co., Ltd, Shanghai 201210 (CN)
(72) Inventor: CHEN, Deheng, Shanghai 201210 (CN); JIA, Haifei, Shanghai 201210 (CN); SHEN, Huaqiong, Shanghai 201210 (CN); ZHAO, Lele, Shanghai 201210 (CN)
(74) Representative: Murgitroyd & Company
(86) International application number: PCT/CN2023/127300
(87) International publication number: WO 2024/088409

(57) **Abstract**

Disclosed in the present invention are nitrogen-containing heterocyclic compounds and pharmaceutically acceptable salts thereof, a preparation method therefor, and the use thereof. Provided in the present invention are compounds represented by formula I or pharmaceutically acceptable salts thereof. The compounds of the present invention can be used as positive allosteric modulators of muscarinic receptors. The compounds of the present invention can be used for treatment of M receptor-mediated (or M receptor-related) diseases.

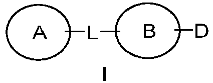

## Description

The present application claims the right of the priority of Chinese patent application No. 202211339268.9 filed on October 28, 2022. The contents of the above Chinese patent application are incorporated herein by reference in its entirety.

The present application claims the right of the priority of Chinese patent application No. 202211686868.2 filed on December 26, 2022. The contents of the above Chinese patent application are incorporated herein by reference in its entirety.

The present application claims the right of the priority of Chinese patent application No. 202310099794.0 filed on February 08, 2023. The contents of the above Chinese patent application are incorporated herein by reference in its entirety.

The present application claims the right of the priority of Chinese patent application No. 202310186197.1 filed on March 01, 2023. The contents of the above Chinese patent application are incorporated herein by reference in its entirety.

The present application claims the right of the priority of Chinese patent application No. 202310263463.6 filed on March 17, 2023. The contents of the above Chinese patent application are incorporated herein by reference in its entirety.

The present application claims the right of the priority of Chinese patent application No. 202310719553.1 filed on June 16, 2023. The contents of the above Chinese patent application are incorporated herein by reference in its entirety.

The present application claims the right of the priority of Chinese patent application No. 202311378324.4 filed on October 23, 2023. The contents of the above Chinese patent application are incorporated herein by reference in its entirety.

### TECHNICAL FIELD

The present disclosure relates to a nitrogen-containing heterocyclic compound, a pharmaceutically acceptable salt thereof, a preparation method therefor, and a use thereof.

### BACKGROUND

Muscarinic receptor (M receptor) belongs to G protein-coupled receptors and is one of acetylcholine receptors. There are five subtypes of M receptors, wherein M1, M3, and M5 are coupled to a Gq protein, activating phospholipase C and increasing intracellular calcium levels. M2 and M4 are coupled to a Gi protein, inhibiting adenylate cyclase and reducing cAMP levels. M1 receptors are distributed in the central nervous system, digestive tract, and lymphoid tissues, which can simultaneously regulate cell excitability and cholinergic transmission as main subtypes of M receptors. M4 receptors are mainly located in the cortex, hippocampus, and striatum, which play an important role in controlling dopamine release and motor activity, but do not modulate important peripheral physiological functions (Neuropharmacology 2018, 136, 362).

The use of M receptor agonists as therapeutic agents for mental diseases has been extensively studied by numerous research institutions and pharmaceutical research and development companies. For example, the M1/M4 receptor agonist Xanomeline underwent a phase II clinical trial for the treatment of Alzheimer's disease in the 1990s, which showed an improvement in the cognitive function of patients treated with the drug, but with serious toxic and side effects in the peripheral nervous system and digestive tract. KarXT, consisting of Xanomeline and the M1 receptor antagonist trospium chloride, is a drug developed by Karuna for the treatment of schizophrenia. Recent phase II clinical studies have indicated a significant improvement in the Positive and Negative Syndrome Scale (PANSS) scores in the KarXT treatment group compared to the placebo group, achieving the primary endpoint, but with peripheral cholinergic side effects (N Engl J Med 2021, 384, 717).

A positive allosteric modulator (PAM) of M receptors is one of the research hotspots in the field of mental diseases in recent years. In a genetic mouse model of schizophrenia, the M1 receptor positive allosteric modulator TAK-071 significantly ameliorates the deficits in memory cognition, social competence, and sensory-motor gating in mice (Neurosci Lett 2021, 764, 136240). The compound is undergoing a phase II clinical trial for the treatment of Parkinsonism. Another M1 receptor positive allosteric modulator MK-7622 is undergoing a phase II clinical trial to assess its efficacy for the treatment of Alzheimer's disease (ACS Med Chem Lett 2018, 9, 652). In a phase Ib clinical trial involving patients with schizophrenia, the M4 receptor positive allosteric modulator CVL-231, developed by Cerevel, was able to significantly reduce the PANSS total score in patients, while common adverse reactions were similar to those in the placebo group, and no extrapyramidal adverse reactions were reported. Compared to traditional M receptor orthosteric agonists, positive allosteric modulators pose lower potential risks to both the peripheral and central nervous systems, and may result in fewer toxic and side effects while maintaining efficacy. Therefore, acting on allosteric modulation pockets is a new direction for targeting M receptors to treat mental diseases. M receptor positive allosteric modulators with good druggability, *in vivo* efficacy, and safety profiles have great development value and market prospect.

### CONTENT OF THE PRESENT INVENTION

The present disclosure provides a nitrogen-containing heterocyclic compound, a pharmaceutically acceptable salt thereof, a preparation method therefor, and a use thereof. The compound of the present disclosure can be used as a positive allosteric modulator of a muscarinic receptor. The compound of the present disclosure can be used for the treatment of an M receptor-mediated (or M receptor-related) disease.

The present disclosure provides a compound of formula I or a pharmaceutically acceptable salt thereof:
wherein 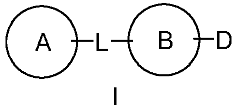 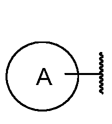 is 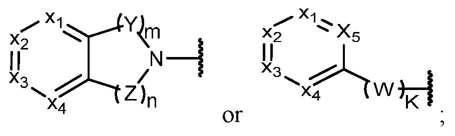
m is a natural number of 0 to 3;
n is a natural number of 0 to 3; m and n are not simultaneously 0;
k is a natural number of 0 to 3;
X₁, X₂, X₃, X₄, and X₅ are independently -CR¹-, N, O, S, 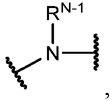 or a chemical bond;
R^{N-1} is hydrogen, C₁-C₆ alkyl, C₁-C₆ alkoxy, or 3- to 7-membered cycloalkyl, wherein the C₁-C₆ alkyl and C₁-C₆ alkoxy are optionally and independently substituted by 1, 2, 3, or 4 R^{N-2};
each R^{N-2} is independently halogen;
Y and Z are independently carbonyl (CO), -(CR²R³)ᵣ-, or a chemical bond; r is a natural number of 0 to 5;
each W is independently carbonyl (CO), -O-, -(CR⁴R⁵)-, -NR⁶-, or a chemical bond;
each R¹ is independently hydrogen, halogen, cyano, hydroxyl, C₁-C₆ alkyl, C₁-C₆ alkoxy, C₁-C₆ alkylthio, 3- to 7-membered cycloalkyl, 3- to 7-membered heterocycloalkyl, or -NR¹⁻¹R¹⁻², wherein the C₁-C₆ alkylthio, C₁-C₆ alkyl, and C₁-C₆ alkoxy are optionally and independently substituted by 1, 2, 3, or 4 R^{a}; or two R¹ together with the atom to which they are attached form a 3- to 7-membered cycloalkyl group or a 3- to 7-membered heterocycloalkyl group;
each R^{a} is independently halogen, cyano, hydroxyl, C₁-C₃ alkyl, C₁-C₆ alkoxy, or - NR¹⁻⁴R¹⁻⁵; or two R^{a} together with the atom to which they are attached form a 3- to 7-membered cycloalkyl group or a 3- to 7-membered heterocycloalkyl group;
R¹⁻¹ and R¹⁻² are independently hydrogen or C₁-C₆ alkyl, wherein the C₁-C₆ alkyl is optionally and independently substituted by 1, 2, 3, or 4 R^{b}; or R¹⁻¹ and R¹⁻² together with the atom to which they are attached form a 3- to 7-membered heterocycloalkyl group, wherein the 3- to 7-membered heterocycloalkyl group is optionally substituted by 1, 2, 3, or 4 R^{b-2};
each R^{b} is independently halogen, cyano, hydroxyl, C₁-C₆ alkoxy, 3- to 7-membered cycloalkyl, 3- to 7-membered heterocycloalkyl, C₁-C₃ alkyl, or NR¹⁻¹⁻¹R¹⁻²⁻¹, wherein the 3- to 7-membered cycloalkyl, 3- to 7-membered heterocycloalkyl, and C₁-C₃ alkyl are optionally and independently substituted by 1, 2, 3, or 4 R^{b-1};
each R^{b-1} is independently halogen, hydroxyl, or cyano;
each R^{b-2} is independently halogen, C₁-C₆ alkyl, or cyano;
R¹⁻⁴ and R¹⁻⁵ are independently hydrogen or C₁-C₃ alkyl, wherein the C₁-C₃ alkyl is optionally and independently substituted by 1, 2, 3, or 4 R¹⁻⁴⁻¹; or R¹⁻⁴ and R¹⁻⁵ together with the atom to which they are attached form a 3- to 7-membered heterocycloalkyl group;
R¹⁻¹⁻¹ and R¹⁻²⁻¹ are independently hydrogen or C₁-C₃ alkyl, wherein the C₁-C₃ alkyl is optionally and independently substituted by 1, 2, 3, or 4 R¹⁻¹⁻¹⁻¹;
R¹⁻⁴⁻¹ and R¹⁻¹⁻¹⁻¹ are each independently halogen, hydroxyl, or cyano;
R² and R³ are independently hydrogen, halogen, cyano, hydroxyl, C₁-C₆ alkoxy, or NR²⁻¹R²⁻²; or R² and R³ together with the atom to which they are attached form a 3- to 7-membered cycloalkyl group or a 3- to 7-membered heterocycloalkyl group;
R²⁻¹ and R²⁻² are independently hydrogen or C₁-C₆ alkyl, wherein the C₁-C₆ alkyl is optionally and independently substituted by 1, 2, 3, or 4 R^{d}; or R²⁻¹ and R²⁻² together with the atom to which they are attached form a 3- to 7-membered heterocycloalkyl group;
each R^{d} is independently halogen, cyano, hydroxyl, C₁-C₆ alkoxy, 3- to 7-membered cycloalkyl, 3- to 7-membered heterocycloalkyl, or C₁-C₃ alkyl;
R⁴, R⁵, and R⁶ are independently hydrogen, halogen, cyano, hydroxyl, C₁-C₆ alkoxy, C₁-C₆ alkyl, or NR⁴⁻¹R⁴⁻², wherein the C₁-C₆ alkyl is optionally and independently substituted by 1, 2, 3, or 4 R^{e};
each R^{e} is independently halogen, cyano, hydroxyl, C₁-C₃ alkyl, C₁-C₃ alkoxy, or - NR⁴⁻⁴R⁴⁻⁵;
R⁴⁻¹ and R⁴⁻² are independently hydrogen or C₁-C₆ alkyl, wherein the C₁-C₆ alkyl is optionally and independently substituted by 1, 2, 3, or 4 R^{f}; or R⁴⁻¹ and R⁴⁻² together with the atom to which they are attached form a 3- to 7-membered heterocycloalkyl group;
each R^{f} is independently halogen, cyano, hydroxyl, 3- to 7-membered cycloalkyl, 3-to 7-membered heterocycloalkyl, C₁-C₃ alkyl, C₁-C₃ alkoxy, or -NR⁴⁻¹⁻¹R⁴⁻²⁻¹;
R⁴⁻⁴ and R⁴⁻⁵ are independently hydrogen or C₁-C₃ alkyl; or R⁴ and R⁵ together with the atom to which they are attached form a 3- to 7-membered cycloalkyl group or a 3- to 7-membered heterocycloalkyl group;
R⁴⁻¹⁻¹ and R⁴⁻²⁻ are independently hydrogen or C₁-C₃ alkyl;
L is 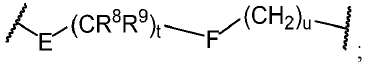
t is a natural number of 0 to 3;
u is a natural number of 0 to 3;
E is carbonyl, 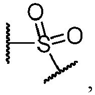 -NHCO-, or a chemical bond;
F is carbonyl, 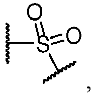 -O-, -NH-, or a chemical bond;
R⁸ and R⁹ are independently hydrogen, halogen, cyano, hydroxyl, or C₁-C₆ alkyl, wherein the C₁-C₆ alkyl is optionally and independently substituted by 1, 2, 3, or 4 R^{g}; or R⁸ and R⁹ together with the atom to which they are attached form a 3- to 7-membered cycloalkyl group or a 3- to 7-membered heterocycloalkyl group;
each R^{g} is independently halogen, cyano, hydroxyl, C₁-C₃ alkyl, or C₁-C₃ alkoxy;
B is 3- to 7-membered cycloalkyl, 4- to 6-membered heterocycloalkyl, 6- to 10-membered aryl, or 5- to 12-membered heteroaryl; wherein the 3- to 7-membered cycloalkyl, 4-to 6-membered heterocycloalkyl, 6- to 10-membered aryl, and 5- to 12-membered heteroaryl are optionally and independently substituted by 1, 2, or 3 Rⁱ;
each Rⁱ is independently hydrogen, halogen, hydroxyl, C₁-C₃ alkyl, 3- to 7-membered cycloalkyl, 3- to 7-membered heterocycloalkyl, cyano, -NR¹¹⁻¹R¹¹⁻², -OR¹¹⁻³, or -SR¹¹⁻⁴; wherein the C₁-C₃ alkyl is optionally and independently substituted by 1, 2, 3 or 4 Rⁱ⁻¹, or two Rⁱ on the same atom together with the atom to which they are attached form a 3- to 7-membered cycloalkyl group or a 3- to 7-membered heterocycloalkyl group; or two adjacent Rⁱ together with the atom to which they are attached form a 3- to 7-membered cycloalkyl group or a 3- to 7-membered heterocycloalkyl group (wherein the 3- to 7-membered cycloalkyl group or the 3-to 7-membered heterocycloalkyl group together with B forms a fused ring);
each Rⁱ⁻¹ is independently C₁-C₃ alkyl, halogen, cyano, or hydroxyl;
R¹¹⁻¹ and R¹¹⁻² are independently hydrogen, C₁-C₃ alkyl, 3- to 7-membered cycloalkyl, or 3- to 7-membered heterocycloalkyl; or R¹¹⁻¹ and R¹¹⁻² together with the atom to which they are attached form a 3- to 7-membered cycloalkyl group or a 3- to 7-membered heterocycloalkyl group;
R¹¹⁻³ and R¹¹⁻⁴ are independently C₁-C₃ alkyl, 3- to 7-membered cycloalkyl, or 3- to 7-membered heterocycloalkyl;
in R¹¹⁻¹, R¹¹⁻², R¹¹⁻³, and R¹¹⁻⁴, the C₁-C₃ alkyl, 3- to 7-membered cycloalkyl, and 3- to 7-membered heterocycloalkyl are optionally and independently substituted by 1, 2, or 3 Rⁱ⁻²;
each Rⁱ⁻² is independently C₁-C₃ alkyl, 3- to 7-membered cycloalkyl, 3- to 7-membered heterocycloalkyl, halogen, cyano, or hydroxyl;
D is hydrogen, halogen, cyano, hydroxyl, C₁-C₆ alkyl, 3- to 7-membered cycloalkyl, 3- to 7-membered heterocycloalkyl, 6- to 10-membered aryl, or 5- to 12-membered heteroaryl; wherein the C₁-C₆ alkyl, 3- to 7-membered cycloalkyl, 3- to 7-membered heterocycloalkyl, 6-to 10-membered aryl, and 5- to 12-membered heteroaryl are optionally and independently substituted by 1, 2, or 3 R^{j};
each R^{j} is independently hydrogen, halogen, cyano, hydroxyl, C₁-C₃ alkyl, 3- to 7-membered cycloalkyl, 3- to 7-membered heterocycloalkyl, -NR¹²⁻¹R¹²⁻², -OR¹²⁻³, or -SR¹²⁻⁴; wherein the C₁-C₃ alkyl, 3- to 7-membered cycloalkyl, and 3- to 7-membered heterocycloalkyl are optionally and independently substituted by 1, 2, or 3 R^{k};
R¹²⁻¹, R¹²⁻², R¹²⁻³, and R¹²⁻⁴ are independently hydrogen, C₁-C₃ alkyl, 3- to 7-membered cycloalkyl, or 3- to 7-membered heterocycloalkyl; wherein the C₁-C₃ alkyl, 3- to 7-membered cycloalkyl, and 3- to 7-membered heterocycloalkyl are optionally and independently substituted by 1, 2, or 3 R^{k-1}; or R¹²⁻¹ and R¹²⁻² together with the atom to which they are attached form a 3- to 7-membered heterocycloalkyl group;
each R^{k} is independently halogen, cyano, hydroxyl, C₁-C₃ alkyl, 3- to 7-membered cycloalkyl, or 3- to 7-membered heterocycloalkyl;
each R^{k-1} is independently halogen, cyano, hydroxyl, C₁-C₃ alkyl, 3- to 7-membered cycloalkyl, or 3- to 7-membered heterocycloalkyl;
when A is 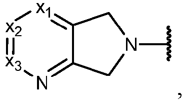 and X₁, X₂, and X₃ are CR¹, then the compound of formula I satisfies any one of the following conditions:
   (1) E is -NHCO- or a chemical bond, and F is -O-, -NH-, or a chemical bond,
   (2) E is carbonyl, and F is -NH-,
   (3) L is -(CH₂)-, -(CH₂)₂-, 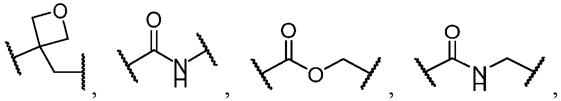 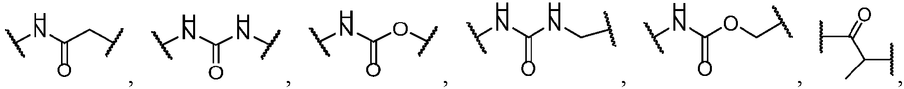 or 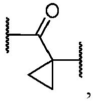
   (4) when E or F is carbonyl, B is 4- to 7-membered cycloalkyl, 6- to 10-membered aryl, 5- to 12-membered heteroaryl, or 4- to 6-membered heterocycloalkyl substituted by 1, 2, or 3 Rⁱ, wherein two Rⁱ on the same atom together with the atom to which they are attached form a 3- to 7-membered cycloalkyl group or a 3- to 7-membered heterocycloalkyl group; or two adjacent Rⁱ together with the atom to which they are attached form a 3- to 7-membered cycloalkyl group or a 3- to 7-membered heterocycloalkyl group (wherein the 3- to 7-membered cycloalkyl group or the 3- to 7-membered heterocycloalkyl group together with B forms a fused ring); wherein the 4- to 7-membered cycloalkyl, 6- to 10-membered aryl, and 5- to 12-membered heteroaryl are optionally and independently substituted by 1, 2, or 3 Rⁱ,
   (5) 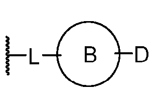 is 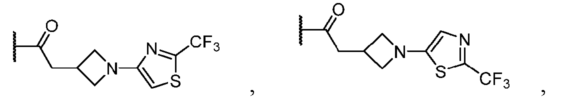 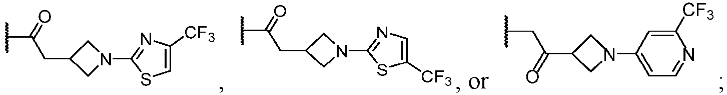
when A is 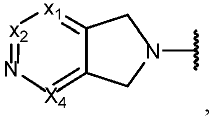 the number of heteroatoms in X₁, X₂, and X₄ is 1 or 2;
when A is 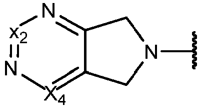 and L is carbonyl, then u is a natural number of 1 to 3, B is 4- to 6-membered heterocycloalkyl, and the 4- to 6-membered heterocycloalkyl is optionally and independently substituted by 1, 2, or 3 Rⁱ;
the heteroatom in the 3- to 7-membered heterocycloalkyl, 4- to 6-membered heterocycloalkyl, and 5- to 12-membered heteroaryl is one or more kinds of N, O, and S, and the number of heteroatoms is 1 to 5.

In a certain embodiment, in the compound of formula I or the pharmaceutically acceptable salt thereof, certain groups are defined as follows, while groups not mentioned are defined as described in any one of the embodiments of the present disclosure (hereinafter referred to in this section as "in a certain embodiment", "in some embodiments", "in a particular embodiment", or "in a preferred embodiment").

In a certain embodiment, 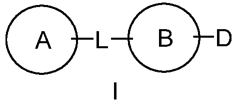
wherein 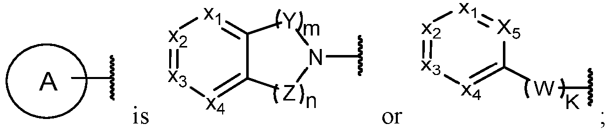
m is a natural number of 0 to 3;
n is a natural number of 0 to 3; m and n are not simultaneously 0;
k is a natural number of 0 to 3;
X₁, X₂, X₃, X₄, and X₅ are independently -CR¹-, N, O, S, 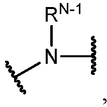 or a chemical bond;
R^{N-1} is hydrogen, C₁-C₆ alkyl, C₁-C₆ alkoxy, or 3- to 7-membered cycloalkyl, wherein the C₁-C₆ alkyl and C₁-C₆ alkoxy are optionally and independently substituted by 1, 2, 3, or 4 R^{N-2};
each R^{N-2} is independently halogen;
Y and Z are independently carbonyl (CO), -(CR²R³)ᵣ-, or a chemical bond; r is a natural number of 0 to 5;
each W is independently carbonyl (CO), -O-, -(CR⁴R⁵)-, -NR⁶-, or a chemical bond;
each R¹ is independently hydrogen, halogen, cyano, hydroxyl, C₁-C₆ alkyl, C₁-C₆ alkoxy, 3- to 7-membered cycloalkyl, 3- to 7-membered heterocycloalkyl, or -NR¹⁻¹R¹⁻², wherein the C₁-C₆ alkyl and C₁-C₆ alkoxy are optionally and independently substituted by 1, 2, 3, or 4 R^{a}; or two R¹ together with the atom to which they are attached form a 3- to 7-membered cycloalkyl group or a 3- to 7-membered heterocycloalkyl group;
each R^{a} is independently halogen, cyano, hydroxyl, C₁-C₃ alkyl, C₁-C₆ alkoxy, or - NR¹⁻⁴R¹⁻⁵; or two R^{a} together with the atom to which they are attached form a 3- to 7-membered cycloalkyl group or a 3- to 7-membered heterocycloalkyl group; R¹⁻¹ and R¹⁻² are independently hydrogen or C₁-C₆ alkyl, wherein the C₁-C₆ alkyl is optionally and independently substituted by 1, 2, 3, or 4 R^{b}; or R¹⁻¹ and R¹⁻² together with the atom to which they are attached form a 3-to 7-membered heterocycloalkyl group;
each R^{b} is independently halogen, cyano, hydroxyl, C₁-C₆ alkoxy, 3- to 7-membered cycloalkyl, 3- to 7-membered heterocycloalkyl, C₁-C₃ alkyl, or NR¹⁻¹⁻¹R¹⁻²⁻¹, wherein the 3- to 7-membered cycloalkyl, 3- to 7-membered heterocycloalkyl, and C₁-C₃ alkyl are optionally and independently substituted by 1, 2, 3, or 4 R^{b-1};
each R^{b-1} is independently halogen, hydroxyl, or cyano;
R¹⁻⁴ and R¹⁻⁵ are independently hydrogen or C₁-C₃ alkyl, wherein the C₁-C₃ alkyl is optionally and independently substituted by 1, 2, 3, or 4 R¹⁻⁴⁻¹; or R¹⁻⁴ and R¹⁻⁵ together with the atom to which they are attached form a 3- to 7-membered heterocycloalkyl group;
R¹⁻¹⁻¹ and R¹⁻²⁻¹ are independently hydrogen or C₁-C₃ alkyl, wherein the C₁-C₃ alkyl is optionally and independently substituted by 1, 2, 3, or 4 R¹⁻¹⁻¹⁻¹;
R¹⁻⁴⁻¹ and R¹⁻¹⁻¹⁻¹ are each independently halogen, hydroxyl, or cyano;
R² and R³ are independently hydrogen, halogen, cyano, hydroxyl, C₁-C₆ alkoxy, or NR²⁻¹R²⁻²; or R² and R³ together with the atom to which they are attached form a 3- to 7-membered cycloalkyl group or a 3- to 7-membered heterocycloalkyl group;
R²⁻¹ and R²⁻² are independently hydrogen or C₁-C₆ alkyl, wherein the C₁-C₆ alkyl is optionally and independently substituted by 1, 2, 3, or 4 R^{d}; or R²⁻¹ and R²⁻² together with the atom to which they are attached form a 3- to 7-membered heterocycloalkyl group;
each R^{d} is independently halogen, cyano, hydroxyl, C₁-C₆ alkoxy, 3- to 7-membered cycloalkyl, 3- to 7-membered heterocycloalkyl, or C₁-C₃ alkyl;
R⁴, R⁵, and R⁶ are independently hydrogen, halogen, cyano, hydroxyl, C₁-C₆ alkoxy, C₁-C₆ alkyl, or NR⁴⁻¹R⁴⁻², wherein the C₁-C₆ alkyl is optionally and independently substituted by 1, 2, 3, or 4 R^{e};
each R^{e} is independently halogen, cyano, hydroxyl, C₁-C₃ alkyl, C₁-C₃ alkoxy, or - NR⁴⁻⁴R⁴⁻⁵;
R⁴⁻¹ and R⁴⁻² are independently hydrogen or C₁-C₆ alkyl, wherein the C₁-C₆ alkyl is optionally and independently substituted by 1, 2, 3, or 4 R^{f}; or R⁴⁻¹ and R⁴⁻² together with the atom to which they are attached form a 3- to 7-membered heterocycloalkyl group;
each R^{f} is independently halogen, cyano, hydroxyl, 3- to 7-membered cycloalkyl, 3-to 7-membered heterocycloalkyl, C₁-C₃ alkyl, C₁-C₃ alkoxy, or -NR⁴⁻¹⁻¹R⁴⁻²⁻¹;
R⁴⁻⁴ and R⁴⁻⁵ are independently hydrogen or C₁-C₃ alkyl; or R⁴ and R⁵ together with the atom to which they are attached form a 3- to 7-membered cycloalkyl group or a 3- to 7-membered heterocycloalkyl group;
R⁴⁻¹⁻¹ and R⁴⁻²⁻ are independently hydrogen or C₁-C₃ alkyl;
L is 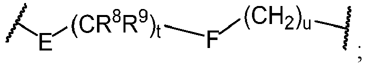
t is a natural number of 0 to 3;
u is a natural number of 0 to 3;
E is carbonyl, -NHCO-, or a chemical bond;
F is carbonyl, -O-, -NH-, or a chemical bond;
R⁸ and R⁹ are independently hydrogen, halogen, cyano, hydroxyl, or C₁-C₆ alkyl, wherein the C₁-C₆ alkyl is optionally and independently substituted by 1, 2, 3, or 4 R^{g}; or R⁸ and R⁹ together with the atom to which they are attached form a 3- to 7-membered cycloalkyl group or a 3- to 7-membered heterocycloalkyl group;
each R^{g} is independently halogen, cyano, hydroxyl, C₁-C₃ alkyl, or C₁-C₃ alkoxy;
B is 3- to 7-membered cycloalkyl, 4- to 6-membered heterocycloalkyl, 6- to 10-membered aryl, or 5- to 12-membered heteroaryl; wherein the 3- to 7-membered cycloalkyl, 4-to 6-membered heterocycloalkyl, 6- to 10-membered aryl, and 5- to 12-membered heteroaryl are optionally and independently substituted by 1, 2, or 3 Rⁱ;
each Rⁱ is independently hydrogen, halogen, hydroxyl, C₁-C₃ alkyl, 3- to 7-membered cycloalkyl, 3- to 7-membered heterocycloalkyl, cyano, -NR¹¹⁻¹R¹¹⁻², -OR¹¹-³, or -SR¹¹⁻⁴; wherein the C₁-C₃ alkyl is optionally and independently substituted by 1, 2, 3 or 4 Rⁱ⁻¹, or two Rⁱ on the same atom together with the atom to which they are attached form a 3- to 7-membered cycloalkyl group or a 3- to 7-membered heterocycloalkyl group; or two adjacent Rⁱ together with the atom to which they are attached form a 3- to 7-membered cycloalkyl group or a 3- to 7-membered heterocycloalkyl group (wherein the 3- to 7-membered cycloalkyl group or the 3-to 7-membered heterocycloalkyl group together with B forms a fused ring);
each Rⁱ⁻¹ is independently C₁-C₃ alkyl, halogen, cyano, or hydroxyl;
R¹¹⁻¹ and R¹¹⁻² are independently hydrogen, C₁-C₃ alkyl, 3- to 7-membered cycloalkyl, or 3- to 7-membered heterocycloalkyl; or R¹¹⁻¹ and R¹¹⁻² together with the atom to which they are attached form a 3- to 7-membered cycloalkyl group or a 3- to 7-membered heterocycloalkyl group;
R¹¹⁻³ and R¹¹⁻⁴ are independently C₁-C₃ alkyl, 3- to 7-membered cycloalkyl, or 3- to 7-membered heterocycloalkyl;
in R¹¹⁻¹, R¹¹⁻², R¹¹⁻³, and R¹¹⁻⁴, the C₁-C₃ alkyl, 3- to 7-membered cycloalkyl, and 3- to 7-membered heterocycloalkyl are optionally and independently substituted by 1, 2, or 3 Rⁱ⁻²;
each Rⁱ⁻² is independently C₁-C₃ alkyl, 3- to 7-membered cycloalkyl, 3- to 7-membered heterocycloalkyl, halogen, cyano, or hydroxyl;
D is hydrogen, halogen, cyano, hydroxyl, C₁-C₆ alkyl, 3- to 7-membered cycloalkyl, 3- to 7-membered heterocycloalkyl, 6- to 10-membered aryl, or 5- to 12-membered heteroaryl; wherein the C₁-C₆ alkyl, 3- to 7-membered cycloalkyl, 3- to 7-membered heterocycloalkyl, 6-to 10-membered aryl, and 5- to 12-membered heteroaryl are optionally and independently substituted by 1, 2, or 3 R^{j};
each R^{j} is independently hydrogen, halogen, cyano, hydroxyl, C₁-C₃ alkyl, 3- to 7-membered cycloalkyl, 3- to 7-membered heterocycloalkyl, -NR¹²⁻¹R¹²⁻², -OR¹²⁻³, or -SR¹²⁻⁴; wherein the C₁-C₃ alkyl, 3- to 7-membered cycloalkyl, and 3- to 7-membered heterocycloalkyl are optionally and independently substituted by 1, 2, or 3 R^{k};
R¹²⁻¹, R¹²⁻², R¹²⁻³, and R¹²⁻⁴ are independently hydrogen, C₁-C₃ alkyl, 3- to 7-membered cycloalkyl, or 3- to 7-membered heterocycloalkyl; wherein the C₁-C₃ alkyl, 3- to 7-membered cycloalkyl, and 3- to 7-membered heterocycloalkyl are optionally and independently substituted by 1, 2, or 3 R^{k-1}; or R¹²⁻¹ and R¹²⁻² together with the atom to which they are attached form a 3- to 7-membered heterocycloalkyl group;
each R^{k} is independently halogen, cyano, hydroxyl, C₁-C₃ alkyl, 3- to 7-membered cycloalkyl, or 3- to 7-membered heterocycloalkyl;
each R^{k-1} is independently halogen, cyano, hydroxyl, C₁-C₃ alkyl, 3- to 7-membered cycloalkyl, or 3- to 7-membered heterocycloalkyl;
when A is 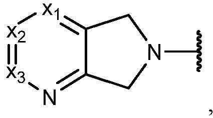 and X₁, X₂, and X₃ are CR¹, then the compound of formula I satisfies any one of the following conditions:
   (1) E is -NHCO- or a chemical bond, and F is -O-, -NH-, or a chemical bond,
   (2) E is carbonyl, and F is -NH-,
   (3) L is -(CH₂)-, -(CH₂)₂-, 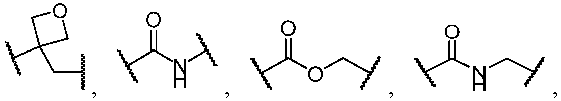 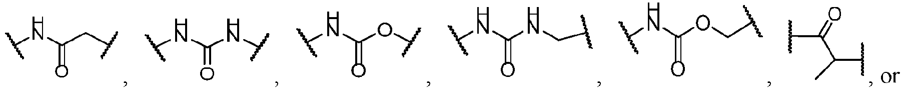 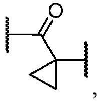
   (4) when E or F is carbonyl, B is 4- to 7-membered cycloalkyl, 6- to 10-membered aryl, 5- to 12-membered heteroaryl, or 4- to 6-membered heterocycloalkyl substituted by 1, 2, or 3 Rⁱ, wherein two Rⁱ on the same atom together with the atom to which they are attached form a 3- to 7-membered cycloalkyl group or a 3- to 7-membered heterocycloalkyl group; or two adjacent Rⁱ together with the atom to which they are attached form a 3- to 7-membered cycloalkyl group or a 3- to 7-membered heterocycloalkyl group (wherein the 3- to 7-membered cycloalkyl group or the 3- to 7-membered heterocycloalkyl group together with B forms a fused ring); wherein the 4- to 7-membered cycloalkyl, 6- to 10-membered aryl, and 5- to 12-membered heteroaryl are optionally and independently substituted by 1, 2, or 3 Rⁱ, 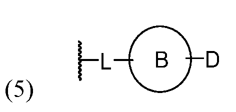
   is 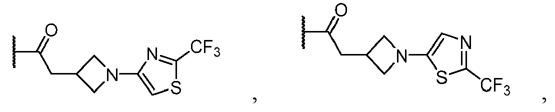 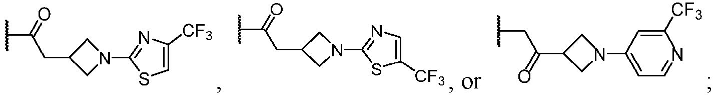
when A is 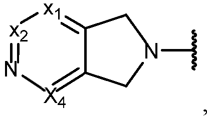 the number of heteroatoms in X₁, X₂, and X₄ is 1 or 2;
when A is 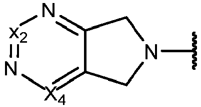 and L is carbonyl, then u is a natural number of 1 to 3, B is 4- to 6-membered heterocycloalkyl, and the 4- to 6-membered heterocycloalkyl is optionally and independently substituted by 1, 2, or 3 Rⁱ;
the heteroatom in the 3- to 7-membered heterocycloalkyl, 4- to 6-membered heterocycloalkyl, and 5- to 12-membered heteroaryl is N, O, or S, and the number of heteroatoms is 1 to 5; or the heteroatom in the 3- to 7-membered heterocycloalkyl, 4- to 6-membered heterocycloalkyl, and 5- to 12-membered heteroaryl is one or more kinds of N, O, and S, and the number of heteroatoms is 1 to 5.

In a certain embodiment, m is preferably a natural number of 0 to 3, such as 0, 1, 2, or 3; for another example, 1, 2, or 3.

In a certain embodiment, n is preferably a natural number of 1 to 3, such as 1, 2, or 3; for another example, 1 or 2.

In a certain embodiment, n is 1.

In a certain embodiment, when X₁ is N, and X₂, X₃, and X₄ are -CR¹-, then Y and Z are independently -(CH₂)-, m and n are 2, and R^{j} is trifluoromethyl.

In a certain embodiment,

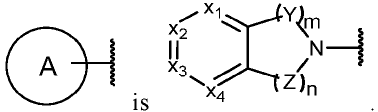

In a certain embodiment, 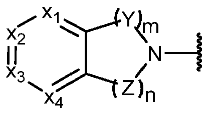 is

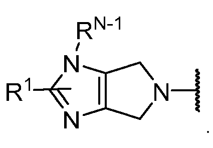

In a certain embodiment, m is 1.

In a certain embodiment, k is preferably a natural number of 0 to 3, such as 0, 1, 2, or 3; for another example, 1, 2, or 3.

In a certain embodiment, r is preferably a natural number of 0 to 5, such as 0, 1, 2, 3, 4, or 5; for another example, 1 or 2.

In a certain embodiment, r is 1.

In a certain embodiment, r is a natural number of 0 to 3, such as 1 or 2.

In a certain embodiment, R^{N-1} is C₁-C₆ alkyl.

In a certain embodiment, R^{N-1} is hydrogen, C₁-C₆ alkyl, or 3- to 7-membered cycloalkyl.

In a certain embodiment, each W is independently -(CR⁴R⁵)-, -O-, -NR⁶-, or a chemical bond.

In a certain embodiment, each W is independently -(CR⁴R⁵)-, -NR⁶-, or a chemical bond.

In a certain embodiment, Y and Z are independently carbonyl (CO) or -(CR²R³)ᵣ-.

In a certain embodiment, Y and Z are independently -(CR²R³)ᵣ-.

In a certain embodiment, each R¹ is independently hydrogen, halogen, C₁-C₆ alkyl, C₁-C₆ alkoxy, C₁-C₆ alkylthio, 3- to 7-membered cycloalkyl, 3- to 7-membered heterocycloalkyl, or -NR¹⁻¹R¹⁻².

In a certain embodiment, each R¹ is independently C₁-C₆ alkyl, C₁-C₆ alkoxy, C₁-C₆ alkylthio, or -NR¹⁻¹R¹⁻².

In a certain embodiment, each R¹ is independently hydrogen, C₁-C₆ alkyl, C₁-C₆ alkoxy, C₁-C₆ alkylthio, 3- to 7-membered cycloalkyl, 3- to 7-membered heterocycloalkyl, or -NR¹⁻¹R¹⁻²; for example, C₁-C₆ alkyl, C₁-C₆ alkoxy, C₁-C₆ alkylthio, or -NR¹⁻¹R¹⁻².

In a certain embodiment, each R¹ is independently hydrogen, halogen, C₁-C₆ alkyl, C₁-C₆ alkoxy, C₁-C₆ alkylthio, 3- to 7-membered cycloalkyl, 3- to 7-membered heterocycloalkyl, or -NR¹⁻¹R¹⁻², wherein the C₁-C₆ alkylthio, C₁-C₆ alkyl, and C₁-C₆ alkoxy are optionally and independently substituted by 1, 2, 3, or 4 R^{a}.

In a certain embodiment, each R¹ is independently hydrogen, halogen, C₁-C₆ alkyl, C₁-C₆ alkoxy, 3- to 7-membered cycloalkyl, 3- to 7-membered heterocycloalkyl, or -NR¹⁻¹R¹⁻².

In a certain embodiment, each R¹ is independently C₁-C₆ alkyl.

In a certain embodiment, each R¹ is independently C₁-C₆ alkyl or C₁-C₆ alkoxy.

In a certain embodiment, R¹⁻¹ and R¹⁻² are independently hydrogen or C₁-C₆ alkyl; or R¹⁻¹ and R¹⁻² together with the atom to which they are attached form a 3- to 7-membered heterocycloalkyl group; for example, R¹⁻¹ and R¹⁻² together with the atom to which they are attached form a 3- to 7-membered heterocycloalkyl group.

In a certain embodiment, when X₁ and X₃ are independently N, and X₄ and X₂ are independently -CR¹-, then each R¹ is independently C₁-C₆ alkyl, C₁-C₆ alkoxy, C₁-C₆ alkylthio, 3- to 7-membered heterocycloalkyl, or -NR¹⁻¹R¹⁻².

In a certain embodiment, when X₂ is independently -CR¹-, R¹ (at X₂) is C₁-C₆ alkyl or C₁-C₆ alkoxy.

In a certain embodiment, when D is pyrimidyl, each R¹ is independently halogen or 3- to 7-membered cycloalkyl.

In a certain embodiment, when X₂ and X₃ are independently N, and X₄ and X₁ are independently -CR¹-, then each R¹ is independently C₁-C₆ alkyl or 3- to 7-membered heterocycloalkyl.

In a certain embodiment, each R^{a} is independently hydroxyl, C₁-C₃ alkoxy, or NR¹⁻⁴R¹⁻⁵.

In a certain embodiment, each R^{a} is independently halogen, hydroxyl, C₁-C₆ alkoxy, or -NR¹⁻⁴R¹⁻⁵; for example, each R^{a} is independently hydroxyl, C₁-C₆ alkoxy, or -NR¹⁻⁴R¹⁻⁵; for another example, hydroxyl.

In a certain embodiment, each R^{b} is independently hydroxyl, 3- to 7-membered cycloalkyl, 3- to 7-membered heterocycloalkyl, C₁-C₃ alkyl, C₁-C₃ alkoxy, or NR¹⁻¹⁻¹R¹⁻²⁻¹

In a certain embodiment, each R¹ is independently hydroxyl, 3- to 7-membered cycloalkyl, or C₁-C₃ alkyl; for example, each R^{b} is independently hydroxyl or 3- to 7-membered cycloalkyl.

In a certain embodiment, each R^{b} is independently 3- to 7-membered cycloalkyl.

In a certain embodiment, R^{b-1} is independently hydroxyl.

In a certain embodiment, each R^{b-1} is independently hydroxyl.

In a certain embodiment, each R^{b-2} is independently halogen or C₁-C₆ alkyl.

In a certain embodiment, R¹⁻⁴ and R¹⁻⁵ are independently C₁-C₃ alkyl.

In a certain embodiment, each R¹⁻⁴⁻¹ is independently halogen.

In a certain embodiment, R¹⁻⁴⁻¹ is halogen.

In a certain embodiment, R² and R³ are independently hydrogen or NR²⁻¹R²⁻².

In a certain embodiment, R² and R³ are independently hydrogen.

In a certain embodiment, R²⁻¹ and R²⁻² are independently hydrogen or C₁-C₆ alkyl; for example, hydrogen.

In a certain embodiment, R⁴, R⁵, and R⁶ are independently hydrogen.

In a certain embodiment, R⁴, R⁵, and R⁶ are independently hydrogen or NR⁴⁻¹R⁴⁻².

In a certain embodiment, R⁴⁻¹ and R⁴⁻² are independently hydrogen.

In a certain embodiment, Rⁱ is -NR⁴⁻⁴R⁴⁻⁵.

In a certain embodiment, R⁴⁻⁴ and R⁴⁻⁵ are independently hydrogen or C₁-C₃ alkyl.

In a certain embodiment, E is carbonyl, 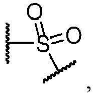 -NHCO-, or a chemical bond.

In a certain embodiment, E is carbonyl or a chemical bond.

In a certain embodiment, E is carbonyl.

In a certain embodiment, t is 0.

In a certain embodiment, F is -NH-, -O-, or a chemical bond.

In a certain embodiment, F is carbonyl, 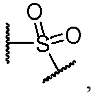 -O-, -NH-, or a chemical bond.

In a certain embodiment, F is a chemical bond.

In a certain embodiment, R⁸ and R⁹ are independently hydrogen or C₁-C₆ alkyl; or R⁸ and R⁹ together with the atom to which they are attached form a 3- to 7-membered cycloalkyl group or a 3- to 7-membered heterocycloalkyl group.

In a certain embodiment, R⁸ and R⁹ are independently hydrogen or C₁-C₆ alkyl; or R⁸ and R⁹ together with the atom to which they are attached form a 3- to 7-membered cycloalkyl group.

In a certain embodiment, R⁸ and R⁹ are independently C₁-C₆ alkyl; or R⁸ and R⁹ together with the atom to which they are attached form a 3- to 7-membered cycloalkyl group or a 3- to 7-membered heterocycloalkyl group.

In a certain embodiment, B is 4- to 6-membered heterocycloalkyl, 6- to 10-membered aryl, or 5- to 12-membered heteroaryl, wherein the 5- to 12-membered heteroaryl, 6- to 10-membered aryl, and 4- to 6-membered heterocycloalkyl are optionally and independently substituted by 1, 2, or 3 Rⁱ.

In a certain embodiment, B is 4- to 6-membered heterocycloalkyl or 5- to 12-membered heteroaryl, wherein the 5- to 12-membered heteroaryl and 4- to 6-membered heterocycloalkyl are optionally and independently substituted by 1, 2, or 3 Rⁱ.

In a certain embodiment, B is 4- to 6-membered heterocycloalkyl, wherein the 4- to 6-membered heterocycloalkyl is optionally and independently substituted by 1, 2, or 3 Rⁱ.

In a certain embodiment, when B is 6- to 10-membered aryl or 5- to 12-membered heteroaryl, D is hydrogen, halogen, cyano, hydroxyl, C₁-C₆ alkyl, 3- to 7-membered cycloalkyl, or 3- to 7-membered heterocycloalkyl, wherein the C₁-C₆ alkyl, 3- to 7-membered cycloalkyl, and 3- to 7-membered heterocycloalkyl are optionally and independently substituted by 1, 2, or 3 R^{j}; preferably, D is hydrogen or C₁-C₆ alkyl.

In a certain embodiment, when X₂ and X₃ are independently N, and X₄ and X₁ are independently -CR¹-, then B is azetidinyl (e.g., 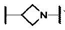 ).

In a certain embodiment, when X₁ and X₃ (or X₂ and X₄) are -CR¹-, and R¹ is C₁-C₆ alkyl, then each R^{j} is independently -OR¹²⁻³ or -SR¹²⁻⁴.

In a certain embodiment, Rⁱ is hydrogen, halogen, hydroxyl, or C₁-C₃ alkyl; or two Rⁱ together with the atom to which they are attached form a 3- to 7-membered cycloalkyl group or a 3- to 7-membered heterocycloalkyl group.

In a certain embodiment, each Rⁱ is independently hydrogen, halogen, hydroxyl, or C₁-C₃ alkyl; or two Rⁱ together with the atom to which they are attached form a 3- to 7-membered cycloalkyl group; for example, Rⁱ is hydrogen or C₁-C₃ alkyl, such as C₁-C₃ alkyl. In a certain embodiment, Rⁱ is hydrogen.

In a certain embodiment, D is hydrogen, C₁-C₆ alkyl, or 5- to 12-membered heteroaryl; wherein the C₁-C₆ alkyl and 5- to 12-membered heteroaryl are optionally and independently substituted by 1, 2, or 3 R^{j}.

In a certain embodiment, D is hydrogen, 3- to 7-membered cycloalkyl, C₁-C₆ alkyl, or 5- to 6-membered heteroaryl; wherein the C₁-C₆ alkyl and 5- to 6-membered heteroaryl are optionally and independently substituted by 1, 2, or 3 R^{j}.

In a certain embodiment, D is 5- to 12-membered heteroaryl; wherein the 5- to 12-membered heteroaryl is optionally and independently substituted by 1, 2, or 3 R^{j}.

In a certain embodiment, D is 5- to 6-membered heteroaryl; wherein the 5- to 6-membered heteroaryl is optionally and independently substituted by 1, 2, or 3 R^{j}.

In a certain embodiment, when X₂ and X₃ are independently N, and X₄ and X₁ are independently -CR¹-, then D is 6-membered heteroaryl (*e*.*g*., 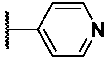 ); wherein the 6-membered heteroaryl is optionally and independently substituted by 1, 2, or 3 R^{j}.

In a certain embodiment, when D is 5-membered heteroaryl, the 5-membered heteroaryl is 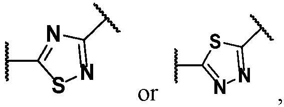 and each R¹ is independently C₁-C₆ alkyl or C₁-C₆ alkoxy.

In a certain embodiment, R^{j} is halogen, C₁-C₃ alkyl, OR¹²⁻³, or SR¹²⁻⁴.

In a certain embodiment, each R^{j} is independently C₁-C₃ alkyl, OR¹²⁻³, or SR¹²⁻⁴, and the C₁-C₃ alkyl is optionally and independently substituted by 1, 2, or 3 R^{k}; preferably, each R^{j} is independently OR¹²⁻³ or SR¹²⁻⁴.

In a certain embodiment, each R^{j} is independently halogen, C₁-C₃ alkyl, OR¹²⁻³, or SR¹²⁻⁴.

In a certain embodiment, each R^{j} is independently hydrogen, halogen, C₁-C₃ alkyl, 3-to 7-membered cycloalkyl, -OR¹²⁻³, or -SR¹²⁻⁴.

In a certain embodiment, each R^{j} is independently hydrogen, halogen, C₁-C₃ alkyl, 3-to 7-membered cycloalkyl, -OR¹²⁻³, or -SR¹²⁻⁴; the C₁-C₃ alkyl and 3- to 7-membered cycloalkyl are optionally and independently substituted by 1, 2, or 3 R^{k}.

In a certain embodiment, R^{j} is OR¹²⁻³.

In a certain embodiment, each R^{j} is independently OR¹²⁻³.

In a certain embodiment, R^{j} is halogen or C₁-C₃ alkyl.

In a certain embodiment, each R^{j} is independently halogen or C₁-C₃ alkyl.

In a certain embodiment, when X₁ and X₃ are independently N, X₄ and X₂ are independently -CR¹-, and each R¹ is independently C₁-C₆ alkyl or C₁-C₆ alkoxy, then each R^{j} is independently halogen, 3- to 7-membered cycloalkyl, OCH₂CF₃, 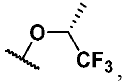 or SR¹²⁻⁴.

In a certain embodiment, when 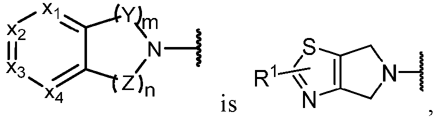 each R^{j} is independently OR¹²⁻³ or SR¹²⁻⁴.

In a certain embodiment, when X₁ or X₄ is -CR¹-, and R¹ is independently 3- to 7-membered heterocycloalkyl or -NR¹⁻¹R¹⁻², then each R^{j} is independently OR¹²⁻³ or SR¹²⁻⁴.

In a certain embodiment, R¹²⁻³ and R¹²⁻⁴ are independently C₁-C₃ alkyl.

In a certain embodiment, R¹²⁻³ and R¹²⁻⁴ are independently C₁-C₃ alkyl; wherein the C₁-C₃ alkyl is optionally and independently substituted by 1, 2, or 3 R^{k-1}.

In a certain embodiment, R¹²⁻¹, R¹²⁻², R¹²⁻³, and R¹²⁻⁴ are independently C₁-C₃ alkyl.

In a certain embodiment, each R^{k} is independently halogen.

In a certain embodiment, R^{k} is independently halogen.

In a certain embodiment, each R^{k-1} is independently halogen.

In a certain embodiment, R^{k-1} is independently halogen.

In a certain embodiment, the heteroatom in the 3- to 7-membered heterocycloalkyl, 4-to 6-membered heterocycloalkyl, and 5- to 12-membered heteroaryl is N, O, or S, and the number of heteroatoms is 1 to 3.

In a certain embodiment, the heteroatom in the 3- to 7-membered heterocycloalkyl, 4-to 6-membered heterocycloalkyl, and 5- to 12-membered heteroaryl is N, O, or S, and the number of heteroatoms is 1 to 5.

In a certain embodiment, the heteroatom in the 3- to 7-membered heterocycloalkyl, 4-to 6-membered heterocycloalkyl, 5- to 6-membered heteroaryl, and 5- to 12-membered heteroaryl is N, O, or S, and the number of heteroatoms is 1 to 3.

In a certain embodiment, the heteroatom in the 3- to 7-membered heterocycloalkyl, 4-to 6-membered heterocycloalkyl, 5- to 6-membered heteroaryl, and 5- to 12-membered heteroaryl is selected from one, two, or three kinds of N, O, or S, and the number of heteroatoms is 1, 2, or 3.

In a certain embodiment, the 3- to 7-membered heterocycloalkyl is 3- to 6-membered heterocycloalkyl; the heteroatom is, for example, one or two kinds of N and O, and the number of heteroatoms is, for example, 1 or 2.

In a certain embodiment, the 4- to 6-membered heterocycloalkyl is 4-membered heterocycloalkyl; the heteroatom is, for example, one or two kinds of N and O, and the number of heteroatoms is, for example, 1.

In a certain embodiment, the 5- to 6-membered heteroaryl is 6-membered heteroaryl; the heteroatom is, for example, one or two kinds of N and O, and the number of heteroatoms is, for example, 1.

In a certain embodiment, the 5- to 12-membered heteroaryl is 5- to 10-membered heterocycloalkyl; the heteroatom is, for example, one or two kinds of N and O, and the number of heteroatoms is, for example, 1.

In a certain embodiment, when A is 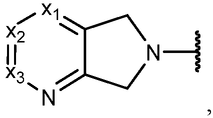 and X₁, X₂, and X₃ are CR¹, then E or F is 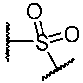 in the compound of formula I.

In a certain embodiment, in R^{N-1}, the C₁-C₆ alkyl is preferably C₁-C₃ alkyl, such as methyl, ethyl, *n*-propyl, or isopropyl; for another example, methyl, ethyl, or isopropyl.

In a certain embodiment, in R^{N-1}, the C₁-C₆ alkoxy is preferably C₁-C₃ alkoxy, such as methoxy, ethoxy, *n*-propoxy, or isopropoxy.

In a certain embodiment, in R^{N-1}, the 3- to 7-membered cycloalkyl is preferably 3- to 6-membered cycloalkyl, such as cyclopropyl, cyclobutyl, or cyclopentyl.

In a certain embodiment, in R^{N-2}, the halogen is F, Cl, Br, or I, such as F or Cl.

In a certain embodiment, in R¹, the halogen is F, Cl, Br, or I, such as F or Cl; for another example, Cl.

In a certain embodiment, in R¹, the C₁-C₆ alkyl is preferably C₁-C₃ alkyl, such as methyl, ethyl, *n*-propyl, or isopropyl; for another example, methyl, ethyl, or isopropyl.

In a certain embodiment, in R¹, the C₁-C₆ alkoxy is preferably C₁-C₃ alkoxy, such as methoxy, ethoxy, *n*-propoxy, or isopropoxy; for another example, methoxy or ethoxy.

In a certain embodiment, in R¹, the C₁-C₆ alkylthio is preferably C₁-C₃ alkylthio, such as methylthio, ethylthio, *n*-propylthio, or isopropylthio; for another example, methylthio.

In a certain embodiment, in R¹, the 3- to 7-membered cycloalkyl is preferably 3- to 6-membered cycloalkyl, such as cyclopropyl, cyclobutyl, or cyclopentyl; for another example, cyclopropyl or cyclobutyl.

In a certain embodiment, in R¹, the 3- to 7-membered heterocycloalkyl is preferably 3- to 6-membered heterocycloalkyl; the heteroatom in the 3- to 6-membered heterocycloalkyl is preferably N or O; the number of heteroatoms in the 3- to 6-membered heterocycloalkyl is preferably 1 or 2; the 3- to 6-membered heterocycloalkyl is more preferably 4-membered heterocycloalkyl, such as

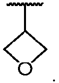

In a certain embodiment, in R¹, the 3- to 7-membered heterocycloalkyl is preferably 3- to 6-membered heterocycloalkyl; the heteroatom in the 3- to 6-membered heterocycloalkyl is preferably N or O; the number of heteroatoms in the 3- to 6-membered heterocycloalkyl is preferably 1 or 2; the 3- to 6-membered heterocycloalkyl is more preferably 4-membered heterocycloalkyl, such as

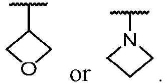

In a certain embodiment, in R^{a}, the halogen is preferably F, Cl, Br, or I, such as F or Cl; for another example, F.

In a certain embodiment, in R^{a}, the C₁-C₃ alkyl is preferably methyl, ethyl, *n*-propyl, or isopropyl.

In a certain embodiment, in R^{a}, the C₁-C₆ alkoxy is preferably C₁-C₃ alkoxy, such as methoxy, ethoxy, *n*-propoxy, or isopropoxy; for another example, methoxy.

In a certain embodiment, in R^{a}, the 3- to 7-membered heterocycloalkyl is preferably 3- to 6-membered heterocycloalkyl; the heteroatom in the 3- to 6-membered heterocycloalkyl is preferably N or O; the number of heteroatoms in the 3- to 6-membered heterocycloalkyl is preferably 1 or 2; the 3- to 6-membered heterocycloalkyl is more preferably 4-membered heterocycloalkyl, such as

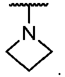

In a certain embodiment, in R^{a}, the 3- to 7-membered cycloalkyl is preferably 3- to 6-membered cycloalkyl, such as cyclopropyl, cyclobutyl, or cyclopentyl.

In a certain embodiment, in R¹⁻¹ and R¹⁻², the C₁-C₆ alkyl is preferably C₁-C₃ alkyl, such as methyl, ethyl, n-propyl, or isopropyl; for another example, methyl or ethyl (*e.g.*, methyl).

In a certain embodiment, in R¹⁻¹ and R¹⁻², the 3- to 7-membered heterocycloalkyl is preferably 3- to 6-membered heterocycloalkyl; the heteroatom in the 3- to 6-membered heterocycloalkyl is preferably N or O; the number of heteroatoms in the 3- to 6-membered heterocycloalkyl is preferably 1 or 2; the 3- to 6-membered heterocycloalkyl is, for example, 4-membered heterocycloalkyl; for another example,

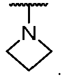

In a certain embodiment, in R^{b}, the halogen is preferably F, Cl, Br, or I, such as F or Cl.

In a certain embodiment, in R^{b}, the C₁-C₆ alkoxy is preferably C₁-C₃ alkoxy, such as methoxy, ethoxy, n-propoxy, or isopropoxy.

In a certain embodiment, in R^{b}, the 3- to 7-membered cycloalkyl is preferably 3- to 6-membered cycloalkyl, such as cyclopropyl, cyclobutyl, or cyclopentyl; for another example, cyclopropyl.

In a certain embodiment, in R^{b}, the 3- to 7-membered heterocycloalkyl is preferably 3- to 6-membered heterocycloalkyl; the heteroatom in the 3- to 6-membered heterocycloalkyl is preferably N or O; the number of heteroatoms in the 3- to 6-membered heterocycloalkyl is preferably 1 or 2.

In a certain embodiment, in R^{b}, the C₁-C₃ alkyl is preferably methyl, ethyl, *n*-propyl, or isopropyl, such as methyl.

In a certain embodiment, in R^{b-1}, the halogen is preferably F, Cl, Br, or I, such as F or Cl.

In a certain embodiment, in R^{b-2}, the halogen is preferably F, Cl, Br, or I, such as F.

In a certain embodiment, in R^{b-2}, the C₁-C₆ alkyl is preferably C₁-C₃ alkyl, such as methyl, ethyl, n-propyl, or isopropyl; for another example, methyl (*e.g*.,

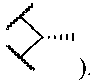

In a certain embodiment, in R¹⁻⁴ and R¹⁻⁵, the C₁-C₃ alkyl is preferably methyl, ethyl, n-propyl, or isopropyl, such as methyl, ethyl, or *n*-propyl; for another example, methyl.

In a certain embodiment, in R¹⁻⁴ and R¹⁵, the 3- to 7-membered heterocycloalkyl is preferably 3- to 6-membered heterocycloalkyl; the heteroatom in the 3- to 6-membered heterocycloalkyl is preferably N or O; the number of heteroatoms in the 3- to 6-membered heterocycloalkyl is preferably 1 or 2; the 3- to 6-membered heterocycloalkyl is more preferably 4-membered heterocycloalkyl, such as

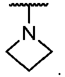

In a certain embodiment, in R¹⁻¹⁻¹ and R¹⁻²⁻¹, the C₁-C₃ alkyl is preferably methyl, ethyl, *n*-propyl, or isopropyl.

In a certain embodiment, in R¹⁻⁴⁻¹ and R¹⁻¹⁻¹⁻¹, the halogen is preferably F, Cl, Br, or I, such as F or Cl; for another example, F.

In a certain embodiment, in R² and R³, the halogen is preferably F, Cl, Br, or I, such as F or Cl.

In a certain embodiment, in R² and R³, the C₁-C₆ alkoxy is preferably C₁-C₃ alkoxy, such as methoxy, ethoxy, n-propoxy, or isopropoxy.

In a certain embodiment, in R² and R³, the 3- to 7-membered cycloalkyl is preferably 3- to 6-membered cycloalkyl, such as cyclopropyl, cyclobutyl, or cyclopentyl.

In a certain embodiment, in R² and R³, the 3- to 7-membered heterocycloalkyl is preferably 3- to 6-membered heterocycloalkyl; the heteroatom in the 3- to 6-membered heterocycloalkyl is preferably N or O; the number of heteroatoms in the 3- to 6-membered heterocycloalkyl is preferably 1 or 2.

In a certain embodiment, in R²⁻¹ and R²⁻², the C₁-C₆ alkyl is preferably C₁-C₃ alkyl, such as methyl, ethyl, *n*-propyl, or isopropyl; for another example, methyl or ethyl.

In a certain embodiment, in R²⁻¹ and R²⁻², the 3- to 7-membered heterocycloalkyl is preferably 3- to 6-membered heterocycloalkyl; the heteroatom in the 3- to 6-membered heterocycloalkyl is preferably N or O; the number of heteroatoms in the 3- to 6-membered heterocycloalkyl is preferably 1 or 2.

In a certain embodiment, in R^{d}, the halogen is preferably F, Cl, Br, or I, such as F or Cl.

In a certain embodiment, in R^{d}, the C₁-C₆ alkoxy is preferably C₁-C₃ alkoxy, such as methoxy, ethoxy, *n*-propoxy, or isopropoxy.

In a certain embodiment, in R^{d}, the 3- to 7-membered cycloalkyl is preferably 3- to 6-membered cycloalkyl, such as cyclopropyl, cyclobutyl, or cyclopentyl.

In a certain embodiment, in R^{d}, the 3- to 7-membered heterocycloalkyl is preferably 3- to 6-membered heterocycloalkyl; the heteroatom in the 3- to 7-membered heterocycloalkyl is preferably N or O; the number of heteroatoms in the 3- to 6-membered heterocycloalkyl is preferably 1 or 2.

In a certain embodiment, in R^{d}, the C₁-C₃ alkyl is preferably methyl, ethyl, n-propyl, or isopropyl.

In a certain embodiment, in R⁴, R⁵, and R⁶, the halogen is preferably F, Cl, Br, or I, such as F or Cl.

In a certain embodiment, in R⁴, R⁵, and R⁶, the C₁-C₆ alkoxy is preferably C₁-C₃ alkoxy, such as methoxy, ethoxy, *n*-propoxy, or isopropoxy.

In a certain embodiment, in R⁴, R⁵, and R⁶, the C₁-C₆ alkyl is preferably C₁-C₃ alkyl, such as methyl, ethyl, *n*-propyl, or isopropyl.

In a certain embodiment, in R^{e}, the halogen is preferably F, Cl, Br, or I, such as F or Cl.

In a certain embodiment, in R^{e}, the C₁-C₃ alkyl is preferably methyl, ethyl, *n*-propyl, or isopropyl.

In a certain embodiment, in R^{e}, the C₁-C₃ alkoxy is preferably methoxy, ethoxy, *n-*propoxy, or isopropoxy.

In a certain embodiment, in R⁴⁻¹ and R⁴⁻², the C₁-C₆ alkyl is preferably C₁-C₃ alkyl, such as methyl, ethyl, *n*-propyl, or isopropyl.

In a certain embodiment, in R⁴⁻¹ and R⁴⁻², the 3- to 7-membered heterocycloalkyl is preferably 3- to 6-membered heterocycloalkyl; the heteroatom in the 3- to 6-membered heterocycloalkyl is preferably N or O; the number of heteroatoms in the 3- to 6-membered heterocycloalkyl is preferably 1 or 2.

In a certain embodiment, in R^{f}, the halogen is preferably F, Cl, Br, or I, such as F or Cl.

In a certain embodiment, in R^{f}, the 3- to 7-membered cycloalkyl is preferably 3- to 6-membered cycloalkyl, such as cyclopropyl, cyclobutyl, or cyclopentyl.

In a certain embodiment, in R^{f}, the 3- to 7-membered heterocycloalkyl is preferably 3- to 6-membered heterocycloalkyl; the heteroatom in the 3- to 6-membered heterocycloalkyl is preferably N or O; the number of heteroatoms in the 3- to 6-membered heterocycloalkyl is preferably 1 or 2.

In a certain embodiment, in R^{f}, the C₁-C₃ alkyl is preferably methyl, ethyl, *n*-propyl, or isopropyl.

In a certain embodiment, in R^{f}, the C₁-C₃ alkoxy is preferably methoxy, ethoxy, *n-*propoxy, or isopropoxy.

In a certain embodiment, in R⁴⁻⁴ and R⁴⁻⁵, the C₁-C₃ alkyl is preferably methyl, ethyl, *n*-propyl, or isopropyl.

In a certain embodiment, t is a natural number of 0 to 3, such as 0, 1, 2, or 3; for another example, 0, 1, or 2, such as 0.

In a certain embodiment, u is a natural number of 0 to 3, such as 0, 1, 2, or 3; for another example, 0, 1, or 2, such as 1.

In a certain embodiment, in R⁸ and R⁹, the halogen is preferably F, Cl, Br, or I, such as F or Cl.

In a certain embodiment, in R⁸ and R⁹, the C₁-C₆ alkyl is preferably C₁-C₃ alkyl, such as methyl, ethyl, *n*-propyl, or isopropyl; for another example, methyl.

In a certain embodiment, in R⁸ and R⁹, the 3- to 7-membered cycloalkyl is preferably 3- to 6-membered cycloalkyl, such as cyclopropyl, cyclobutyl, or cyclopentyl; for another example, cyclopropyl.

In a certain embodiment, in R⁸ and R⁹, the 3- to 7-membered heterocycloalkyl is preferably 3- to 6-membered heterocycloalkyl; the heteroatom in the 3- to 6-membered heterocycloalkyl is preferably N or O; the number of heteroatoms in the 3- to 6-membered heterocycloalkyl is preferably 1 or 2; the 3- to 6-membered heterocycloalkyl is, for example, 4-membered heterocycloalkyl; for another example, oxetanyl.

In a certain embodiment, in R^{g}, the halogen is preferably F, Cl, Br, or I, such as F or Cl.

In a certain embodiment, in R^{g}, the C₁-C₃ alkyl is preferably methyl, ethyl, *n*-propyl, or isopropyl, such as methyl.

In a certain embodiment, in R^{g}, the C₁-C₃ alkoxy is preferably methoxy, ethoxy, *n-*propoxy, or isopropoxy.

In a certain embodiment, in B, the 3- to 7-membered cycloalkyl is preferably 3- to 6-membered cycloalkyl, such as cyclopropyl, cyclobutyl, or cyclopentyl.

In a certain embodiment, in B, the 4- to 7-membered cycloalkyl is preferably 3- to 6-membered cycloalkyl, such as cyclobutyl or cyclopentyl.

In a certain embodiment, in B, the 4- to 6-membered heterocycloalkyl is preferably 4-membered heterocycloalkyl, 5-membered heterocycloalkyl, or 6-membered heterocycloalkyl; the heteroatom in the 4-membered heterocycloalkyl, 5-membered heterocycloalkyl, and 6-membered heterocycloalkyl is preferably N; the number of heteroatoms in the 4- to 6-membered heterocycloalkyl is preferably 1 or 2; for example, the 4- to 6-membered heterocycloalkyl is 4-membered azacycloalkyl (*e.g*., 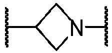 ) or 5-membered azacycloalkyl (*e.g*.,

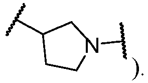

In a certain embodiment, in B, the 6- to 10-membered aryl is preferably phenyl or naphthyl, such as phenyl.

In a certain embodiment, in R^{j}, the halogen is preferably F, Cl, Br, or I, such as F or Cl; for another example, F.

In a certain embodiment, in Rⁱ, the C₁-C₃ alkyl is preferably methyl, ethyl, n-propyl, or isopropyl; for another example, methyl.

In a certain embodiment, in Rⁱ, the 3- to 7-membered cycloalkyl is preferably 3- to 6-membered cycloalkyl, such as cyclopropyl, cyclobutyl, or cyclopentyl; for another example, cyclopropyl or cyclobutyl, such as cyclopropyl.

In a certain embodiment, in Rⁱ, the 3- to 7-membered heterocycloalkyl is preferably 3- to 6-membered heterocycloalkyl; the heteroatom in the 3- to 6-membered heterocycloalkyl is preferably N or O; the number of heteroatoms in the 3- to 6-membered heterocycloalkyl is preferably 1 or 2.

In a certain embodiment, in Rⁱ⁻¹, the halogen is preferably F, Cl, Br, or I, such as F or Cl.

In a certain embodiment, in Rⁱ⁻¹, the C₁-C₃ alkyl is preferably methyl, ethyl, n-propyl, or isopropyl.

In a certain embodiment, in R¹¹⁻¹, R¹¹⁻², R¹¹⁻³, and R¹¹⁻⁴, the C₁-C₃ alkyl is, for example, methyl, ethyl, n-propyl, or isopropyl.

In a certain embodiment, in R¹¹⁻³ and R¹¹⁻⁴, the 3- to 7-membered cycloalkyl may be 3- to 6-membered cycloalkyl, such as cyclopropyl, cyclobutyl, or cyclopentyl.

In a certain embodiment, in R¹¹⁻³ and R¹¹⁻⁴, the 3- to 7-membered heterocycloalkyl is preferably 3- to 6-membered heterocycloalkyl; the heteroatom in the 3- to 6-membered heterocycloalkyl is preferably N or O; the number of heteroatoms in the 3- to 6-membered heterocycloalkyl is preferably 1 or 2.

In a certain embodiment, in R¹¹⁻¹ and R¹¹⁻², the 3- to 7-membered cycloalkyl is preferably 3- to 6-membered cycloalkyl, such as cyclopropyl, cyclobutyl, or cyclopentyl.

In a certain embodiment, in R¹¹⁻¹ and R¹¹⁻², the 3- to 7-membered heterocycloalkyl is preferably 3- to 6-membered heterocycloalkyl; the heteroatom in the 3- to 6-membered heterocycloalkyl is preferably N or O; the number of heteroatoms in the 3- to 6-membered heterocycloalkyl is preferably 1 or 2.

In a certain embodiment, in Rⁱ⁻², the halogen is preferably F, Cl, Br, or I, such as F or Cl.

In a certain embodiment, in Rⁱ⁻², the C₁-C₃ alkyl is preferably methyl, ethyl, n-propyl, or isopropyl.

In a certain embodiment, in Rⁱ⁻², the 3- to 7-membered cycloalkyl is preferably 3- to 6-membered cycloalkyl, such as cyclopropyl, cyclobutyl, or cyclopentyl.

In a certain embodiment, in Rⁱ⁻², the 3- to 7-membered heterocycloalkyl is preferably 3- to 6-membered heterocycloalkyl; the heteroatom in the 3- to 6-membered heterocycloalkyl is preferably N or O; the number of heteroatoms in the 3- to 6-membered heterocycloalkyl is preferably 1 or 2.

In a certain embodiment, in D, the halogen is preferably F, Cl, Br, or I, such as F or Cl.

In a certain embodiment, in D, the C₁-C₆ alkyl is preferably C₁-C₃ alkyl, such as methyl, ethyl, n-propyl, or isopropyl; for another example, methyl.

In a certain embodiment, in D, the 3- to 7-membered cycloalkyl is preferably 3- to 6-membered cycloalkyl, such as cyclopropyl, cyclobutyl, or cyclopentyl, such as cyclopropyl.

In a certain embodiment, in D, the 6- to 10-membered aryl is phenyl or naphthyl.

In a certain embodiment, in R^{j}, the halogen is preferably F, Cl, Br, or I, such as F or Cl; for another example, F.

In a certain embodiment, in R^{j}, the C₁-C₃ alkyl is preferably methyl, ethyl, *n*-propyl, or isopropyl; for another example, methyl, ethyl, or isopropyl, preferably methyl.

In a certain embodiment, in R^{j}, the 3- to 7-membered cycloalkyl is preferably 3- to 6-membered cycloalkyl, such as cyclopropyl, cyclobutyl, or cyclopentyl; for another example, cyclopropyl.

In a certain embodiment, in R^{j}, the 3- to 7-membered heterocycloalkyl is preferably 3- to 6-membered heterocycloalkyl; the heteroatom in the 3- to 6-membered heterocycloalkyl is preferably N or O; the number of heteroatoms in the 3- to 6-membered heterocycloalkyl is preferably 1 or 2.

In a certain embodiment, in R¹²⁻¹, R¹²⁻², R¹²⁻³, and R¹²⁻⁴, the C₁-C₃ alkyl is preferably methyl, ethyl, *n*-propyl, or isopropyl, such as methyl or ethyl; for another example, methyl, ethyl, or isopropyl.

In a certain embodiment, in R¹²⁻¹, R¹²⁻², R¹²⁻³, and R¹²⁻⁴, the 3- to 7-membered cycloalkyl is preferably 3- to 6-membered cycloalkyl, such as cyclopropyl, cyclobutyl, or cyclopentyl.

In a certain embodiment, in R¹²⁻¹, R¹²⁻², R¹²⁻³, and R¹²⁻⁴, the 3- to 7-membered heterocycloalkyl is preferably 3- to 6-membered heterocycloalkyl; the heteroatom in the 3- to 6-membered heterocycloalkyl is preferably N or O; the number of heteroatoms in the 3- to 6-membered heterocycloalkyl is preferably 1 or 2.

In a certain embodiment, in R^{k}, the halogen is preferably F, Cl, Br, or I, such as F or Cl; for another example, F.

In a certain embodiment, in R^{k}, the C₁-C₃ alkyl is preferably methyl, ethyl, *n*-propyl, or isopropyl.

In a certain embodiment, in R^{k}, the 3- to 7-membered cycloalkyl is preferably 3- to 6-membered cycloalkyl, such as cyclopropyl, cyclobutyl, or cyclopentyl.

In a certain embodiment, in R^{k}, the 3- to 7-membered heterocycloalkyl is preferably 3- to 6-membered heterocycloalkyl; the heteroatom in the 3- to 6-membered heterocycloalkyl is preferably N or O; the number of heteroatoms in the 3- to 6-membered heterocycloalkyl is preferably 1 or 2.

In a certain embodiment, in R^{k-1}, the halogen is preferably F, Cl, Br, or I, such as F or Cl; for another example, F.

In a certain embodiment, in R^{k-1}, the C₁-C₃ alkyl is preferably methyl, ethyl, *n*-propyl, or isopropyl, such as methyl.

In a certain embodiment, in R^{k-1}, the 3- to 7-membered cycloalkyl is preferably 3- to 6-membered cycloalkyl, such as cyclopropyl, cyclobutyl, or cyclopentyl.

In a certain embodiment, in R^{k-1}, the 3- to 7-membered heterocycloalkyl is preferably 3- to 6-membered heterocycloalkyl; the heteroatom in the 3- to 6-membered heterocycloalkyl is preferably N or O; the number of heteroatoms in the 3- to 6-membered heterocycloalkyl is preferably 1 or 2.

In some embodiments, the compound of formula I is a compound of formula I-1, a compound of formula I-2, a compound of formula I-3, a compound of formula I-4, or a compound of formula I-5; 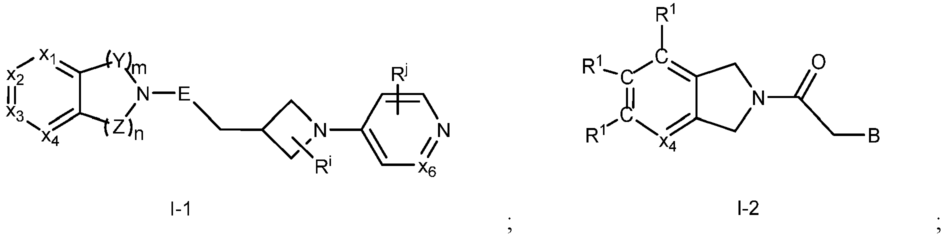 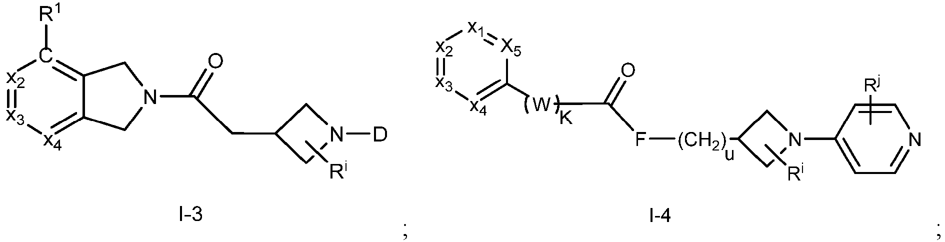 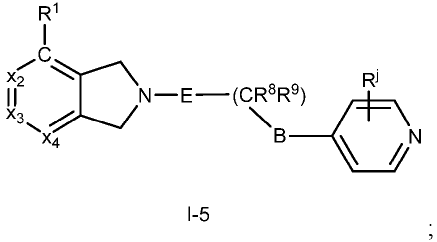 wherein X₆ is N or CH; X₁, Rⁱ, X₂, X₃, X₄, X₅, Y, Z, m, n, E, R^{j}, R¹, B, D, W, k, F, u, R⁸, and R⁹ are as described in any one of the embodiments of the present disclosure.

In some embodiments, the compound of formula I-1 is a compound of formula I-1-1, a compound of formula I-1-2, a compound of formula I-1-3, or a compound of formula 1-1-4; 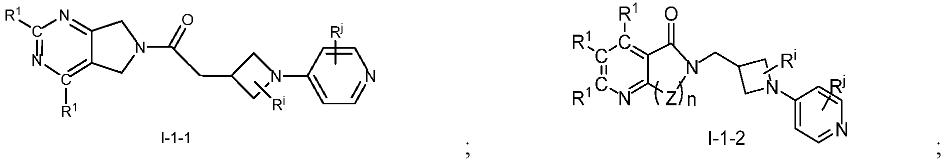 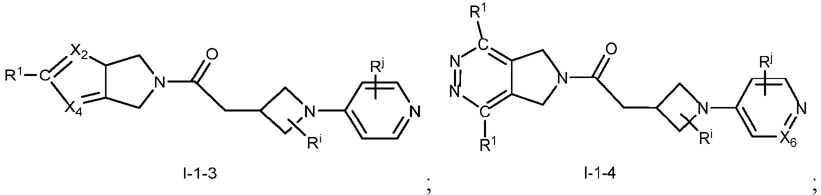 wherein R¹, R^{j}, Z, n, X₆, X₂, and X₄ are as described in any one of the embodiments of the present disclosure.

In some embodiments, the compound of formula I-5 is a compound of formula I-5-1; 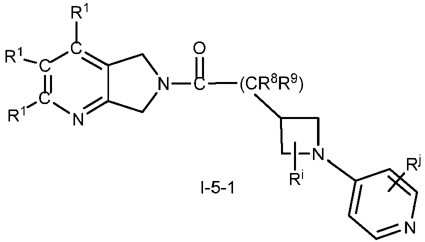 wherein R¹, R⁸, R⁹, and R^{j} are as described in any one of the embodiments of the present disclosure.

In some embodiments, the compound of formula I-4 is a compound of formula I-4-1; 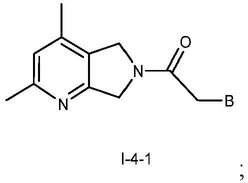 wherein B is as described in any one of the embodiments of the present disclosure.

In a certain embodiment, X₁, X₂, X₃, X₄, and X₅ are independently -CR¹-, N, O, S, 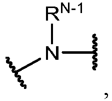 or a chemical bond, and the number of heteroatoms in X₁, X₂, X₃, X₄, and X₅ is 0, 1, 2, or 3.

In a certain embodiment, X₁, X₂, X₃, X₄, and X₅ are independently -CR¹-, N, S, 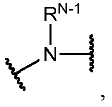 , or a chemical bond, and the number of heteroatoms in X₁, X₂, X₃, X₄, and X₅ is 1 or 2.

In a certain embodiment, X₁ and X₃ are independently N; X₄ and X₂ are independently -CR¹-.

In a certain embodiment, X₂ and X₃ are independently N, X₄ and X₁ are independently -CR¹-.

In a certain embodiment, X₁ and X₄ are independently N; X₂ and X₃ are independently -CR¹-.

In some embodiments, in 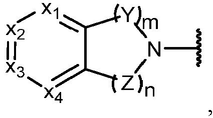 X₁, X₂, X₃, and X₄ are independently -CR¹- or N; the number of heteroatoms is 0, 1, or 2.

In some embodiments, in 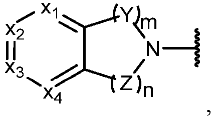 Y and Z are independently carbonyl (CO) or -(CR²R³)ᵣ-; r is 1, and R² and R³ are independently H, -(CH₂)-, -(NHCH₂)-, - (NHCH₂CH₂)-, or

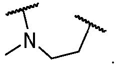

In some embodiments, in 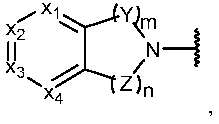 , m is 2, n is 2, and Y and Z are independently -(CR²R³)ᵣ-; r is 1, and R² and R³ are independently H.

In some embodiments, 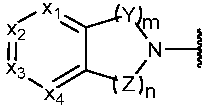 is phenyl-fused 5-membered heterocycloalkyl, wherein the heteroatom in the 5-membered heterocycloalkyl is N, and the number of heteroatoms is 1; for example, 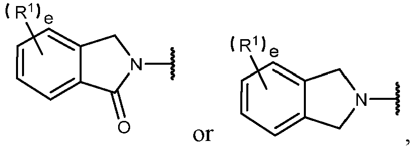 wherein e is independently 1, 2, or 3; for another example,

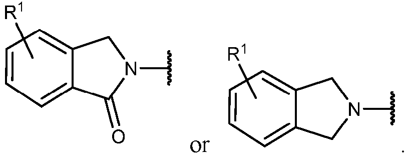

In some embodiments, 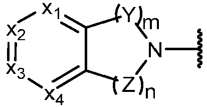 is phenyl-fused 5-membered heterocycloalkyl, wherein the heteroatom in the 5-membered heterocycloalkyl is N, and the number of heteroatoms is 1; for example,

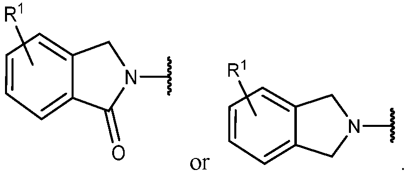

In some embodiments, 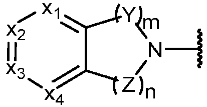 is phenyl-fused 7-membered heterocycloalkyl, wherein the heteroatom in the 7-membered heterocycloalkyl is N, and the number of heteroatoms is 1 or 2; for example, 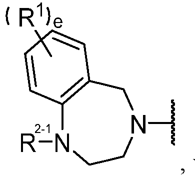 wherein e is independently 0, 1, 2, or 3; for another example,

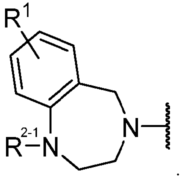

In some embodiments, 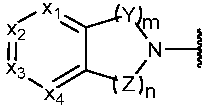 is phenyl-fused 7-membered heterocycloalkyl, wherein the heteroatom in the 7-membered heterocycloalkyl is N, and the number of heteroatoms is 1 or 2; for example,

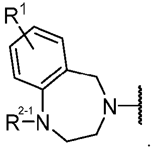

In some embodiments, 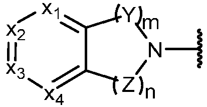 is 6-membered heteroaryl-fused 5-membered heterocycloalkyl, wherein the heteroatom in the 6-membered heteroaryl is N, and the number of heteroatoms is 1 or 2; preferably, the heteroatom in the 5-membered heterocycloalkyl is N, and the number of heteroatoms is 1; for example, 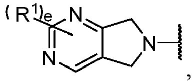 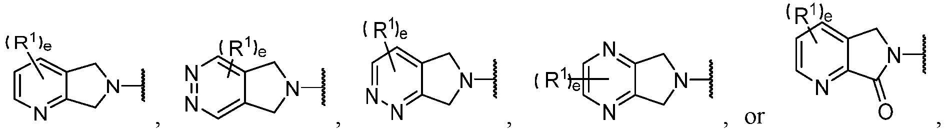 wherein e is independently 0, 1, or 2; for another example,

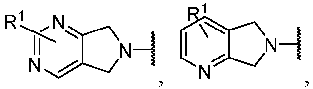

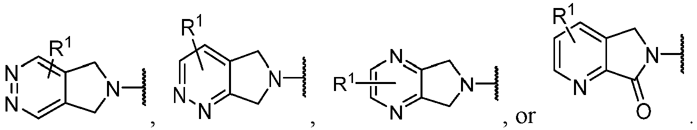

In some embodiments, 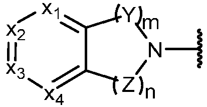 is 6-membered heteroaryl-fused 5-membered heterocycloalkyl, wherein the heteroatom in the 6-membered heteroaryl is N, and the number of heteroatoms is 1 or 2; preferably, the heteroatom in the 5-membered heterocycloalkyl is N, and the number of heteroatoms is 1; for example,

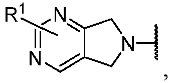

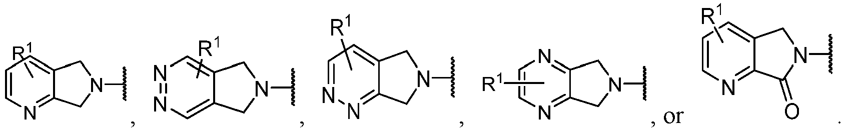

In some embodiments, 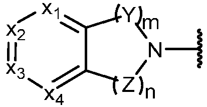 is 6-membered heteroaryl-fused 5-membered heterocycloalkyl, wherein the heteroatom in the 6-membered heteroaryl is N, and the number of heteroatoms is 2; preferably, the heteroatom in the 5-membered heterocycloalkyl is N, and the number of heteroatoms is 1; for example, 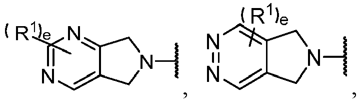 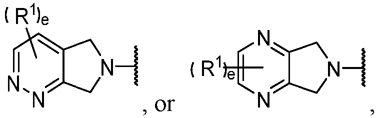 wherein e is independently 0, 1, or 2.

In some embodiments, 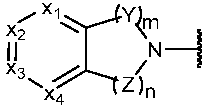 is 6-membered heteroaryl-fused 5-membered heterocycloalkyl, wherein the heteroatom in the 6-membered heteroaryl is N, and the number of heteroatoms is 1; for example, 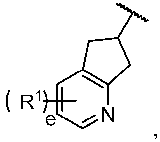 wherein e is independently 0, 1, 2, or 3; for another example,

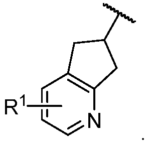

In some embodiments, 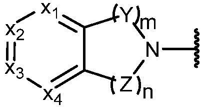 is 6-membered heteroaryl-fused 5-membered heterocycloalkyl, wherein the heteroatom in the 6-membered heteroaryl is N, and the number of heteroatoms is 1; for example,

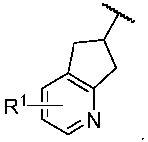

In some embodiments, 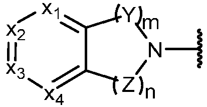 is 6-membered heteroaryl-fused 6-membered heterocycloalkyl, wherein the heteroatom in the 6-membered heteroaryl is N, and the number of heteroatoms is 1; preferably, the heteroatom in the 6-membered heterocycloalkyl is N, and the number of heteroatoms is 1; for example, 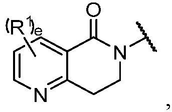 wherein e is independently 0, 1, 2, or 3; for another example,

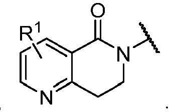

In some embodiments, 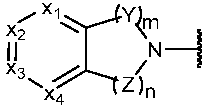 is 6-membered heteroaryl-fused 6-membered heterocycloalkyl, wherein the heteroatom in the 6-membered heteroaryl is N, and the number of heteroatoms is 1; preferably, the heteroatom in the 6-membered heterocycloalkyl is N, and the number of heteroatoms is 1; for example,

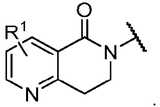

In some embodiments, 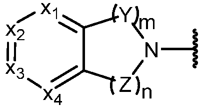 is 6-membered heteroaryl-fused 7-membered heterocycloalkyl, wherein the heteroatom in the 6-membered heteroaryl is N, and the number of heteroatoms is 1 or 2; preferably, the heteroatom in the 7-membered heterocycloalkyl is N, and the number of heteroatoms is 1; for example, 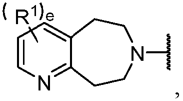 wherein e is independently 0, 1, 2, or 3;

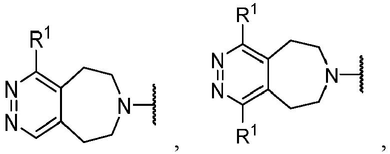

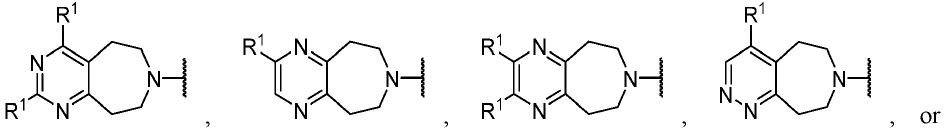

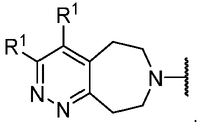

In some embodiments, 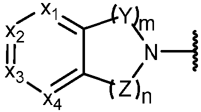 is 6-membered heteroaryl-fused 7-membered heterocycloalkyl, wherein the heteroatom in the 6-membered heteroaryl is N, and the number of heteroatoms is 1 or 2; preferably, the heteroatom in the 7-membered heterocycloalkyl is N, and the number of heteroatoms is 1; for example,

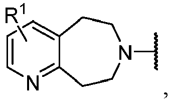

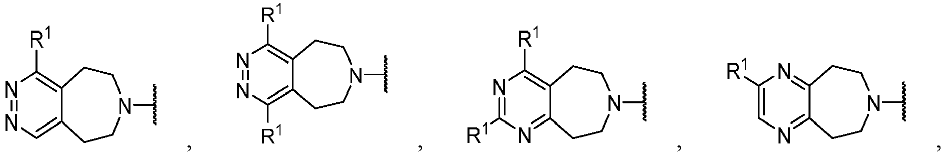

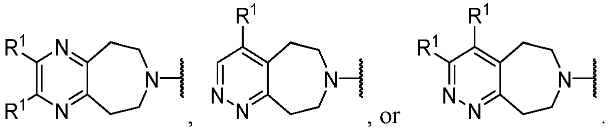

In some embodiments, 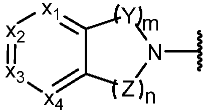 is 5-membered heteroaryl-fused 5-membered heterocycloalkyl, wherein the heteroatom in the 5-membered heteroaryl is N or S, and the number of heteroatoms is 1 or 2; preferably, the heteroatom in the 5-membered heterocycloalkyl is N, and the number of heteroatoms is 1; for example,

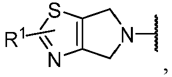

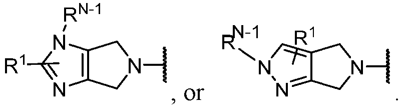

In some embodiments, 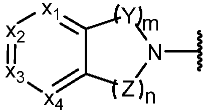 is 5-membered heteroaryl-fused 5-membered heterocycloalkyl, wherein the heteroatom in the 5-membered heteroaryl is N and/or S, and the number of heteroatoms is 1 or 2; preferably, the heteroatom in the 5-membered heterocycloalkyl is N, and the number of heteroatoms is 1; for example,

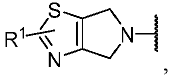

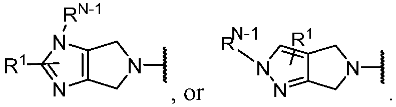

In some embodiments, 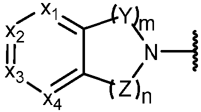 is 5-membered heteroaryl-fused 6-membered heterocycloalkyl, wherein the heteroatom in the 5-membered heteroaryl is N, and the number of heteroatoms is 1 or 2; preferably, the heteroatom in the 6-membered heterocycloalkyl is N, and the number of heteroatoms is 1; for example, 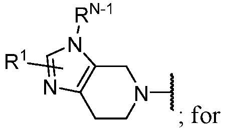 another example,

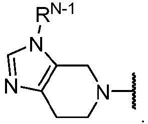

In some embodiments, 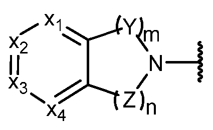 is 5-membered heteroaryl-fused 6-membered heterocycloalkyl, wherein the heteroatom in the 5-membered heteroaryl is N, and the number of heteroatoms is 1; preferably, the heteroatom in the 6-membered heterocycloalkyl is N, and the number of heteroatoms is 1; for example, 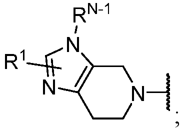 for another example,

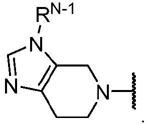

In some embodiments, 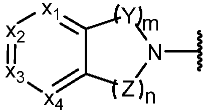 is 5-membered heteroaryl-fused 6-membered heterocycloalkyl, wherein the heteroatom in the 5-membered heteroaryl is N, and the number of heteroatoms is 1; preferably, the heteroatom in the 6-membered heterocycloalkyl is N, and the number of heteroatoms is 1; for example,

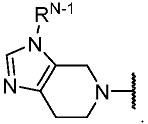

In some embodiments, in 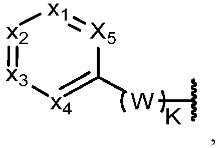 X₁, X₂, X₃, X₄, and X₅ are independently -CR¹-, N, S, 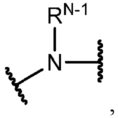 or a chemical bond, and the number of heteroatoms in X₁, X₂, X₃, X₄, and X₅ is 0, 1, or 2.

In some embodiments, 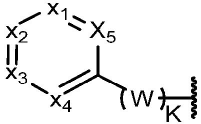 is C-(W)ₖ-, wherein C is 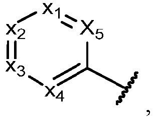 5-membered heteroaryl, 6-membered heteroaryl, or phenyl; preferably, the heteroatom in the 5-membered heteroaryl is N, and the number of heteroatoms is 1 or 2; or the heteroatom in the 6-membered heteroaryl is N, and the number of heteroatoms is 1; for example, the C is pyridyl, phenyl, or

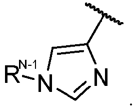

In some embodiments, in 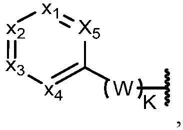 W is -O-, -CH₂-, or -NH-.

In some embodiments, in B, the 5- to 12-membered heteroaryl is preferably 5- to 6- membered heteroaryl, phenyl-fused 5- to 6-membered heteroaryl, 5- to 7-membered cycloalkyl-fused phenyl, 5- to 7-membered cycloalkyl-fused 5- to 6-membered heteroaryl, 5-to 6-membered heteroaryl-fused 5- to 6-membered heteroaryl, 5- to 7-membered heterocycloalkyl-fused 5- to 6-membered heteroaryl, or 5- to 7-membered heterocycloalkyl-fused 5- to 6-membered aryl; further preferably 5- to 6-membered heteroaryl, phenyl-fused 5-to 6-membered heteroaryl, 5- to 6-membered heteroaryl-fused 5- to 6-membered heteroaryl, 5-to 7-membered heterocycloalkyl-fused 5- to 6-membered heteroaryl, or 5- to 7-membered heterocycloalkyl-fused phenyl.

In some embodiments, in B, the heteroatom in the 4- to 6-membered heterocycloalkyl is N, and the number of heteroatoms is 1; for example, 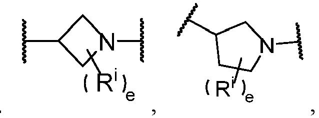 or 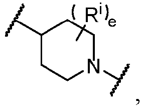 wherein e is independently 0, 1, 2, or 3; for another example,

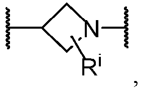

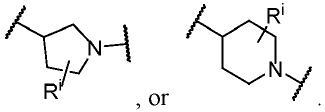

In some embodiments, in B, the heteroatom in the 4- to 6-membered heterocycloalkyl is N, and the number of heteroatoms is 1; for example, 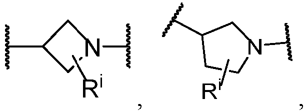 or

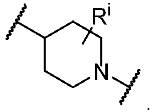

In some embodiments, B is 4- to 6-membered heterocycloalkyl substituted by two Rⁱ; two Rⁱ on the same atom together with the atom to which they are attached form a 3- to 7-membered cycloalkyl group; the 3- to 7-membered cycloalkyl group is preferably a 3- to 6-membered cycloalkyl group; for example, B is 4-membered heterocycloalkyl or 5-membered heterocycloalkyl; two Rⁱ form a 3- to 4-membered cycloalkyl group; for another example, B is

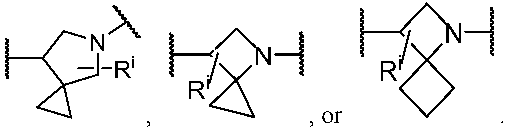

In some embodiments, B is 4- to 6-membered heterocycloalkyl substituted by two Rⁱ; two Rⁱ on the same atom together with the atom to which they are attached form a 3- to 7-membered cycloalkyl group; the 3- to 7-membered cycloalkyl group is preferably a 3- to 6-membered cycloalkyl group; for example, B is 4-membered heterocycloalkyl; two Rⁱ form a 3-to 4-membered cycloalkyl group; for another example, B is

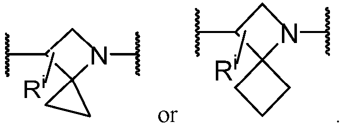

In some embodiments, B is 4- to 6-membered heterocycloalkyl substituted by two Rⁱ; two Rⁱ on the same atom together with the atom to which they are attached form a 3- to 7-membered cycloalkyl group; the 3- to 7-membered cycloalkyl group is preferably a 3- to 6-membered cycloalkyl group; for example, B is 5-membered heterocycloalkyl; two Rⁱ form a 3-to 4-membered cycloalkyl group; for another example, B is

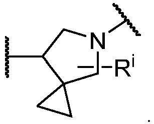

In some embodiments, B is 4- to 6-membered heterocycloalkyl ortho-substituted by two Rⁱ; two adjacent Rⁱ together with the atom to which they are attached form a 3- to 7-membered cycloalkyl group (wherein the 3- to 7-membered cycloalkyl group together with B forms a fused ring); the 3- to 7-membered cycloalkyl group is preferably a 3- to 6-membered cycloalkyl group; for example, B is 5-membered heterocycloalkyl; two Rⁱ together with the atom to which they are attached form a 3-membered cycloalkyl group; for another example, B is

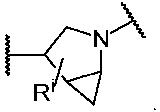

In some embodiments, B is 5-membered heteroaryl, wherein the heteroatom in the 5-membered heteroaryl is N and/or S, and the number of heteroatoms is 1 or 2; for example,

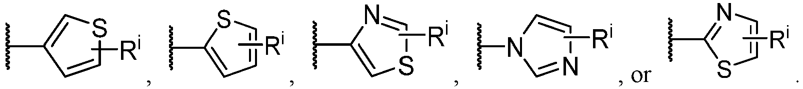

In some embodiments, B is 5-membered heteroaryl, wherein the heteroatom in the 5-membered heteroaryl is N or S, and the number of heteroatoms is 1 or 2; for example,

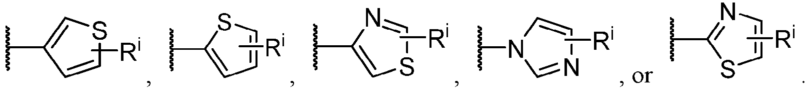

In some embodiments, B is 6-membered heteroaryl, wherein the heteroatom in the 6-membered heteroaryl is N, and the number of heteroatoms is 1 or 2; for example,

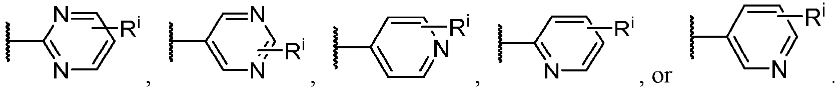

In some embodiments, in B, the 6- to 10-membered aryl is, for example, phenyl.

In some embodiments, B is phenyl-fused 5-membered heterocycloalkyl, wherein the heteroatom in the 5-membered heterocycloalkyl is N, and the number of heteroatoms is 1; for example, 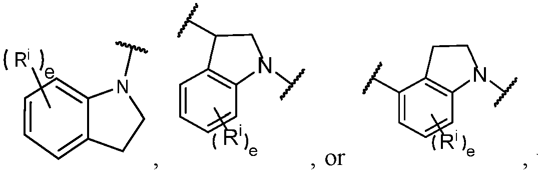 wherein e is independently 0, 1, 2, or 3; for another example,

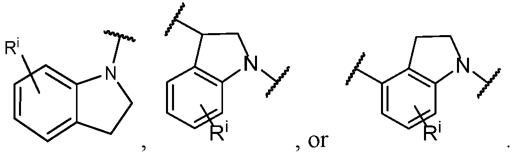

In some embodiments, B is phenyl-fused 5-membered heterocycloalkyl, wherein the heteroatom in the 5-membered heterocycloalkyl is N, and the number of heteroatoms is 1; for example,

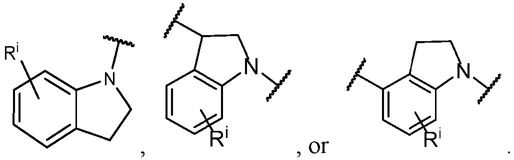

In some embodiments, B is phenyl-fused 6-membered heteroaryl, wherein the heteroatom in the 6-membered heteroaryl is N, and the number of heteroatoms is 1 or 2; for example,

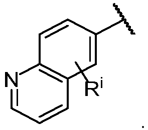

In some embodiments, B is phenyl-fused 6-membered heterocycloalkyl, wherein the heteroatom in the 6-membered heterocycloalkyl is N and/or O, and the number of heteroatoms is 1 or 2; for example, 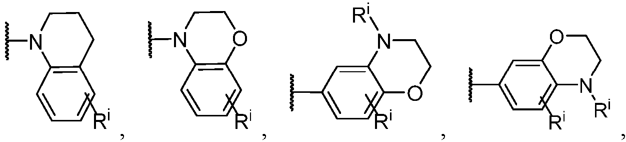 or

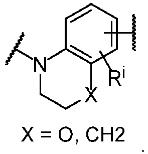

In some embodiments, B is phenyl-fused 6-membered heterocycloalkyl, wherein the heteroatom in the 6-membered heterocycloalkyl is N or O, and the number of heteroatoms is 1 or 2; for example, 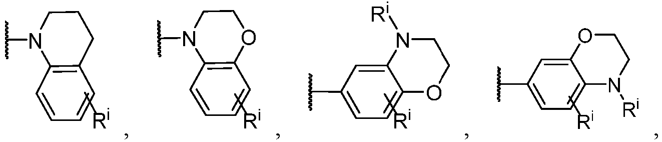 or

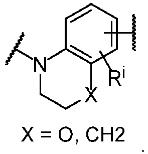

In some embodiments, B is 6-membered heteroaryl-fused 5-membered heteroaryl, wherein the heteroatom in the 6-membered heteroaryl is N, and the number of heteroatoms is 1; preferably, the heteroatom in the 5-membered heteroaryl is N or S, and the number of heteroatoms is 1; for example,

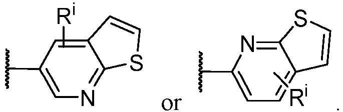

In some embodiments, B is 6-membered heteroaryl-fused 6-membered heterocycloalkyl, wherein the heteroatom in the 6-membered heteroaryl is N, and the number of heteroatoms is 1; preferably, the heteroatom in the 6-membered heterocycloalkyl is O, and the number of heteroatoms is 1; for example,

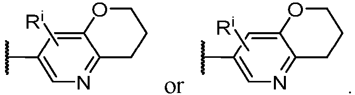

In some embodiments, in D, the 5- to 12-membered heteroaryl is preferably 5- to 6-membered heteroaryl, phenyl-fused 5- to 6-membered heteroaryl, 5- to 7-membered cycloalkyl-fused phenyl, 5- to 7-membered cycloalkyl-fused 5- to 6-membered heteroaryl, 5-to 6-membered heteroaryl-fused 5- to 6-membered heteroaryl, 5- to 7-membered heterocycloalkyl-fused 5- to 6-membered heteroaryl, or 5- to 7-membered heterocycloalkyl-fused 5- to 6-membered aryl; further preferably 5- to 6-membered heteroaryl, phenyl-fused 5-to 6-membered heteroaryl, 5- to 7-membered cycloalkyl-fused 5- to 6-membered heteroaryl, 5- to 6-membered heteroaryl-fused 5- to 6-membered heteroaryl, 5- to 7-membered heterocycloalkyl-fused 5- to 6-membered heteroaryl, or 5- to 7-membered heterocycloalkyl-fused phenyl.

In some embodiments, in D, the 5- to 12-membered heteroaryl is 5- to 6-membered heteroaryl, phenyl-fused 5- to 6-membered heteroaryl, 5- to 7-membered cycloalkyl-fused 5-to 6-membered heteroaryl, 5- to 6-membered heteroaryl-fused 5- to 6-membered heteroaryl, 5-to 7-membered heterocycloalkyl-fused 5- to 6-membered heteroaryl, or 5- to 7-membered heterocycloalkyl-fused phenyl, wherein the heteroatom in the 5- to 6-membered heteroaryl and 5- to 7-membered heterocycloalkyl is preferably selected from one, two, or three kinds of N, O, and S, and the number of heteroatoms is preferably 1, 2, or 3.

In some embodiments, in D, the 5- to 6-membered heteroaryl is preferably 5-membered heteroaryl or 6-membered heteroaryl.

In some embodiments, D is hydrogen, methyl, ethyl, isopropyl, cyclopropyl, or trifluoromethyl.

In some embodiments, D is 6-membered heteroaryl, wherein the heteroatom in the 6-membered heteroaryl is N, and the number of heteroatoms is 1 or 2; for example, 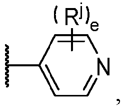 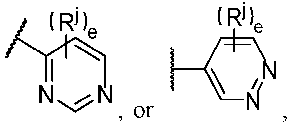 wherein e is independently 0, 1, 2, or 3; for another example,

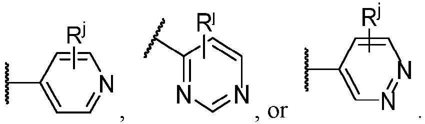

In some embodiments, D is 6-membered heteroaryl, wherein the heteroatom in the 6-membered heteroaryl is N, and the number of heteroatoms is 1 or 2; for example,

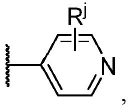

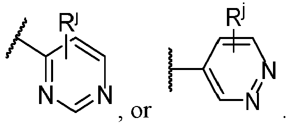

In some embodiments, D is 5-membered heteroaryl, wherein the heteroatom in the 5-membered heteroaryl is one or more kinds of N, O, and S, and the number of heteroatoms is 1, 2, or 3; for example,

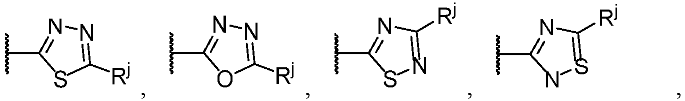

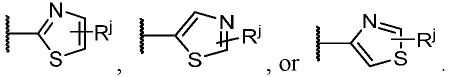

In some embodiments, D is 5-membered heteroaryl, wherein the heteroatom in the 5-membered heteroaryl is N and/or S, and the number of heteroatoms is 1, 2, or 3; for example,

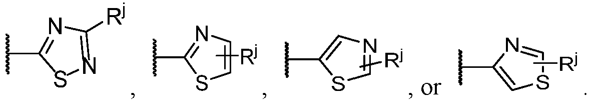

In some embodiments, D is 5-membered heteroaryl, wherein the heteroatom in the 5-membered heteroaryl is N or S, and the number of heteroatoms is 1, 2, or 3; for example,

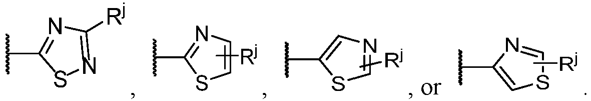

In some embodiments, D is 6-membered heteroaryl-fused 5-membered heterocycloalkyl, wherein the heteroatom in the 6-membered heteroaryl is N, and the number of heteroatoms is 1; preferably, the heteroatom in the 5-membered heterocycloalkyl is N or O, and the number of heteroatoms is 1; for example, 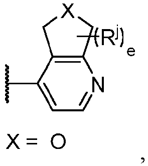 wherein e is independently 0, 1, 2, or 3; for another example,

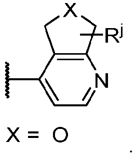

In some embodiments, D is 6-membered heteroaryl-fused 5-membered heterocycloalkyl, wherein the heteroatom in the 6-membered heteroaryl is N, and the number of heteroatoms is 1; preferably, the heteroatom in the 5-membered heterocycloalkyl is N or O, and the number of heteroatoms is 1; for example,

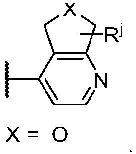

In some embodiments, D is 6-membered heteroaryl-fused 5-membered cycloalkyl, wherein the heteroatom in the 6-membered heteroaryl is N, and the number of heteroatoms is 1; for example, 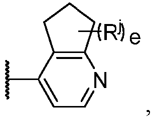 wherein e is independently 0, 1, 2, or 3; for another example,

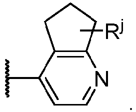

In some embodiments, D is 6-membered heteroaryl-fused 5-membered cycloalkyl, wherein the heteroatom in the 6-membered heteroaryl is N, and the number of heteroatoms is 1; for example,

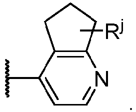

In some embodiments, D is phenyl-fused 5-membered heteroaryl, wherein the heteroatom in the 5-membered heteroaryl is N and/or S, and the number of heteroatoms is 2; for example, 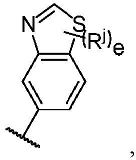 wherein e is independently 0, 1, 2, or 3; for another example,

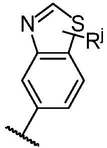

In some embodiments, D is 5- to 6-membered heteroaryl-fused 5- to 6-membered heteroaryl, wherein the heteroatom in the 5- to 6-membered heteroaryl is N and/or S, and the number of heteroatoms is 1, 2, or 3; for example, 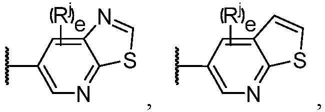 or 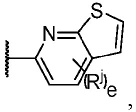 wherein e is independently 0, 1, 2, or 3.

In some embodiments, D is 5- to 6-membered heteroaryl-fused 5- to 6-membered heterocycloalkyl, wherein the heteroatom in the 5- to 6-membered heteroaryl is N, and the number of heteroatoms is 1, 2, or 3; preferably, the heteroatom in the 5- to 6-membered heterocycloalkyl is O, and the number of heteroatoms is 1, 2, or 3; for example, 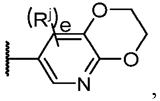 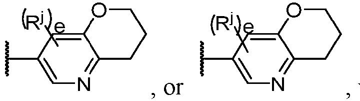 wherein e is independently 0, 1, 2, or 3.

In some embodiments, D is phenyl-fused 5- to 6-membered heterocycloalkyl, wherein the heteroatom in the 5- to 6-membered heterocycloalkyl is one or more kinds of O, N, and O, and the number of heteroatoms is 1, 2, or 3; for example, 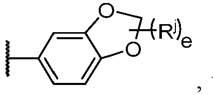 wherein e is independently 0, 1, 2, or 3.

In some embodiments, D is phenyl-fused 5- to 6-membered heteroaryl, wherein the heteroatom in the 5- to 6-membered heteroaryl is N, and the number of heteroatoms is 1, 2, or 3; for example, 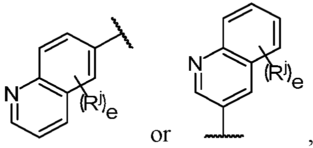 wherein e is independently 0, 1, 2, or 3.

In a certain embodiment, 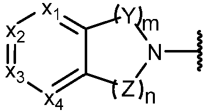 is preferably 6-membered heteroaryl-fused 5-membered heterocycloalkyl, wherein the heteroatom in the 6-membered heteroaryl is N, and the number of heteroatoms is 2; for example, 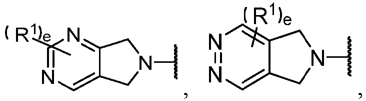 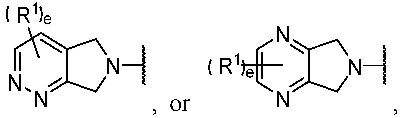 wherein e is independently 0, 1, or 2; for another example,

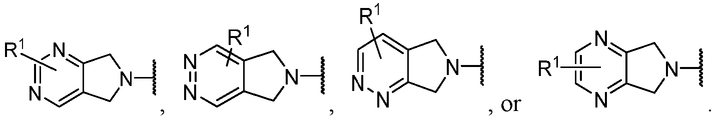

In a certain embodiment, 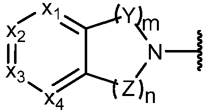 is preferably 6-membered heteroaryl-fused 5-membered heterocycloalkyl, wherein the heteroatom in the 6-membered heteroaryl is N, and the number of heteroatoms is 2; for example,

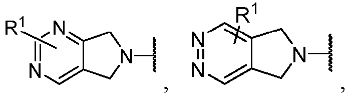

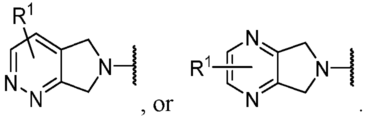

In a certain embodiment, 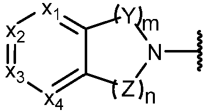 is preferably 6-membered heteroaryl-fused 7-membered heterocycloalkyl, wherein the heteroatom in the 6-membered heteroaryl is N, and the number of heteroatoms is 1 or 2; for example, 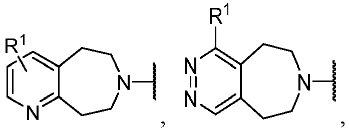 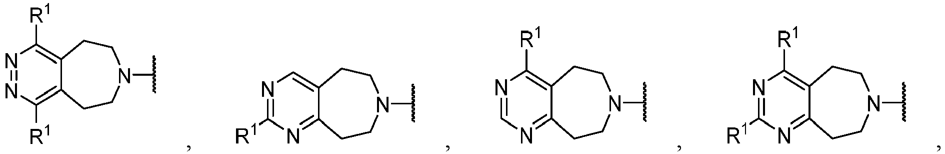 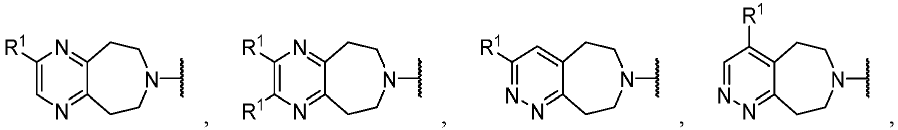 or

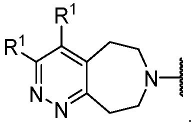

In a certain embodiment, in L, E is preferably carbonyl, F is a chemical bond, t is 0, and u is 1.

In a certain embodiment, B is preferably 4-membered heterocycloalkyl, wherein the heteroatom in the 4-membered heterocycloalkyl is N, and the number of heteroatoms is 1 or 2; for example,

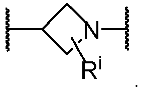

In a certain embodiment, D is preferably 6-membered heteroaryl, wherein the heteroatom in the 6-membered heteroaryl is N, and the number of heteroatoms is 1 or 2; for example,

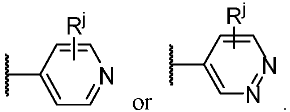

In a preferred embodiment, in the compound of formula I or the pharmaceutically acceptable salt thereof, 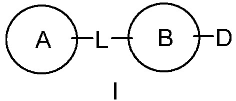
wherein 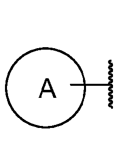 is 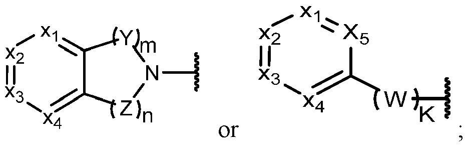
m is a natural number of 0 to 3;
n is a natural number of 0 to 3; m and n are not simultaneously 0;
k is a natural number of 0 to 3;
X₁, X₂, X₃, X₄, and X₅ are independently -CR¹-, N, S, 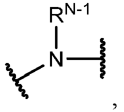 or a chemical bond;
R^{N-1} is hydrogen, C₁-C₆ alkyl, or 3- to 7-membered cycloalkyl;
Y and Z are independently carbonyl (CO) or -(CR²R³)ᵣ-; r is a natural number of 0 to 5;
each W is independently -O-, -(CR⁴R⁵)-, -NR⁶-, or a chemical bond;
each R¹ is independently hydrogen, halogen, C₁-C₆ alkyl, C₁-C₆ alkoxy, C₁-C₆ alkylthio, 3- to 7-membered cycloalkyl, 3- to 7-membered heterocycloalkyl, or -NR¹⁻¹R¹⁻², wherein the C₁-C₆ alkylthio, C₁-C₆ alkyl, and C₁-C₆ alkoxy are optionally and independently substituted by 1, 2, 3, or 4 R^{a};
each R^{a} is independently halogen, hydroxyl, C₁-C₆ alkoxy, or -NR¹⁻⁴R¹⁻⁵;
R¹⁻¹ and R¹⁻² are independently hydrogen or C₁-C₆ alkyl, wherein the C₁-C₆ alkyl is optionally and independently substituted by 1, 2, 3, or 4 R^{b}; or R¹⁻¹ and R¹⁻² together with the atom to which they are attached form a 3- to 7-membered heterocycloalkyl group, wherein the 3- to 7-membered heterocycloalkyl group is optionally substituted by 1, 2, 3, or 4 R^{b-2};
each R^{b} is independently hydroxyl, 3- to 7-membered cycloalkyl, or C₁-C₃ alkyl;
each R^{b-2} is independently halogen or C₁-C₆ alkyl;
R¹⁻⁴ and R¹⁻⁵ are independently hydrogen or C₁-C₃ alkyl, wherein the C₁-C₃ alkyl is optionally and independently substituted by 1, 2, 3, or 4 R¹⁻⁴⁻¹; or R¹⁻⁴ and R¹⁻⁵ together with the atom to which they are attached form a 3- to 7-membered heterocycloalkyl group;
each R¹⁻⁴⁻¹ is independently halogen;
R² and R³ are independently hydrogen or NR²⁻¹R²⁻²;
R²⁻¹ and R²⁻² are independently hydrogen or C₁-C₆ alkyl;
R⁴, R⁵, and R⁶ are independently hydrogen or NR⁴⁻¹R⁴⁻²;
R⁴⁻¹ and R⁴⁻² are independently hydrogen;
L is 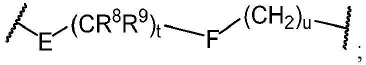
t is a natural number of 0 to 3;
u is a natural number of 0 to 3;
E is carbonyl, 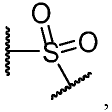 -NHCO-, or a chemical bond;
F is carbonyl, 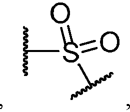 -O-, -NH-, or a chemical bond;
R⁸ and R⁹ are independently hydrogen or C₁-C₆ alkyl; or R⁸ and R⁹ together with the atom to which they are attached form a 3- to 7-membered cycloalkyl group or a 3- to 7-membered heterocycloalkyl group;
B is 4- to 6-membered heterocycloalkyl, 6- to 10-membered aryl, or 5- to 12-membered heteroaryl; wherein the 4- to 6-membered heterocycloalkyl, 6- to 10-membered aryl, and 5- to 12-membered heteroaryl are optionally and independently substituted by 1, 2, or 3 Rⁱ;
each Rⁱ is independently hydrogen, halogen, hydroxyl, or C₁-C₃ alkyl; or two Rⁱ on the same atom together with the atom to which they are attached form a 3- to 7-membered cycloalkyl group; or two adjacent Rⁱ together with the atom to which they are attached form a 3- to 7-membered cycloalkyl group;
D is hydrogen, C₁-C₆ alkyl, or 5- to 12-membered heteroaryl; wherein the C₁-C₆ alkyl and 5- to 12-membered heteroaryl are optionally and independently substituted by 1, 2, or 3 R^{j};
each R^{j} is independently hydrogen, halogen, C₁-C₃ alkyl, 3- to 7-membered cycloalkyl, -OR¹²⁻³, or -SR¹²⁻⁴; the C₁-C₃ alkyl and 3- to 7-membered cycloalkyl are optionally and independently substituted by 1, 2, or 3 R^{k};
R¹²⁻³ and R¹²⁻⁴ are independently C₁-C₃ alkyl; wherein the C₁-C₃ alkyl is optionally and independently substituted by 1, 2, or 3 R^{k-1};
each R^{k} is independently halogen;
each R^{k-1} is independently halogen;
the heteroatom in the 3- to 7-membered heterocycloalkyl, 4- to 6-membered heterocycloalkyl, and 5- to 12-membered heteroaryl is one or more kinds of N, O, and S, and the number of heteroatoms is 1 to 3.

In a preferred embodiment, in the compound of formula I or the pharmaceutically acceptable salt thereof, 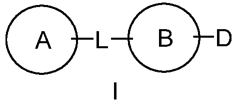
wherein 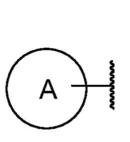 is 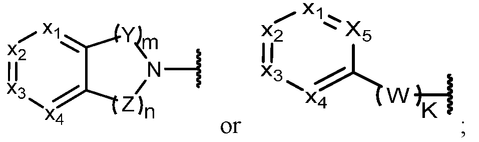
m is a natural number of 0 to 3;
n is a natural number of 0 to 3; m and n are not simultaneously 0;
k is a natural number of 0 to 3;
X₁, X₂, X₃, X₄, and X₅ are independently -CR¹-, N, S, 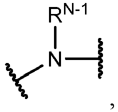 or a chemical bond;
R^{N-1} is C₁-C₆ alkyl;
Y and Z are independently carbonyl (CO) or -(CR²R³)ᵣ-; r is a natural number of 0 to 5;
each W is independently -(CR⁴R⁵)-, -NR⁶-, or a chemical bond;
each R¹ is independently hydrogen, halogen, C₁-C₆ alkyl, C₁-C₆ alkoxy, C₁-C₆ alkylthio, 3- to 7-membered cycloalkyl, 3- to 7-membered heterocycloalkyl, or -NR¹⁻¹R¹⁻², wherein the C₁-C₆ alkylthio, C₁-C₆ alkyl, and C₁-C₆ alkoxy are optionally and independently substituted by 1, 2, 3, or 4 R^{a};
each R^{a} is independently hydroxyl, C₁-C₆ alkoxy, or -NR¹⁻⁴R¹⁻⁵;
R¹⁻¹ and R¹⁻² are independently hydrogen or C₁-C₆ alkyl, wherein the C₁-C₆ alkyl is optionally and independently substituted by 1, 2, 3, or 4 R^{b}; or R¹⁻¹ and R¹⁻² together with the atom to which they are attached form a 3- to 7-membered heterocycloalkyl group, wherein the 3- to 7-membered heterocycloalkyl group is optionally substituted by 1, 2, 3, or 4 R^{b-2};
each R^{b} is independently 3- to 7-membered cycloalkyl;
each R^{b-2} is independently halogen or C₁-C₆ alkyl;
R¹⁻⁴ and R¹⁻⁵ are independently C₁-C₃ alkyl;
R² and R³ are independently hydrogen or NR²⁻¹R²⁻²;
R²⁻¹ and R²⁻² are independently hydrogen;
R⁴, R⁵, and R⁶ are independently hydrogen;
L is 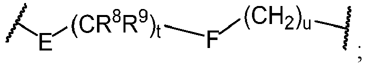
t is a natural number of 0 to 3;
u is a natural number of 0 to 3;
E is carbonyl, 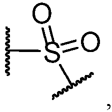 -NHCO-, or a chemical bond;
F is a chemical bond;
R⁸ and R⁹ are independently hydrogen or C₁-C₆ alkyl; or R⁸ and R⁹ together with the atom to which they are attached form a 3- to 7-membered cycloalkyl group;
B is 4- to 6-membered heterocycloalkyl, 6- to 10-membered aryl, or 5- to 12-membered heteroaryl; wherein the 4- to 6-membered heterocycloalkyl, 6- to 10-membered aryl, and 5- to 12-membered heteroaryl are optionally and independently substituted by 1, 2, or 3 Rⁱ;
each Rⁱ is independently hydrogen, halogen, hydroxyl, or C₁-C₃ alkyl; or two Rⁱ on the same atom together with the atom to which they are attached form a 3- to 7-membered cycloalkyl group;
D is hydrogen, C₁-C₆ alkyl, or 5- to 12-membered heteroaryl; wherein the C₁-C₆ alkyl and 5- to 12-membered heteroaryl are optionally and independently substituted by 1, 2, or 3 R^{j};
each R^{j} is independently hydrogen, halogen, C₁-C₃ alkyl, 3- to 7-membered cycloalkyl, -OR¹²⁻³, or -SR¹²⁻⁴; the C₁-C₃ alkyl and 3- to 7-membered cycloalkyl are optionally and independently substituted by 1, 2, or 3 R^{k};
R¹²⁻³ and R¹²⁻⁴ are independently C₁-C₃ alkyl; wherein the C₁-C₃ alkyl is optionally and independently substituted by 1, 2, or 3 R^{k-1};
each R^{k} is independently halogen;
each R^{k-1} is independently halogen;
the heteroatom in the 3- to 7-membered heterocycloalkyl, 4- to 6-membered heterocycloalkyl, and 5- to 12-membered heteroaryl is one or more kinds of N, O, and S, and the number of heteroatoms is 1 to 3.

In a preferred embodiment, in the compound of formula I or the pharmaceutically acceptable salt thereof, 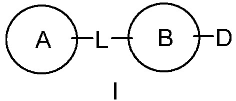
wherein 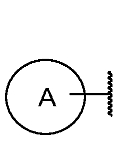 is 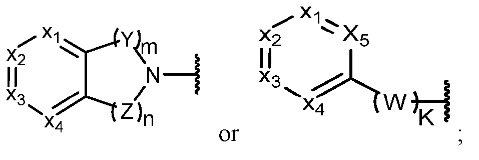
m is a natural number of 1 to 3;
n is a natural number of 1 to 3;
k is a natural number of 1 to 3;
X₁, X₂, X₃, X₄, and X₅ are independently -CR¹-, N, O, S, 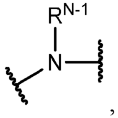 or a chemical bond, and the number of heteroatoms in X₁, X₂, X₃, X₄, and X₅ is 0, 1, 2, or 3;
R^{N-1} is hydrogen or C₁-C₆ alkyl;
Y and Z are independently carbonyl (CO) or -(CR²R³)ᵣ-; r is a natural number of 1 to 2;
each W is independently -O-, -(CR⁴R⁵)-, -NR⁶-, or a chemical bond;
each R¹ is independently hydrogen, halogen, C₁-C₆ alkyl, C₁-C₆ alkoxy, 3- to 7-membered cycloalkyl, 3- to 7-membered heterocycloalkyl, or -NR¹⁻¹R¹⁻², wherein the C₁-C₆ alkyl and C₁-C₆ alkoxy are optionally and independently substituted by 1, 2, 3, or 4 R^{a};
each R^{a} is independently hydroxyl, C₁-C₃ alkoxy, or NR¹⁻⁴R¹⁻⁵;
R¹⁻¹ and R¹⁻² are independently hydrogen or C₁-C₆ alkyl, wherein the C₁-C₆ alkyl is optionally and independently substituted by 1, 2, 3, or 4 R^{b}; or R¹⁻¹ and R¹⁻² together with the atom to which they are attached form a 3- to 7-membered heterocycloalkyl group;
each R^{b} is independently hydroxyl, 3- to 7-membered cycloalkyl, 3- to 7-membered heterocycloalkyl, C₁-C₃ alkyl, C₁-C₃ alkoxy, or NR¹⁻¹⁻¹R¹⁻²⁻¹, wherein the 3- to 7-membered cycloalkyl, 3- to 7-membered heterocycloalkyl, and C₁-C₃ alkyl are optionally and independently substituted by 1, 2, 3, or 4 R^{b-1};
each R^{b-1} is independently hydroxyl;
R¹⁻⁴ and R¹⁻⁵ are independently hydrogen or C₁-C₃ alkyl; wherein the C₁-C₃ alkyl is optionally and independently substituted by 1, 2, 3, or 4 R¹⁻⁴⁻¹; or R¹⁻⁴ and R¹⁻⁵ together with the atom to which they are attached form a 3- to 7-membered heterocycloalkyl group;
each R¹⁻⁴⁻¹ is independently halogen;
R¹⁻¹⁻¹ and R¹⁻²⁻¹ are independently hydrogen or C₁-C₃ alkyl;
R² and R³ are independently hydrogen or NR²⁻¹R²⁻²;
R²⁻¹ and R²⁻² are independently hydrogen or C₁-C₆ alkyl;
R⁴, R⁵, and R⁶ are independently hydrogen;
L is 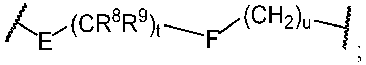
t is a natural number of 0 to 2;
u is a natural number of 0 to 2;
E is carbonyl, -NHCO-, or a chemical bond;
F is -O-, -NH-, or a chemical bond;
R⁸ and R⁹ are independently hydrogen or C₁-C₆ alkyl; or R⁸ and R⁹ together with the atom to which they are attached form a 3- to 7-membered cycloalkyl group or a 3- to 7-membered heterocycloalkyl group;
B is 4- to 6-membered heterocycloalkyl, 6- to 10-membered aryl, or 5- to 12-membered heteroaryl, wherein the 5- to 12-membered heteroaryl, 6- to 10-membered aryl, and 4- to 6-membered heterocycloalkyl are optionally and independently substituted by 1, 2, or 3 Rⁱ;
each Rⁱ is independently hydrogen, hydroxyl, halogen, or C₁-C₃ alkyl; or two Rⁱ together with the atom to which they are attached form a 3- to 7-membered cycloalkyl group or a 3- to 7-membered heterocycloalkyl group; or two adjacent Rⁱ together with the atom to which they are attached form a 3- to 7-membered cycloalkyl group or a 3- to 7-membered heterocycloalkyl group (wherein the 3- to 7-membered cycloalkyl group or the 3- to 7-membered heterocycloalkyl group together with B forms a fused ring);
D is hydrogen, 3- to 7-membered cycloalkyl, C₁-C₆ alkyl, or 5- to 12-membered heteroaryl; wherein the C₁-C₆ alkyl and 5- to 12-membered heteroaryl are optionally and independently substituted by 1, 2, or 3 R^{j};
each R^{j} is independently halogen, C₁-C₃ alkyl, -OR¹²⁻³, or -SR¹²⁻⁴; the C₁-C₃ alkyl is optionally and independently substituted by 1, 2, or 3 R^{k}; each R^{k} is independently halogen;
R¹²⁻³ and R¹²⁻⁴ are independently C₁-C₃ alkyl; the C₁-C₃ alkyl is optionally and independently substituted by 1, 2, or 3 R^{k}; each R^{k} is independently halogen;
the heteroatom in the 3- to 7-membered heterocycloalkyl, 4- to 6-membered heterocycloalkyl, and 5- to 12-membered heteroaryl is N, O, or S, and the number of heteroatoms is 1 to 3; or the heteroatom in the 3- to 7-membered heterocycloalkyl, 4- to 6-membered heterocycloalkyl, and 5- to 12-membered heteroaryl is one or more kinds of N, O, and S, and the number of heteroatoms is 1 to 3.

In a preferred embodiment, in the compound of formula I or the pharmaceutically acceptable salt thereof, 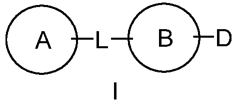
wherein 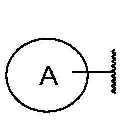 is 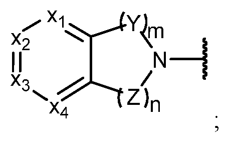
m is a natural number of 1 to 3;
n is a natural number of 1 to 3;
X₁, X₂, X₃, and X₄ are independently -CR¹-, N, S, 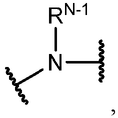 or a chemical bond, and the number of heteroatoms in X₁, X₂, X₃, X₄, and X₅ is 1 or 2;
R^{N-1} is C₁-C₆ alkyl;
Y and Z are independently carbonyl (CO) or -(CR²R³)ᵣ-; r is a natural number of 1 to 2;
each R¹ is independently hydrogen, halogen, C₁-C₆ alkyl, C₁-C₆ alkoxy, 3- to 7-membered cycloalkyl, 3- to 7-membered heterocycloalkyl, or -NR¹⁻¹R¹⁻², wherein the C₁-C₆ alkyl and C₁-C₆ alkoxy are optionally and independently substituted by 1, 2, 3, or 4 R^{a};
each R^{a} is independently hydroxyl, C₁-C₃ alkoxy, or NR¹⁻⁴R¹⁻⁵;
R¹⁻¹ and R¹⁻² are independently hydrogen or C₁-C₆ alkyl, wherein the C₁-C₆ alkyl is optionally and independently substituted by 1, 2, 3, or 4 R^{b}; or R¹⁻¹ and R¹⁻² together with the atom to which they are attached form a 3- to 7-membered heterocycloalkyl group;
each R^{b} is independently hydroxyl, 3- to 7-membered cycloalkyl, 3- to 7-membered heterocycloalkyl, C₁-C₃ alkyl, C₁-C₃ alkoxy, or NR¹⁻¹⁻¹R¹⁻²⁻¹, wherein the 3- to 7-membered cycloalkyl, 3- to 7-membered heterocycloalkyl, and C₁-C₃ alkyl are optionally and independently substituted by 1, 2, 3, or 4 R^{b-1};
each R^{b-1} is independently hydroxyl;
R¹⁻⁴ and R¹⁻⁵ are independently hydrogen or C₁-C₃ alkyl, wherein the C₁-C₃ alkyl is optionally and independently substituted by 1, 2, 3, or 4 R¹⁻⁴⁻¹; or R¹⁻⁴ and R¹⁻⁵ together with the atom to which they are attached form a 3- to 7-membered heterocycloalkyl group;
R¹⁻¹⁻¹ and R¹⁻²⁻¹ are independently hydrogen or C₁-C₃ alkyl;
R¹⁻⁴⁻¹ is halogen;
R² and R³ are independently hydrogen;
L is 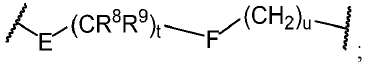
t is a natural number of 0 to 2;
u is a natural number of 0 to 2;
E is carbonyl or a chemical bond;
F is -O-, -NH-, or a chemical bond;
R⁸ and R⁹ are independently C₁-C₆ alkyl; or R⁸ and R⁹ together with the atom to which they are attached form a 3- to 7-membered cycloalkyl group or a 3- to 7-membered heterocycloalkyl group;
B is 4- to 6-membered heterocycloalkyl or 5- to 12-membered heteroaryl; wherein the 5- to 12-membered heteroaryl and 4- to 6-membered heterocycloalkyl are optionally and independently substituted by 1, 2, or 3 Rⁱ;
each Rⁱ is independently hydrogen, hydroxyl, halogen, or C₁-C₃ alkyl; or two Rⁱ together with the atom to which they are attached form a 3- to 7-membered cycloalkyl group or a 3- to 7-membered heterocycloalkyl group; or two adjacent Rⁱ together with the atom to which they are attached form a 3- to 7-membered cycloalkyl group or a 3- to 7-membered heterocycloalkyl group (wherein the 3- to 7-membered cycloalkyl group or the 3- to 7-membered heterocycloalkyl group together with B forms a fused ring);
D is hydrogen, 3- to 7-membered cycloalkyl, C₁-C₆ alkyl, or 5- to 6-membered heteroaryl; wherein the C₁-C₆ alkyl and 5- to 6-membered heteroaryl are optionally and independently substituted by 1, 2, or 3 R^{j};
each R^{j} is independently halogen or C₁-C₃ alkyl; the C₁-C₃ alkyl is optionally and independently substituted by 1, 2, or 3 R^{k}; each R^{k} is independently halogen;
the heteroatom in the 3- to 7-membered heterocycloalkyl, 4- to 6-membered heterocycloalkyl, 5- to 6-membered heteroaryl, and 5- to 12-membered heteroaryl is N, O, or S, and the number of heteroatoms is 1 to 3; or the heteroatom in the 3- to 7-membered heterocycloalkyl, 4- to 6-membered heterocycloalkyl, 5- to 6-membered heteroaryl, and 5- to 12-membered heteroaryl is one or more kinds of N, O, and S, and the number of heteroatoms is 1 to 3.

In a preferred embodiment, in the compound of formula I or the pharmaceutically acceptable salt thereof, 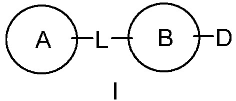
wherein 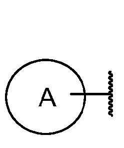 is 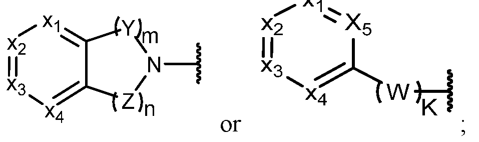
m is a natural number of 1 to 2;
n is a natural number of 1 to 2;
k is a natural number of 1 to 3;
X₁, X₂, X₃, X₄, and X₅ are independently -CR¹-, N, O, S, 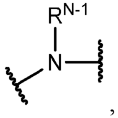 or a chemical bond, and the number of heteroatoms in X₁, X₂, X₃, X₄, and X₅ is 0, 1, 2, or 3;
R^{N-1} is C₁-C₆ alkyl;
each W is independently -(CR⁴R⁵)-, -O-, -NR⁶-, or a chemical bond; Y and Z are independently carbonyl (CO) or -(CR²R³)ᵣ-; r is a natural number of 1 to 2;
each R¹ is independently hydrogen, halogen, C₁-C₆ alkyl, C₁-C₆ alkoxy, 3- to 7-membered cycloalkyl, 3- to 7-membered heterocycloalkyl, or -NR¹⁻¹R¹⁻², wherein the C₁-C₆ alkyl and C₁-C₆ alkoxy are optionally and independently substituted by 1, 2, 3, or 4 R^{a};
each R^{a} is independently hydroxyl, C₁-C₃ alkoxy, or NR¹⁻⁴R¹⁻⁵;
R¹⁻¹ and R¹⁻² are independently hydrogen or C₁-C₆ alkyl, wherein the C₁-C₆ alkyl is optionally and independently substituted by 1, 2, 3, or 4 R^{b}; or R¹⁻¹ and R¹⁻² together with the atom to which they are attached form a 3- to 7-membered heterocycloalkyl group;
each R^{b} is independently hydroxyl, 3- to 7-membered cycloalkyl, 3- to 7-membered heterocycloalkyl, C₁-C₃ alkyl, C₁-C₃ alkoxy, or NR¹⁻¹⁻¹R¹⁻²⁻¹, wherein the 3- to 7-membered cycloalkyl, 3- to 7-membered heterocycloalkyl, and C₁-C₃ alkyl are optionally and independently substituted by 1, 2, 3, or 4 R^{b-1};
each R^{b-1} is independently hydroxyl;
R¹⁻⁴ and R¹⁻⁵ are independently hydrogen or C₁-C₃ alkyl, wherein the C₁-C₃ alkyl is optionally and independently substituted by 1, 2, 3, or 4 R¹⁻⁴⁻¹; or R¹⁻⁴ and R¹⁻⁵ together with the atom to which they are attached form a 3- to 7-membered heterocycloalkyl group;
R¹⁻¹⁻¹ and R¹⁻²⁻¹ are independently hydrogen or C₁-C₃ alkyl;
each R¹⁻⁴⁻¹ is independently halogen;
R² and R³ are independently hydrogen or NR²⁻¹R²⁻²;
R²⁻¹ and R²⁻² are independently hydrogen or C₁-C₆ alkyl;
R⁴, R⁵, and R⁶ are independently hydrogen; L is 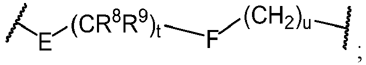
t is a natural number of 0 to 2;
u is a natural number of 0 to 2;
E is carbonyl, -NHCO-, or a chemical bond;
F is -O-, -NH-, or a chemical bond;
R⁸ and R⁹ are independently hydrogen or C₁-C₆ alkyl; or R⁸ and R⁹ together with the atom to which they are attached form a 3- to 7-membered cycloalkyl group or a 3- to 7-membered heterocycloalkyl group;
B is 4- to 6-membered heterocycloalkyl, 6- to 10-membered aryl, or 5- to 12-membered heteroaryl, wherein the 5- to 12-membered heteroaryl, 6- to 10-membered aryl, and 4- to 6-membered heterocycloalkyl are optionally and independently substituted by 1, 2, or 3 Rⁱ;
each Rⁱ is independently hydrogen, hydroxyl, halogen, or C₁-C₃ alkyl; or two Rⁱ together with the atom to which they are attached form a 3- to 7-membered cycloalkyl group or a 3- to 7-membered heterocycloalkyl group; or two adjacent Rⁱ together with the atom to which they are attached form a 3- to 7-membered cycloalkyl group or a 3- to 7-membered heterocycloalkyl group (wherein the 3- to 7-membered cycloalkyl group or the 3- to 7-membered heterocycloalkyl group together with B forms a fused ring);
D is hydrogen, C₁-C₆ alkyl, or 5- to 12-membered heteroaryl; wherein the C₁-C₆ alkyl and 5- to 12-membered heteroaryl are optionally and independently substituted by 1, 2, or 3 R^{j};
each R^{j} is independently halogen, C₁-C₃ alkyl, OR¹²⁻³, or SR¹²⁻⁴; the C₁-C₃ alkyl is optionally and independently substituted by 1, 2, or 3 R^{k}; each R^{k} is independently halogen;
R¹²⁻³ and R¹²⁻⁴ are independently C₁-C₃ alkyl, wherein the C₁-C₃ alkyl is optionally and independently substituted by 1, 2, or 3 R^{k-1}; each R^{k-1} is independently halogen; the heteroatom in the 3- to 7-membered heterocycloalkyl, 4- to 6-membered heterocycloalkyl, and 5- to 12-membered heteroaryl is N, O, or S, and the number of heteroatoms is 1 to 3; or the heteroatom in the 3- to 7-membered heterocycloalkyl, 4- to 6-membered heterocycloalkyl, and 5- to 12-membered heteroaryl is one or more kinds of N, O, and S, and the number of heteroatoms is 1 to 3.

In a preferred embodiment, in the compound of formula I or the pharmaceutically acceptable salt thereof, 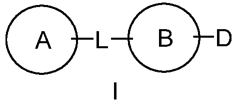
wherein 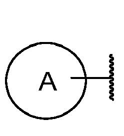 is 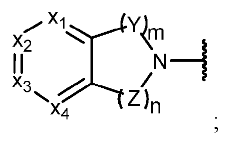
m is 1;
n is 1;
X₁, X₂, X₃, and X₄ are independently -CR¹- or N, and the number of heteroatoms in X₁, X₂, X₃, and X₄ is 2;
Y and Z are independently -(CR²R³)ᵣ-; r is 1;
each R¹ is independently C₁-C₆ alkyl (*e.g.,* methyl) or C₁-C₆ alkoxy (*e.g.,* methoxy);
R² and R³ are independently hydrogen;
L is 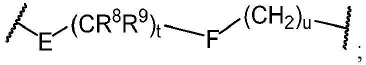
t is 0;
u is 1;
E is carbonyl;
F is a chemical bond;
B is 4- to 6-membered heterocycloalkyl; wherein the 4- to 6-membered heterocycloalkyl is optionally and independently substituted by 1, 2, or 3 Rⁱ;
each Rⁱ is independently hydrogen;
D is 5- to 12-membered heteroaryl; wherein the 5- to 12-membered heteroaryl is optionally and independently substituted by 1, 2, or 3 *(e.g.,* 1) R^{j};
each R^{j} is independently -OR¹²⁻³;
R¹²⁻³ is C₁-C₃ alkyl (e.g., ethyl or isopropyl); the C₁-C₃ alkyl is optionally and independently substituted by 1, 2, or 3 R^{k}; each R^{k} is independently halogen *(e.g.,* F);
the heteroatom in the 4- to 6-membered heterocycloalkyl and 5- to 12-membered heteroaryl is N, O, or S, and the number of heteroatoms is 1 to 3; or the heteroatom in the 4- to 6-membered heterocycloalkyl and 5- to 12-membered heteroaryl is one or more kinds of N, O, and S, and the number of heteroatoms is 1 to 3;
when A is 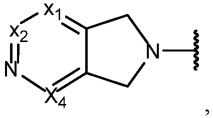 the number of heteroatoms in X₁, X₂, and X₄ is 1 or 2;
when A is 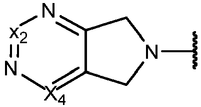 and L is carbonyl, then u is a natural number of 1 to 3, B is 4- to 6-membered heterocycloalkyl, and the 4- to 6-membered heterocycloalkyl is optionally and independently substituted by 1, 2, or 3 Rⁱ.

In a preferred embodiment, in the compound of formula I or the pharmaceutically acceptable salt thereof,
wherein 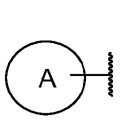 is 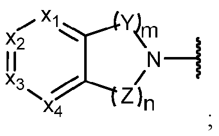
m is 1;
n is 1;
X₂ and X₃ are N;
X₁ and X₄ are independently -CR¹-;
Y and Z are independently -(CR²R³)ᵣ-; r is 1;
each R¹ is independently hydrogen, halogen, C₁-C₆ alkyl, C₁-C₆ alkoxy, 3- to 7-membered cycloalkyl, 3- to 7-membered heterocycloalkyl, or -NR¹⁻¹R¹⁻²; wherein the C₁-C₆ alkyl and C₁-C₆ alkoxy are optionally and independently substituted by 1, 2, 3, or 4 R^{a};
R¹⁻¹ and R¹⁻² are independently hydrogen or C₁-C₆ alkyl; or R¹⁻¹ and R¹⁻² together with the atom to which they are attached form a 3- to 7-membered heterocycloalkyl group;
each R^{a} is independently hydroxyl, C₁-C₆ alkoxy, or NR¹⁻⁴R¹⁻⁵;
R¹⁻⁴ and R¹⁻⁵ are independently hydrogen or C₁-C₃ alkyl;
R² and R³ are independently hydrogen;
L is 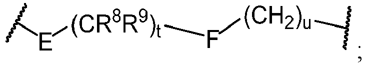
t is 0;
u is 1;
E is carbonyl;
F is a chemical bond;
B is 4- to 6-membered heterocycloalkyl; wherein the 4- to 6-membered heterocycloalkyl is optionally and independently substituted by 1, 2, or 3 Rⁱ;
each Rⁱ is independently hydrogen, halogen, hydroxyl, or C₁-C₃ alkyl;
D is 5- to 12-membered heteroaryl; wherein the 5- to 12-membered heteroaryl is optionally and independently substituted by 1, 2, or 3 R^{j}; preferably, D is 5- to 6-membered heteroaryl; wherein the 5- to 6-membered heteroaryl is optionally and independently substituted by 1, 2, or 3 R^{j}; the heteroatom in the 5- to 6-membered heteroaryl is selected from one, two, or three kinds of N, S, and O, and the number of heteroatoms is 1, 2, or 3;
each R^{j} is independently H, halogen, C₁-C₃ alkyl, OR¹²⁻³, or SR¹²⁻⁴; wherein the C₁-C₃ alkyl is optionally and independently substituted by 1, 2, or 3 R^{k};
R¹²⁻³ and R¹²⁻⁴ are independently C₁-C₃ alkyl; wherein the C₁-C₃ alkyl is optionally and independently substituted by 1, 2, or 3 R^{k-1};
each R^{k} is independently halogen or C₁-C₃ alkyl;
each R^{k-1} is independently halogen or C₁-C₃ alkyl;
the heteroatom in the 3- to 7-membered heterocycloalkyl, 4- to 6-membered heterocycloalkyl, and 5- to 12-membered heteroaryl is one or more kinds of N, O, and S, and the number of heteroatoms is 1 to 3.

In a preferred embodiment, in the compound of formula I or the pharmaceutically acceptable salt thereof,
wherein 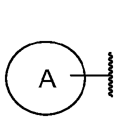 is 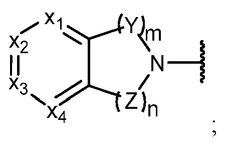
m is 1;
n is 1;
X₁ and X₃ are N;
X₂ and X₄ are independently -CR¹-;
Y and Z are independently -(CR²R³)ᵣ-; r is 1;
each R¹ is independently hydrogen, halogen, cyano, hydroxyl, C₁-C₆ alkyl, C₁-C₆ alkoxy, C₁-C₆ alkylthio, 3- to 7-membered cycloalkyl, 3- to 7-membered heterocycloalkyl, or -NR¹⁻¹R¹⁻²; wherein the C₁-C₆ alkyl, C₁-C₆ alkoxy, and C₁-C₆ alkylthio are optionally and independently substituted by 1, 2, 3, or 4 R^{a};
R¹⁻¹ and R¹⁻² are independently hydrogen or C₁-C₆ alkyl; or R¹⁻¹ and R¹⁻² together with the atom to which they are attached form a 3- to 7-membered heterocycloalkyl group;
each R^{a} is independently hydroxyl, C₁-C₆ alkoxy, or NR¹⁻⁴R¹⁻⁵;
R¹⁻⁴ and R¹⁻⁵ are independently hydrogen or C₁-C₃ alkyl;
R² and R³ are independently hydrogen;
L is 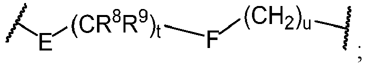
t is 0;
u is 1;
E is carbonyl;
F is a chemical bond;
B is 4- to 6-membered heterocycloalkyl; wherein the 4- to 6-membered heterocycloalkyl is optionally and independently substituted by 1, 2, or 3 Rⁱ;
each Rⁱ is independently hydrogen, halogen, hydroxyl, or C₁-C₃ alkyl; or two Rⁱ together with the atom to which they are attached form a 3- to 7-membered cycloalkyl group;
D is 5- to 12-membered heteroaryl; wherein the 5- to 12-membered heteroaryl is optionally and independently substituted by 1, 2, or 3 R^{j}; preferably, D is 5- to 6-membered heteroaryl; wherein the 5- to 6-membered heteroaryl is optionally and independently substituted by 1, 2, or 3 R^{j}; the heteroatom in the 5- to 6-membered heteroaryl is selected from one, two, or three kinds of N, S, and O, and the number of heteroatoms is 1, 2, or 3;
each R^{j} is independently hydrogen, halogen, cyano, hydroxyl, C₁-C₃ alkyl, 3- to 7-membered cycloalkyl, 3- to 7-membered heterocycloalkyl, -NR¹²⁻¹R¹²⁻², -OR¹²⁻³, or -SR¹²⁻⁴; wherein the C₁-C₃ alkyl, 3- to 7-membered cycloalkyl, and 3- to 7-membered heterocycloalkyl are optionally and independently substituted by 1, 2, or 3 R^{k};
R¹²⁻¹, R¹²⁻², R¹²⁻⁴, and R¹²⁻³ are independently C₁-C₃ alkyl; wherein the C₁-C₃ alkyl is optionally and independently substituted by 1, 2, or 3 R^{k-1};
each R^{k} is independently halogen or C₁-C₃ alkyl;
each R^{k-1} is independently halogen or C₁-C₃ alkyl;
the heteroatom in the 3- to 7-membered heterocycloalkyl, 4- to 6-membered heterocycloalkyl, and 5- to 12-membered heteroaryl is one or more kinds of N, O, and S, and the number of heteroatoms is 1 to 3.

In a certain embodiment, each R¹ is independently hydrogen, methyl, 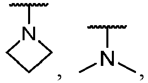 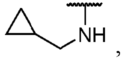 ethyl, chlorine, 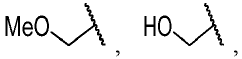 methoxy, 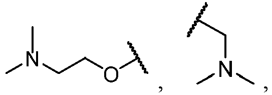 isopropyl, 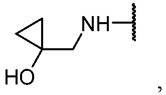 amino, 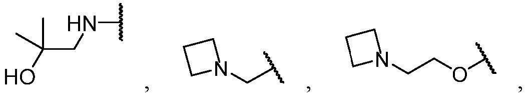 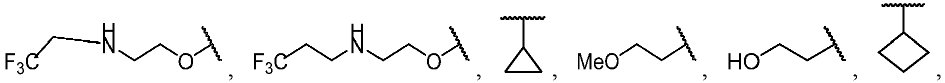 or

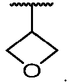

In a certain embodiment, each R¹ is independently hydrogen, methyl, 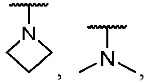 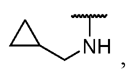 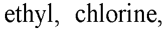 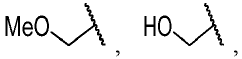 methoxy, 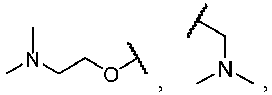 isopropyl, 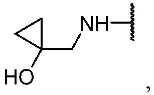 amino (-NH₂), 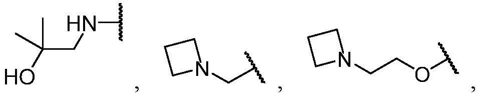 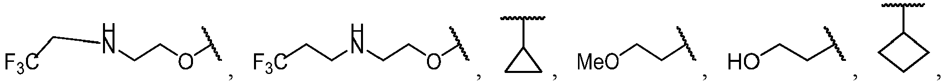 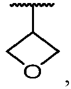 trifluoromethyl, 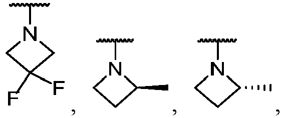 or methylthio.

Preferably, each R¹ is independently methyl,

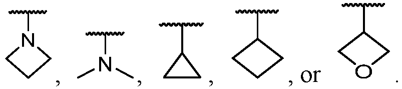

In a certain embodiment,

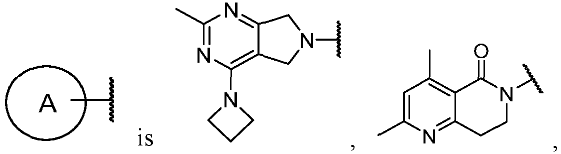

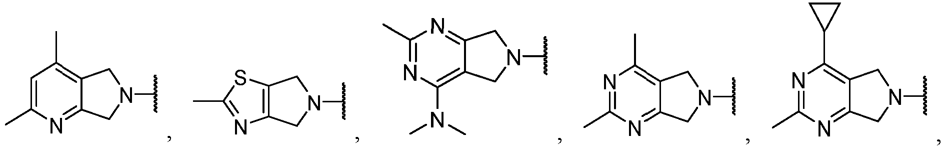

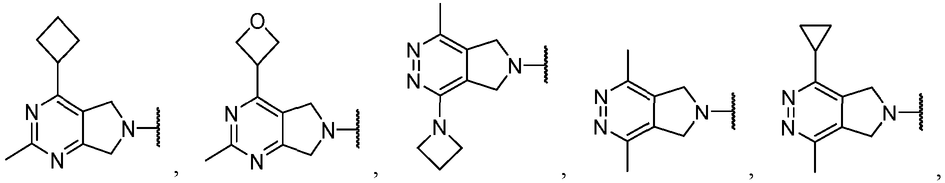

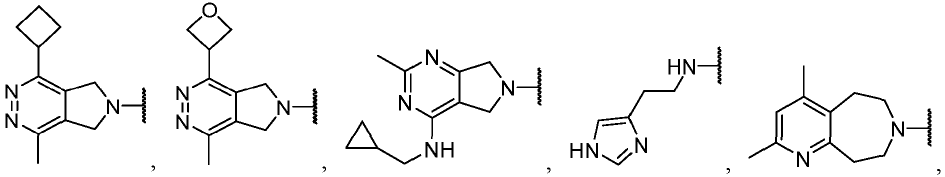

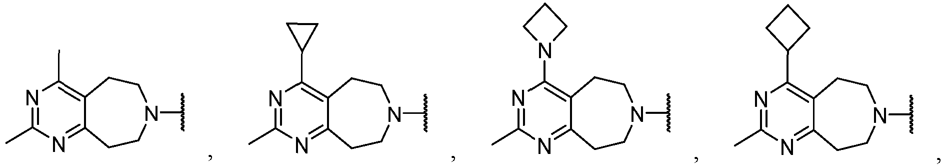

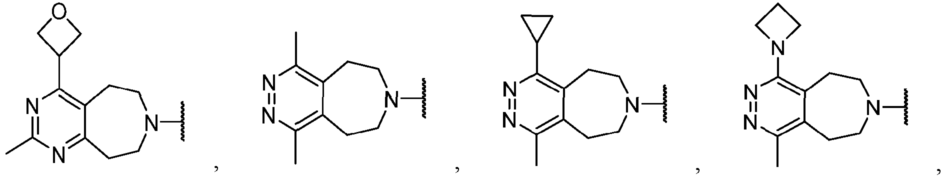

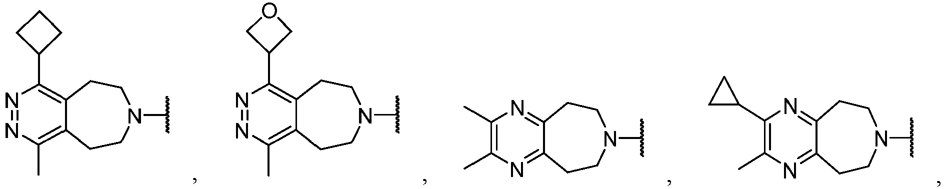

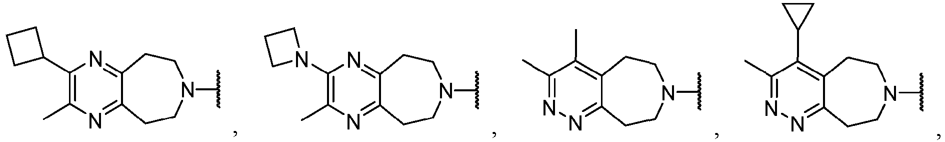

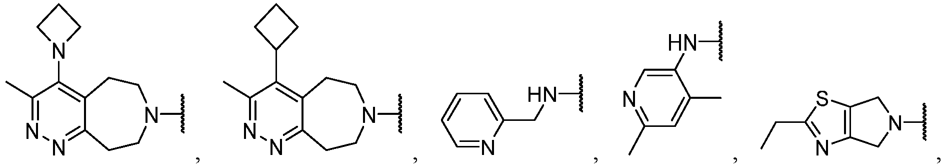

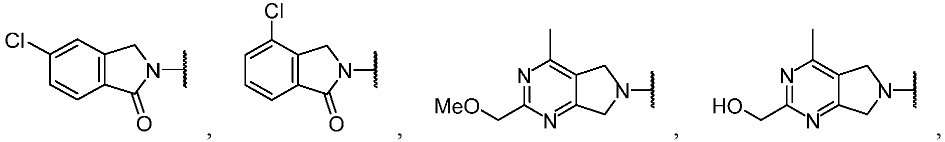

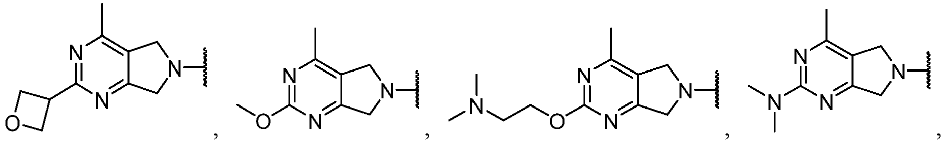

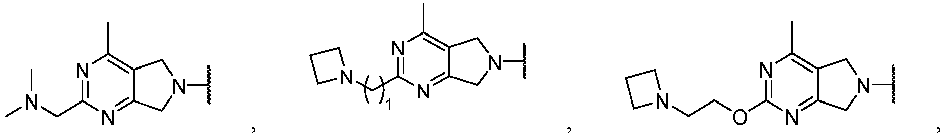

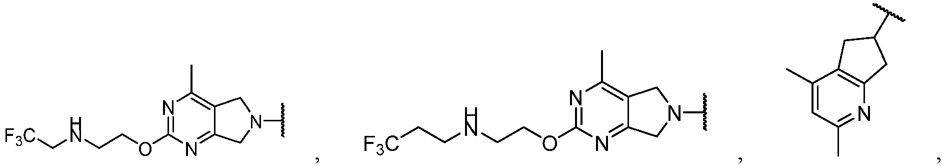

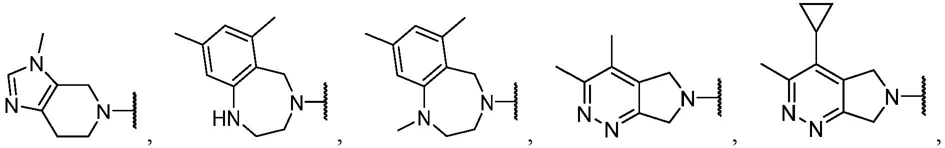

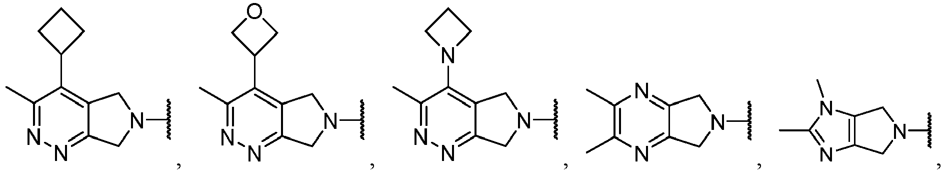

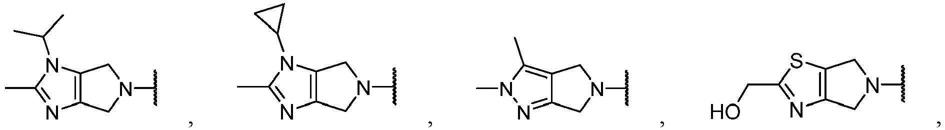

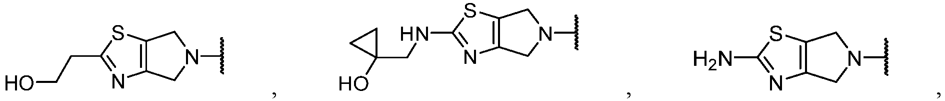

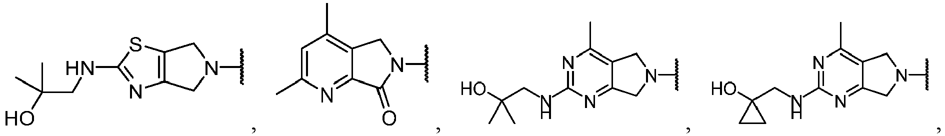

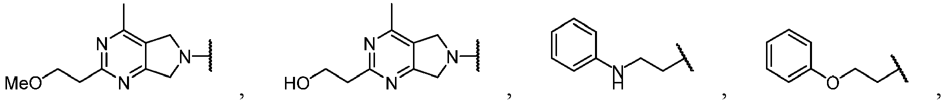

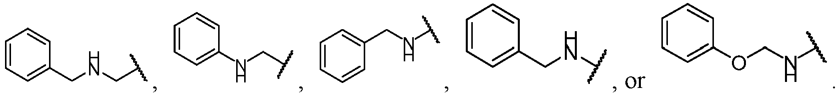

Preferably,

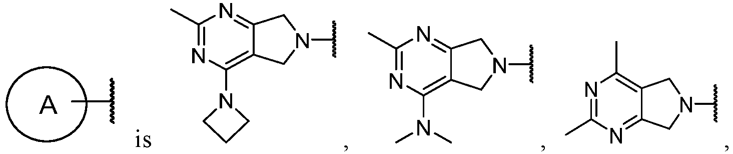

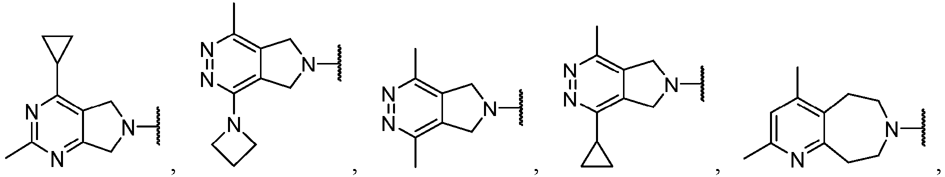

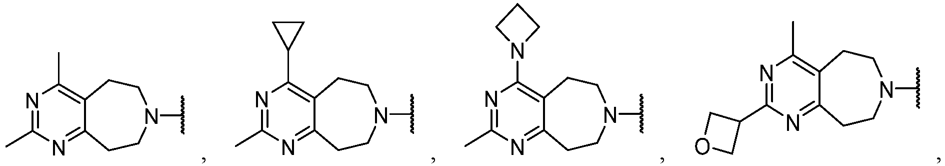

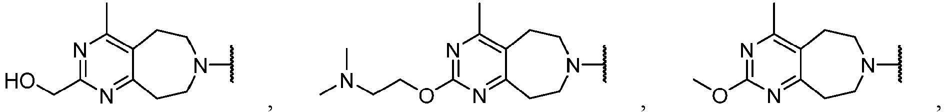

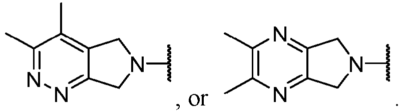

In a certain embodiment,

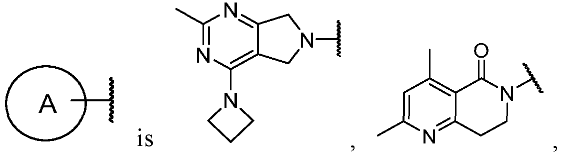

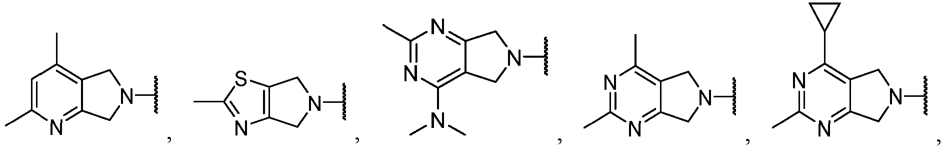

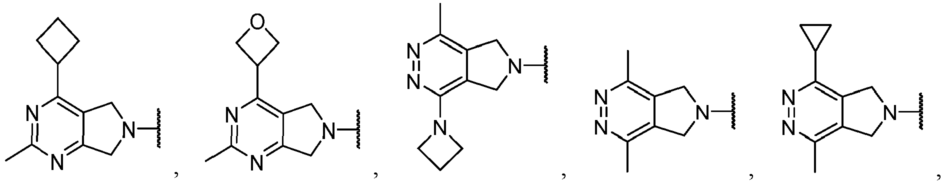

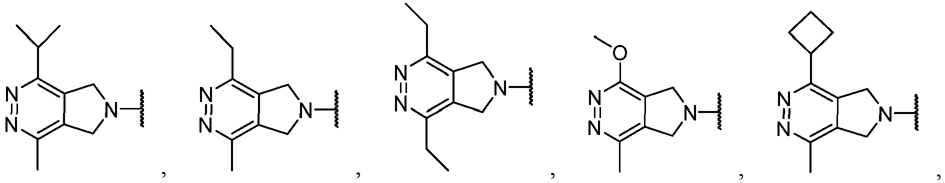

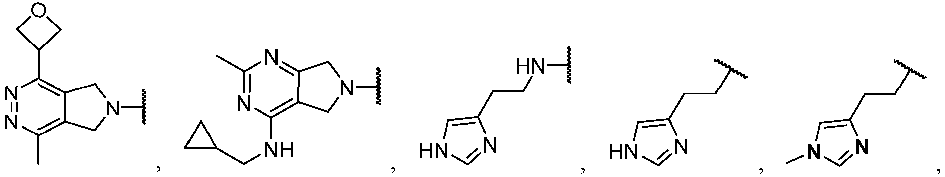

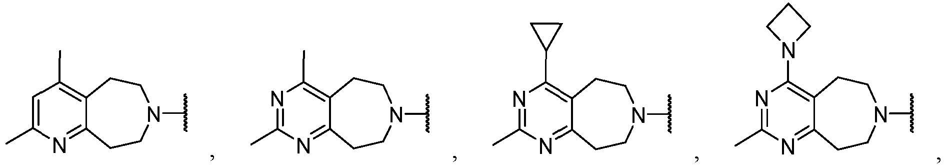

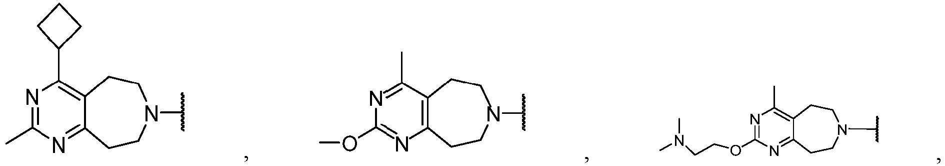

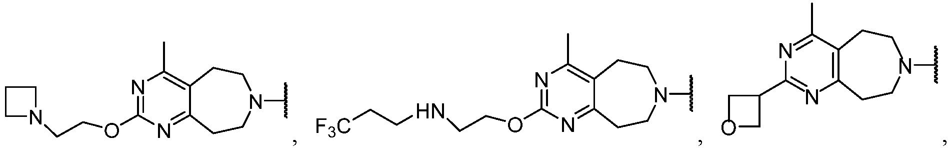

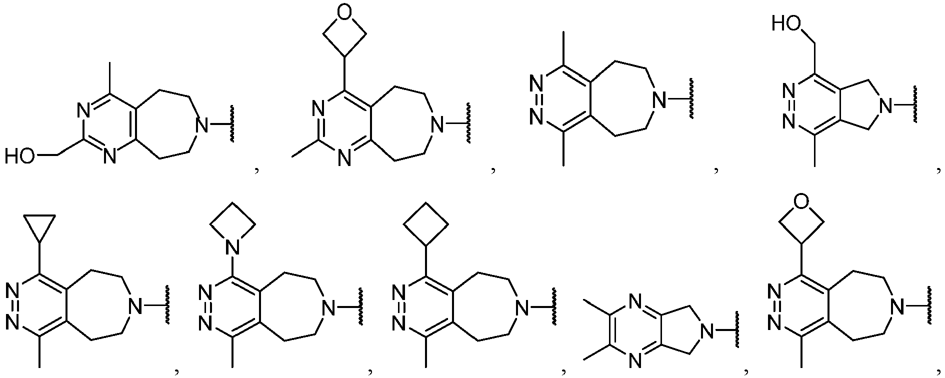

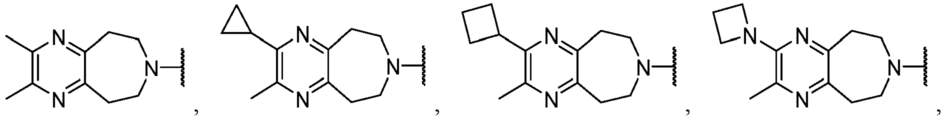

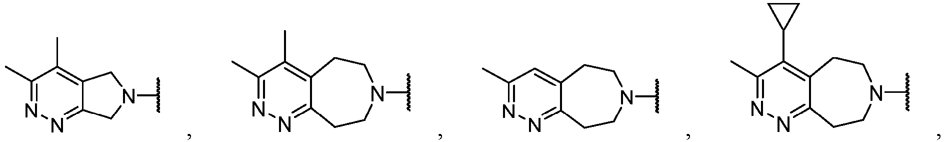

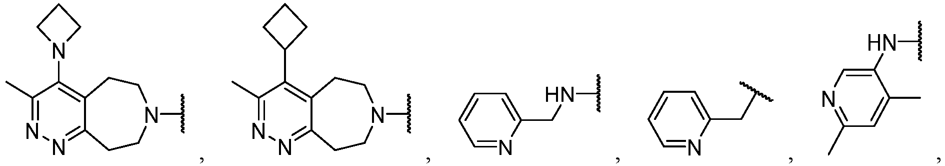

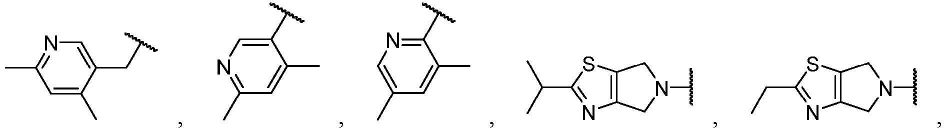

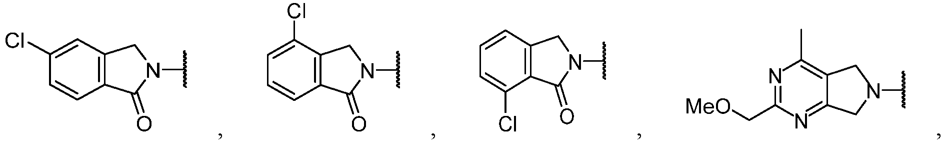

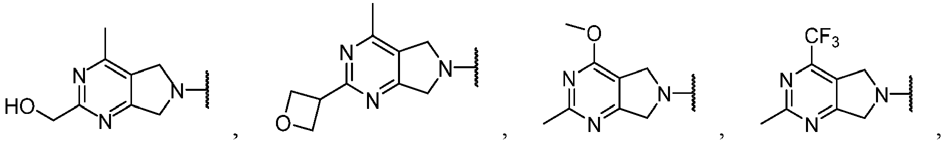

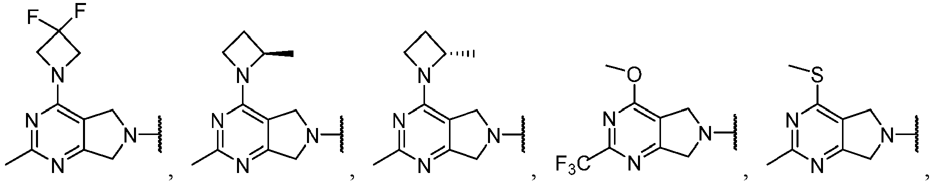

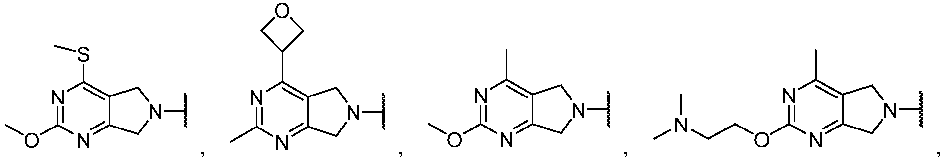

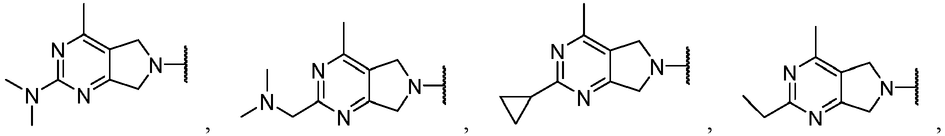

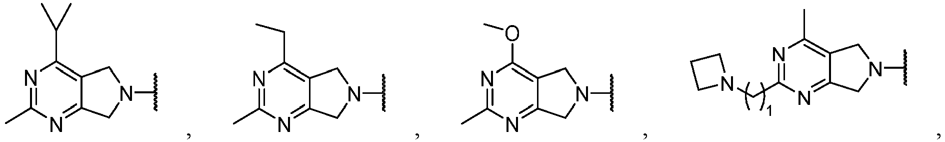

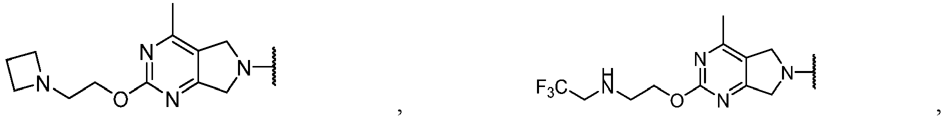

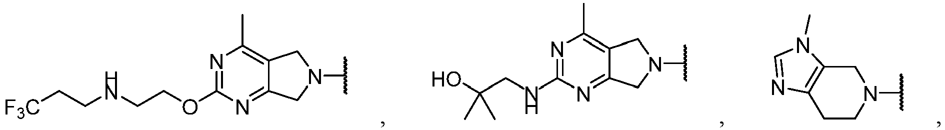

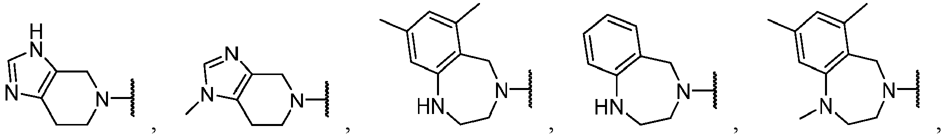

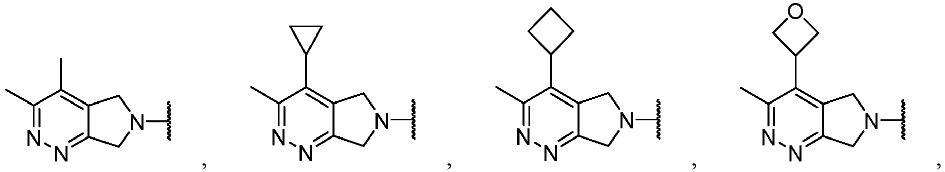

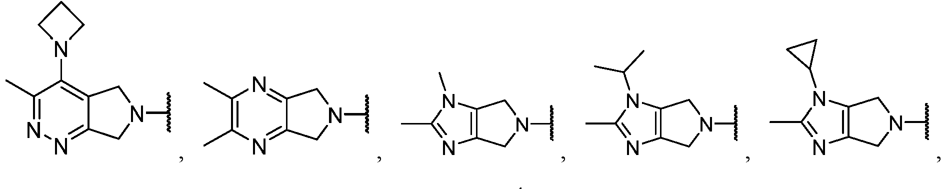

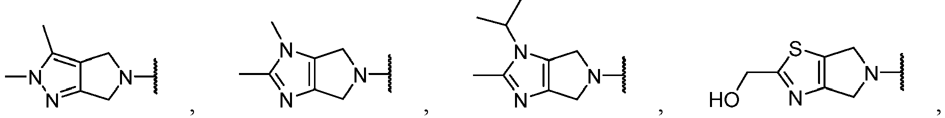

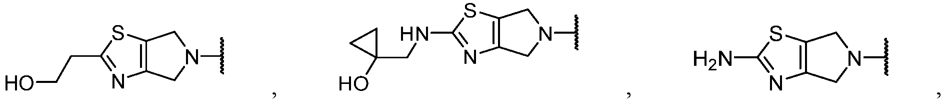

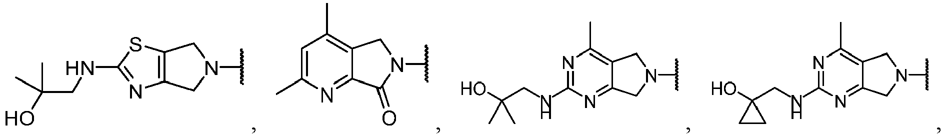

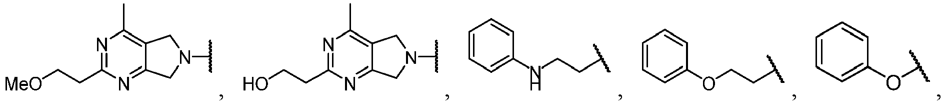

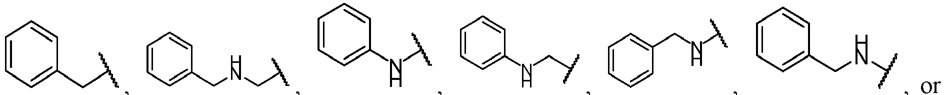

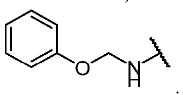

In a certain embodiment, L is -(CH₂)-, -(CH₂)₂-, 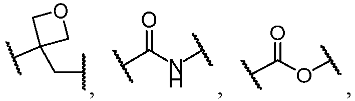 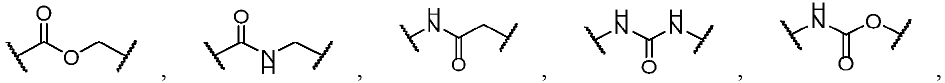 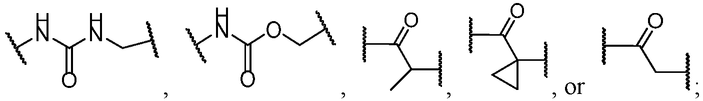 preferably, L is 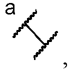 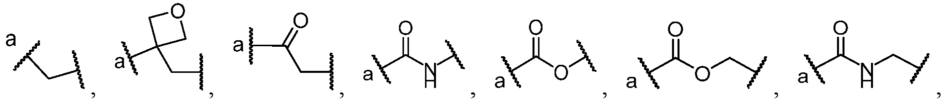 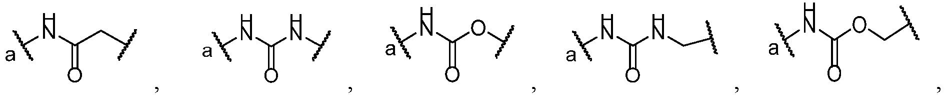 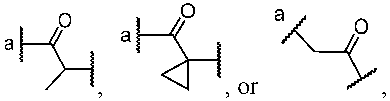 wherein 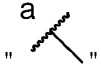 represents the site connected to A.

Preferably, L is

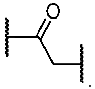

In a certain embodiment, L is -(CH₂)-, -(CH₂)₂-, 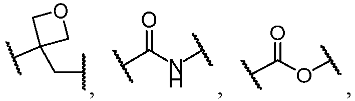 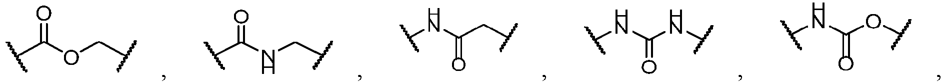 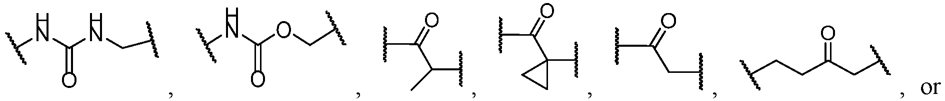 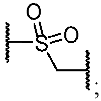 preferably, L is 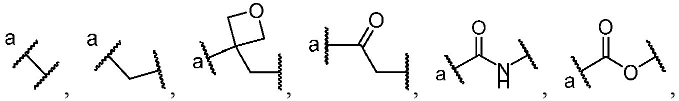 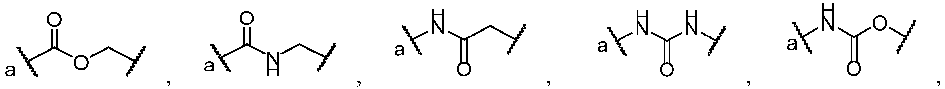 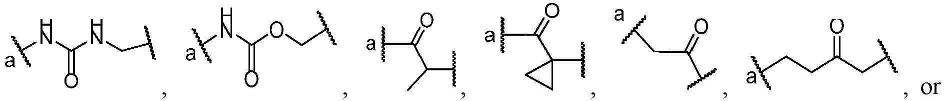 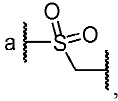 wherein " 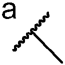 " represents the site connected to A.

In a certain embodiment, B is

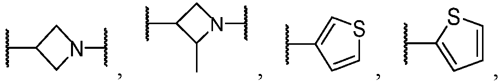

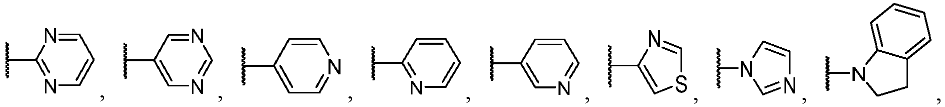

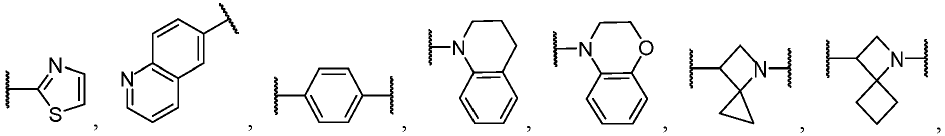

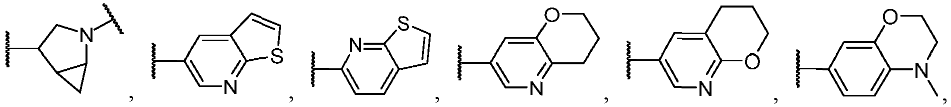

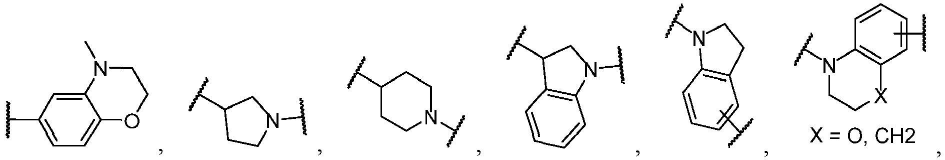

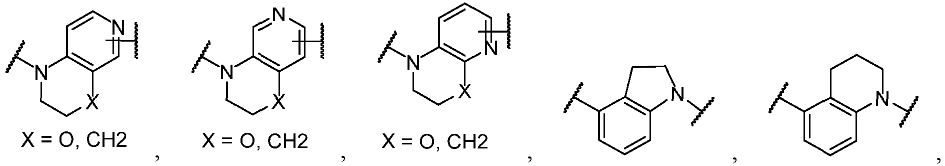

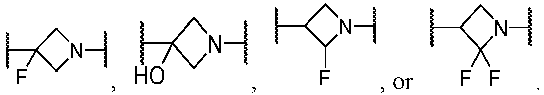

In a certain embodiment, B is

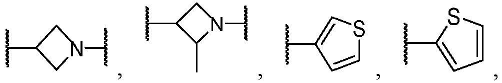

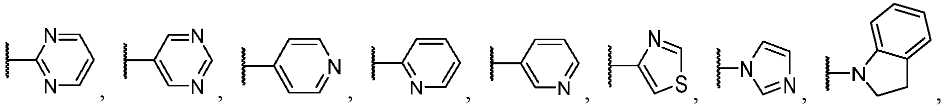

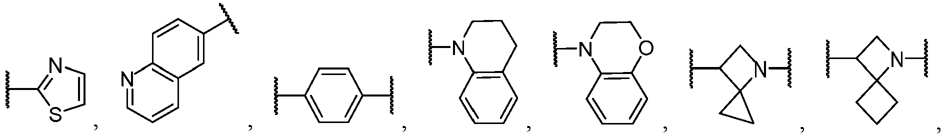

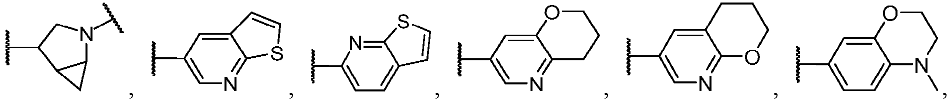

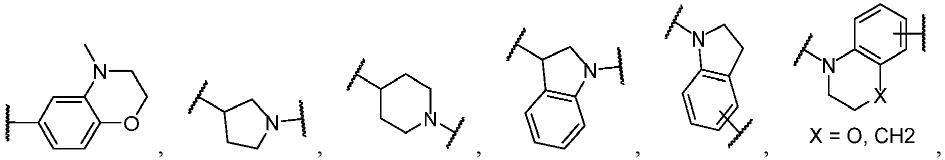

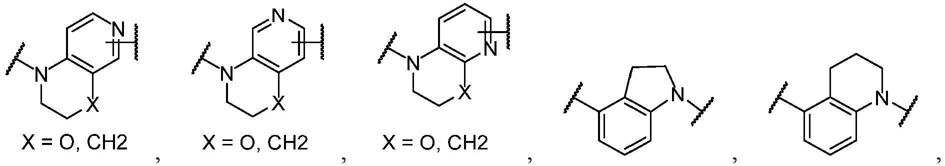

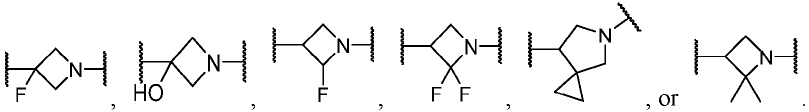

Preferably, B is

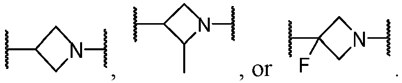

In a certain embodiments, B is 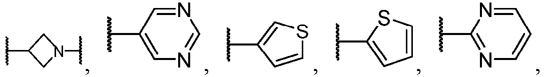 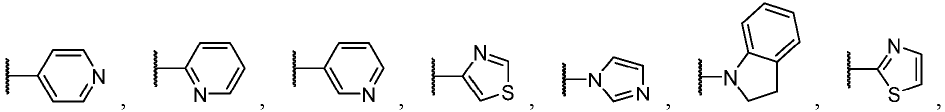 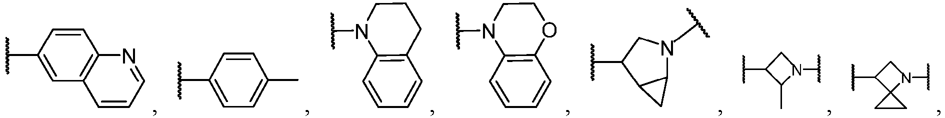 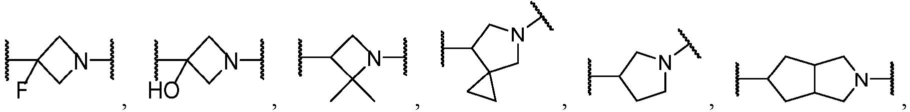 or

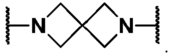

In a certain embodiment, D is hydrogen, methyl, 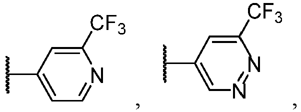 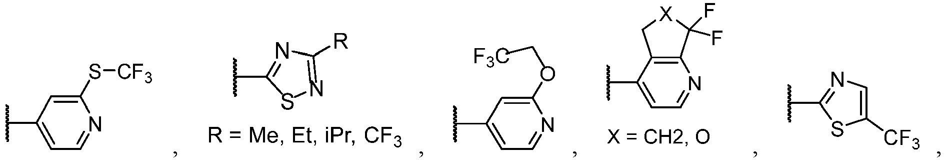 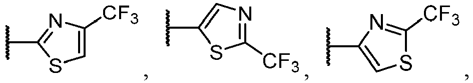 ethyl, isopropyl, cyclopropyl, triuorometyl, or

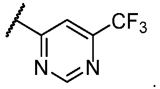

Preferably, D is

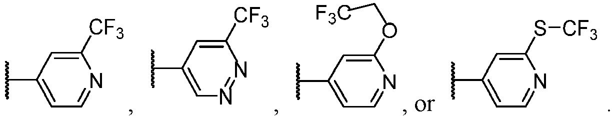

In a certain embodiment, D is hydrogen, methyl, 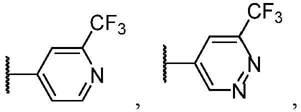 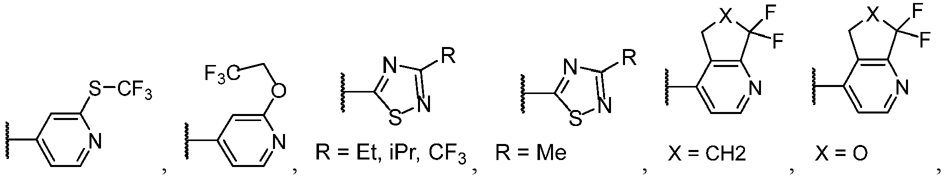 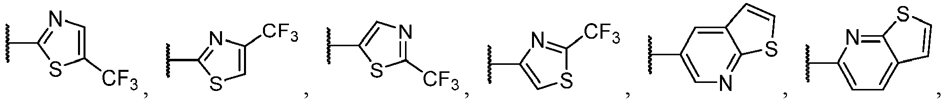 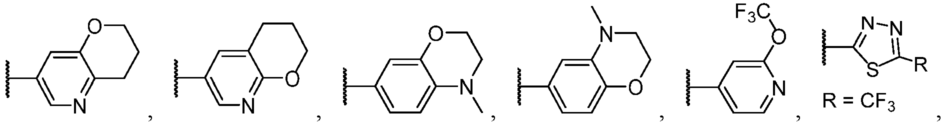 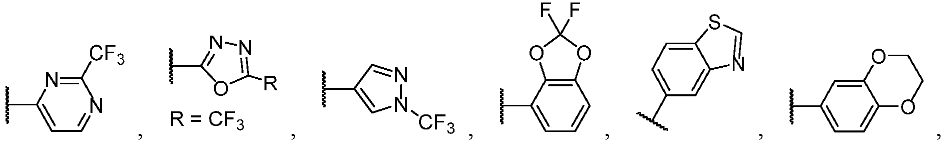 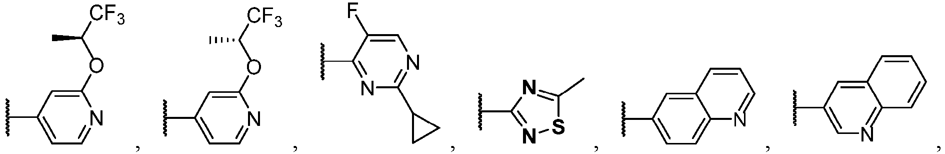 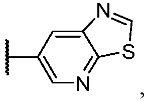 ethyl, isopropyl, cyclopropyl, trifluoromethyl, or

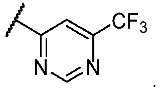

In a certain embodiment, the compound of formula I is any one of the following compounds:

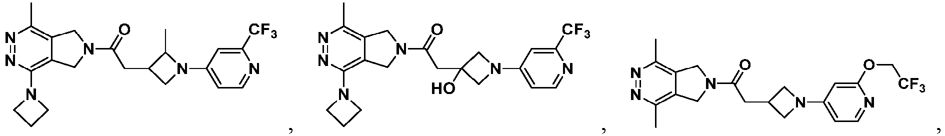

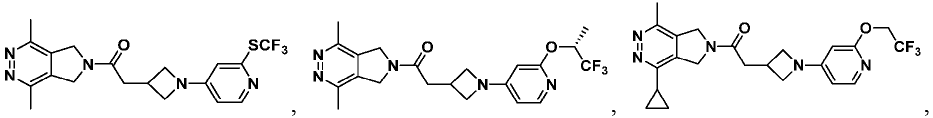

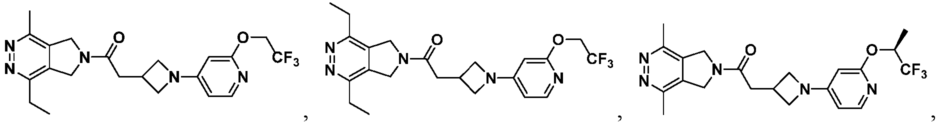

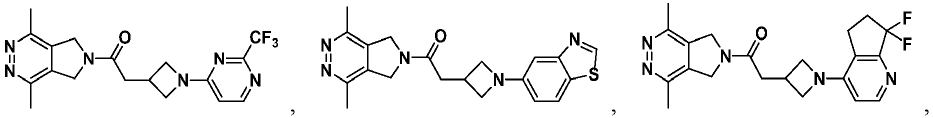

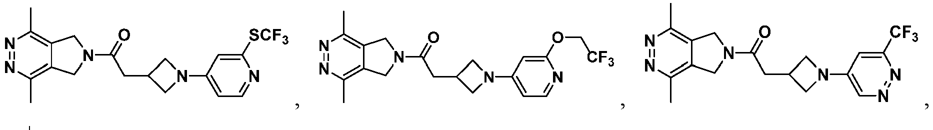

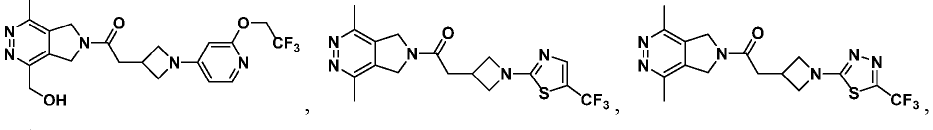

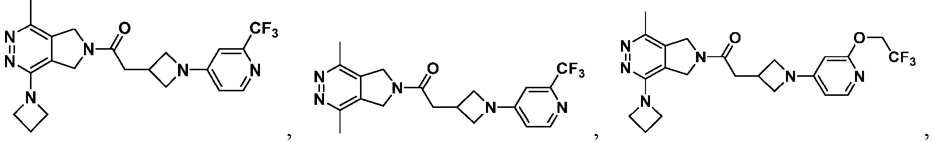

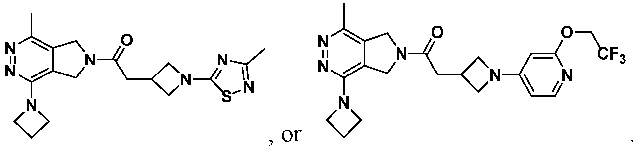

The present disclosure also provides a preparation method for the compound of formula I, which can be prepared by any one of the following schemes:
scheme (a): in an organic solvent, in the presence of a catalyst, performing a condensation reaction between a compound of formula II and a compound of formula III as follows to obtain the compound of formula I; 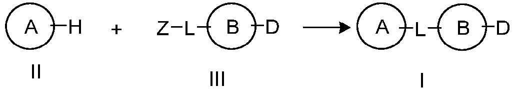
scheme (b): in an organic solvent, in the presence of a catalyst, performing a condensation reaction between a compound of formula IV and a compound of formula V as follows to obtain the compound of formula I; 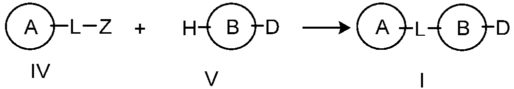
scheme (C): in an organic solvent, in the presence of a catalyst, performing a cyclization reaction between a compound of formula VI and a compound of formula VII as follows to obtain the compound of formula I; 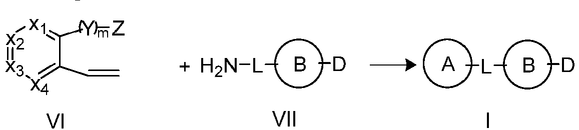
wherein *Z* is halogen (*e.g.,* chlorine or bromine), TsO-, hydroxyl, methoxy, ethoxy, *n-*propoxy, or isopropoxy; preferably, Z is hydroxyl, methoxy, ethoxy, n-propoxy, or isopropoxy;
A, L, B, D, X₁, X₂, X₃, X₄, Y, and m are as described in any one of the embodiments of the present disclosure.

The present disclosure also provides a preparation method for the compound of formula I, which can be prepared by the following scheme:
scheme (D): in an organic solvent, in the presence of a catalyst, performing a cyclization reaction between a compound of formula VIII and a compound of formula VIIII as follows to obtain the compound of formula I; 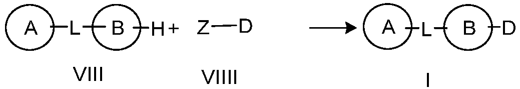
wherein Z, A, L, B, and D are as described in any one of the embodiments of the present disclosure.

The present disclosure also provides a pharmaceutical composition comprising a therapeutically effective amount of substance A and a pharmaceutical excipient; the substance A is the compound of formula I or the pharmaceutically acceptable salt thereof.

The present disclosure also provides a use of substance A in the manufacture of a positive allosteric modulator of a muscarinic receptor; the substance A is the compound of formula I or the pharmaceutically acceptable salt thereof, or the pharmaceutical composition.

The present disclosure also provides a use of substance A in the manufacture of a medicament for treating and/or preventing a muscarinic receptor-mediated disease; the substance A is the compound of formula I or the pharmaceutically acceptable salt thereof, or the pharmaceutical composition; preferably, the disease is Parkinson's disease, Alzheimer's disease, Huntington's disease, schizophrenia, drug addiction, or pain.

The present disclosure also provides a use of substance A in the manufacture of a medicament for treating Parkinson's disease, Alzheimer's disease, Huntington's disease, schizophrenia, drug addiction, or pain.

The positive and progressive effect of the present disclosure is that the compound of the present disclosure can be used as a positive allosteric modulator of the muscarinic receptor; the compound of the present disclosure can be used for the treatment of an M receptor (muscarinic receptor)-mediated (or M receptor-related) disease.

### Explanation of terms:

The term "-" means that the group is attached to the rest of the molecule through the site. For example, "CH₃O-" refers to alkoxy.

The term 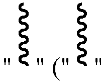 in 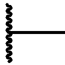 ) means that the structural moiety is attached to the rest of the molecule through the site.

The term "pharmaceutically acceptable" refers to being relatively non-toxic, safe, and suitable for patient use.

The term "pharmaceutically acceptable salt" refers to a salt obtained by reacting a compound with a pharmaceutically acceptable acid or base. When a compound contains a relatively acidic functional group, a base addition salt can be obtained by contacting the compound with a sufficient amount of a pharmaceutically acceptable base in a suitable inert solvent. The pharmaceutically acceptable base addition salt includes, but is not limited to, lithium salts, sodium salts, potassium salts, calcium salts, aluminum salts, magnesium salts, zinc salts, bismuth salts, ammonium salts, and diethanolamine salts. When a compound contains a relatively basic functional group, an acid addition salt can be obtained by contacting the compound with a sufficient amount of a pharmaceutically acceptable acid in a suitable inert solvent. The pharmaceutically acceptable acid includes inorganic acids and organic acids (*e.g*., trifluoroacetic acid, hydrochloric acid, formic acid). For details, please refer to Handbook of Pharmaceutical Salts: Properties, Selection, and Use (P. Heinrich Stahl, Camille G. Wermuth, 2011, 2nd Revised Edition), *e.g*., formates.

The term "pharmaceutical excipient" refers to all substances, other than the active pharmaceutical ingredient, that are contained in the pharmaceutical formulation, and is generally divided into two categories: excipients and additives. For details, please refer to the Pharmacopoeia of the People's Republic of China (2020 Edition) and Handbook of Pharmaceutical Excipients (Paul J Sheskey, Bruno C Hancock, Gary P Moss, David J Goldfarb, 2020, 9th Edition).

The term "treating" refers to the elimination of the cause of a disease or the alleviation of symptoms.

The term "preventing" refers to reducing the risk of developing a disease.

The term "patient" refers to any animal, typically a mammal, such as a human, in need of treatment or prevention of a disease. The mammal includes, but is not limited to, cattle, horses, sheep, pigs, cats, dogs, mice, rats, rabbits, guinea pigs, monkeys, humans, *etc.*

The term "therapeutically effective amount" refers to an amount of a compound administered to a patient that is sufficient to effectively treat a disease. The therapeutically effective amount will vary depending on the type of compound, type of disease, severity of disease, age of patient, *etc,* but can be adjusted by those skilled in the art as appropriate.

The expression "group B substituted by one or more groups A" means that one or more hydrogen atoms in group B are independently replaced by group A. When a plurality of groups A are present at the same time, their definitions are independent of each other and do not affect each other, unless otherwise specified.

The term "halogen" refers to fluorine, chlorine, bromine, or iodine.

The term "oxo" refers to =O, where an oxygen atom replaces two hydrogen atoms on the same atom, *e.g.,* a methylene group (-CH₂-) is oxidized to a carbonyl group (-C(=O)-).

The term "alkyl" refers to a linear or branched, saturated monovalent hydrocarbon group with a specified number of carbon atoms. For example, C₁-C₆ alkyl (C₁₋₆ alkyl) or C₄-C₂₀ alkyl (C₄₋₂₀ alkyl), preferably C₁-C₄ alkyl (C₁₋₄ alkyl) or C₉-C₁₅ alkyl (C₁₋₆ alkyl). Alkyl groups include, but are not limited to, methyl, ethyl, *n*-propyl, isopropyl, *n*-butyl, isobutyl, *sec-*butyl, or *tert*-butyl*.*

The definition of alkyl in the term "alkyl-O-" is as described above. Examples of alkyl-O- include C₁-C₆ alkyl-O- or C₁-C₄ alkyl-O-, more specifically, CH₃-O-, CH₃CH₂-O-, CH₃CH₂CH₂-O-, or CH₃CH(CH₃)-O-. Similarly, for the rest of the expressions in the form of "Rx-O-" in the present disclosure, the definition of Rx corresponds to its respective definition, e.g., the definition of cycloalkyl in "cycloalkyl-O-" is as described in the following term "cycloalkyl".

The definition of alkyl in the term "alkyl-S-" is as described above. Examples of alkyl-S- include C₁-C₆ alkyl-S- or C₁-C₄ alkyl-S-, more specifically, CH₃-S-, CH₃CH₂-S-, CH₃CH₂CH₂-S-, or CH₃CH(CH₃)-S-. Similarly, for the rest of the expressions in the form of "Rx-S-" in the present disclosure, the definition of Rx corresponds to its respective definition, *e.g.,* the definition of cycloalkyl in "cycloalkyl-S-" is as described in the following term "cycloalkyl".

The term "alkoxy" means that an oxygen atom is attached to one end of an alkyl group as a bond to form "alkyl-O-". The definition of "alkyl-O-" is as described above.

The term "alkylthio" means that a sulfur atom is attached to one end of an alkyl group as a bond to form "alkyl-S-". The definition of "alkyl-S-" is as described above.

The term "heteroaryl" refers to a cyclic, unsaturated monovalent group with a specified number of ring atoms (e.g., 5- to 12-membered, 5- to 10-membered, or 5- to 6-membered), a specified number of heteroatoms (e.g., 1, 2, or 3), and a specified type of heteroatoms (one or more kinds of N, O, and S), which is aromatic.

The term "heterocycloalkyl" refers to a saturated heterocycloalkyl group or a partially unsaturated monocyclic or polycyclic (e.g., a bridged, fused (condensed), or spiro system that is bicyclic, tricyclic, or more cyclic) heterocyclic group with a specified number of ring atoms *(e.g.,* 4- to 12-membered, 4- to 10-membered, 3- to 7-membered, 6- to 10-membered, 4- to 7-membered, or 5- to 6-membered), a specified number of heteroatoms (e.g., 1, 2, or 3), and a specified type of heteroatoms or heteroatom group (one or more kinds of N, O, S, S(=O), and S(=O)₂).

The term "cycloalkyl" means a saturated carbocyclic substituent which may be attached to the rest of the molecule by a single bond via any suitable carbon atom; C₃-C₇ cycloalkyl with 3 to 7 carbon atoms, preferably C₃-C₆ cycloalkyl with 3 to 6 carbon atoms, such as cyclopropyl, cyclobutyl, cyclopentyl, or cyclohexyl.

The term "aryl" refers to a cyclic, unsaturated monovalent hydrocarbon group with a specified number of carbon atoms (*e.g*., C₆-C₁₀). Examples of aryl groups include, but are not limited to, phenyl or naphthyl.

The term "heteroaryl" refers to a cyclic or unsaturated monovalent group with a specified number of ring atoms (e.g., 5- to 10-membered, 5- to 6-membered), a specified number of heteroatoms (e.g., 1, 2, or 3), and a specified type of heteroatoms (one or more kinds of N, O, and S), which is monocyclic or polycyclic, and (at least one ring/each ring) is aromatic. The heteroaryl group is attached to the rest of the molecule via a carbon atom or a heteroatom; the heteroaryl group is attached to the rest of the molecule via a ring with or without a heteroatom; the heteroaryl group is attached to the rest of the molecule via a ring with or without aromaticity.

Unless otherwise specified, the absolute configuration of a stereogenic center is represented by a wedged solid bond ( 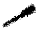 ) and a wedged dashed bond ( 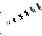 ), and when an atom is connected to its substituent by a wavy line ( 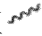 ), it denotes 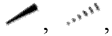 or a mixture thereof.

On the basis of not violating the common sense in the art, the above-mentioned preferred conditions can be arbitrarily combined to obtain preferred examples of the present disclosure.

The reagents and starting materials used in the present disclosure are commercially available.

### Abbreviations:

PMB: 4-Methoxybenzyl.
Boc: tert-Butyloxycarbonyl.
DBU: 1,8-Diazabicyclo[5.4.0]undec-7-ene.
NBS: N-Bromosuccinimide.
DIEA: N,N-Diisopropylethylamine.
DMF: N,N-Dimethylformamide.
Et: Ethyl.
Me: Methyl.
i-Pr: Isopropyl.
DMSO: Dimethyl sulfoxide.
HATU: 2-(7-Azabenzotriazol-1-yl)-*N,N,N',N'*-tetramethyluronium hexafluorophosphate.
PyBOP: 1*H*-Benzotriazol-1-yloxytripyrrolidinophosphonium hexafluorophosphate.
TEA: Triethylamine.
LiHMDS: Lithium bis(trimethylsilyl)amide.
NCS: N-Chlorosuccinimide.

### DETAILED DESCRIPTION OF THE PREFERRED EMBODIMENT

The present disclosure is further illustrated by way of examples below, but the present disclosure is not limited to the scope of the described examples. The experimental methods for which the specific conditions are not specified in the following examples are selected according to the conventional methods and conditions, or according to the commodity instructions.

### Intermediate A

### Synthetic route:

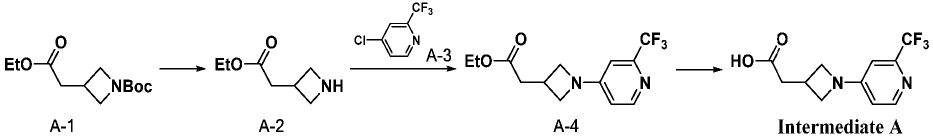

### Step 1

**A-1** (5.0 g, 20.5 mmol) was dissolved in dichloromethane (10 mL) and trifluoroacetic acid (10 mL), and the mixture was stirred at room temperature for 2 hours. The reaction mixture was concentrated under reduced pressure to obtain a trifluoroacetate of **A-2.** ¹H NMR (400 MHz, DMSO-*d*₆) δ 4.13-3.91 (m, 4H), 3.83-3.66 (m, 2H), 3.11-3.03 (m, 1H), 2.74-2.63 (m, 2H), 1.19-1.13 (m, 3H). ESI-MS calculated for C₇H₁₄NO₂ [M+H]⁺ = 144.1, found 144.2.

### Step 2

The trifluoroacetate of **A-2** (5.0 g, 19.4 mmol) and **A-3** (6.0 g, 33.5 mmol) were dissolved in dimethyl sulfoxide (10 mL), then triethylamine (13.4 g, 132.2 mmol) and cesium fluoride (5.0 g, 33.0 mmol) were sequentially added thereto, and the mixture was heated to 80°C and stirred for 18 hours. The reaction mixture was poured into saturated ammonium chloride solution (20 mL) and extracted with dichloromethane (50 mL × 1). The organic phase was dried over anhydrous sodium sulfate, filtered, and concentrated under reduced pressure to obtain a crude product containing the target compound, which was purified by silica gel column chromatography (petroleum ether/ethyl acetate, 3/2, v/v) to obtain **A-4.** ¹H NMR (400 MHz, DMSO-*d*₆) δ 8.21 (d, *J* = 5.64 Hz, 1H), 6.70 (d, *J* = 2.28 Hz, 1H), 6.53-6.48 (m, 1H), 4.17-4.04 (m, 4H), 3.70-3.67 (m, 2H), 3.10-3.01 (m, 1H), 2.76-2.72 (m, 2H), 1.21-1.17 (m, 3H). ESI-MS calculated for C₁₃H₁₆F₃N₂O₂ [M+H]⁺ = 289.1, found 289.2.

### Step 3

**A-4** (1.0 g, 3.5 mmol) was dissolved in tetrahydrofuran (20 mL) and water (4 mL), then lithium hydroxide (0.22 g, 5.2 mmol) was added thereto, and the mixture was stirred at room temperature for 2 hours. The pH of the reaction mixture was adjusted to 7, then water (50 mL) was added thereto, and the mixture was lyophilized to obtain a crude product containing intermediate **A,** which was directly used in the next reaction step. ¹H NMR (400 MHz, DMSO-*d*₆) δ 8.25 (d, *J* = 5.64 Hz, 1H), 6.72 (d, *J* = 2.26 Hz, 1H), 6.55 (dd, *J* = 5.64, 2.26 Hz, 1H), 4.20-4.12 (m, 2H), 3.74-3.64 (m, 2H), 3.14-3.04 (m, 1H), 2.54-2.50 (m, 2H). ESI-MS calculated for C₁₁H₁₁F₃N₂O₂ [M+H]⁺ = 261.1, found 261.0.

### Intermediate G

### Synthetic route:

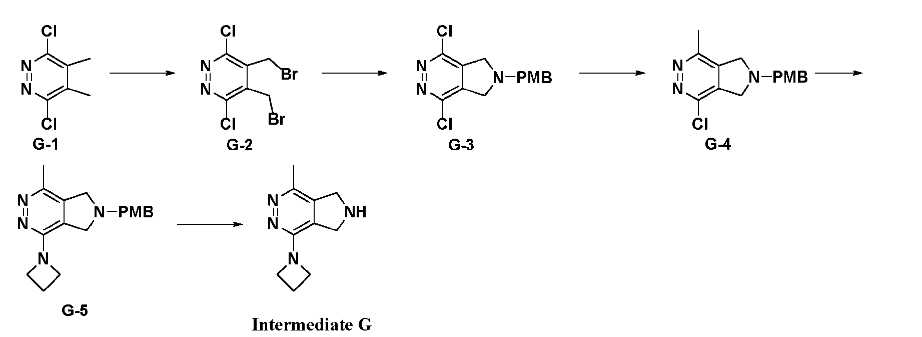

### Step 1

**G-1** (2.0 g, 11.3 mmol), NBS (4.2 g, 23.7 mmol), and azobisisobutyronitrile (370 mg, 2.26 mmol) were sequentially added to carbon tetrachloride (25 mL), and the reaction system was heated to 80°C under nitrogen atmosphere and stirred for 16 hours. The reaction mixture was cooled to room temperature, filtered, and concentrated under reduced pressure to obtain an oil. The oil was diluted with ethyl acetate (20 mL), washed with saturated sodium thiosulfate solution (15 mL × 2), dried over anhydrous sodium sulfate, filtered, and concentrated under reduced pressure to obtain a crude product containing the target compound, which was purified by silica gel column chromatography (petroleum ether/ethyl acetate, 9/1, v/v) to obtain **G-2.** ESI-MS calculated for C₆H₅Br₂Cl₂N₂ [M+H]⁺ = 332.8, found 332.0.

### Step 2

**G-2** (4.00 g, 11.9 mmol) and potassium carbonate (4.9 g, 35.8 mmol) were added to tetrahydrofuran (4 mL), then a solution of 4-methoxybenzylamine in tetrahydrofuran (1.64 g, 11.9 mmol) was added dropwise thereto at room temperature, and the mixture was stirred at room temperature for 1 hour. The reaction mixture was filtered and concentrated under reduced pressure to obtain a crude product containing the target compound, which was purified by silica gel column chromatography (petroleum ether/ethyl acetate, 9/1, v/v) to obtain **G-3.** ESI-MS calculated for C₁₄H₁₄Cl₂N₃O [M+H]⁺ = 310.0, found 310.0.

### Step 3

**G-3** (400 mg, 1.29 mmol), methylboronic acid (116 mg, 1.93 mmol), potassium carbonate (535 mg, 3.87 mmol), [1,1'-bis(diphenylphosphino)ferrocene]dichloropalladium(II) (189 mg, 0.26 mmol), and 1,4-dioxane (3 mL) were added to a 10 mL microwave tube, and the mixture was heated to 90°C under nitrogen atmosphere and stirred for 18 hours. The reaction mixture was cooled to room temperature, filtered, diluted with water (15 mL), and extracted with ethyl acetate (5 mL × 3). The organic phases were combined, washed with saturated brine (20 mL), dried over anhydrous sodium sulfate, filtered, and concentrated under reduced pressure to obtain a crude product containing the target compound, which was purified by silica gel column chromatography (petroleum ether/ethyl acetate, 9/1, v/v) to obtain **G-4.** ESI-MS calculated for C₁₅H₁₇ClN₃O [M+H]⁺ = 290.1, found 290.0.

### Step 4

**G-4** (130 mg, 0.45 mmol) and azetidine (2 mL) were added to a 10 mL microwave tube, and the mixture was heated to 90°C and stirred for 16 hours. The reaction mixture was cooled to room temperature, diluted with water (15 mL), and extracted with ethyl acetate (5 mL × 3). The organic phases were combined, washed with saturated brine (20 mL), dried over anhydrous sodium sulfate, filtered, and concentrated under reduced pressure to obtain a crude product containing the target compound, which was purified by silica gel column chromatography (petroleum ether/ethyl acetate, 4/1, v/v) to obtain G-5. ESI-MS calculated for C₁₈H₂₃N₄O [M+H]⁺ = 311.2, found 311.2.

### Step 5

**G-5** (120 mg, 0.39 mmol) and trifluoroacetic acid (2 mL) were added to a 5 mL microwave tube, and the mixture was heated to 100°C and stirred for 8 hours. The reaction mixture was cooled to room temperature, diluted with water (15 mL), added with saturated sodium bicarbonate solution to adjust the pH to 8, and extracted with ethyl acetate (5 mL × 3). The organic phases were combined, washed with saturated brine (20 mL), dried over anhydrous sodium sulfate, filtered, and concentrated under reduced pressure to obtain intermediate **G,** which was directly used in the next reaction step. ESI-MS calculated for C₁₀H₁₅N₄ [M+H]⁺ = 191.1, found 191.1.

### Intermediate I

### Synthetic route:

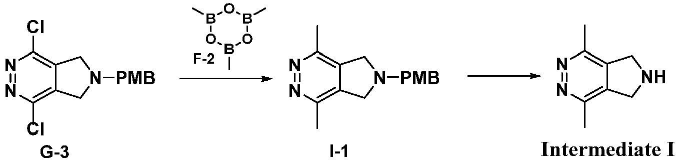

### Step 1

**G-3** (1.5 g, 4.84 mmol), [1,1'-bis(diphenylphosphino)ferrocene]dichloropalladium(II) (710 mg, 0.97 mmol), potassium carbonate (2.0 g, 14.5 mmol), **F-2** (1.21 g, 9.67 mmol), and 1,4-dioxane (10 mL) were heated to 90°C under nitrogen atmosphere and stirred for 18 hours. The reaction mixture was cooled to room temperature and concentrated under reduced pressure to obtain a crude product containing the target compound, which was purified by silica gel column chromatography (petroleum ether/ethyl acetate, 4/1, v/v) to obtain **I-1.** ESI-MS calculated for C₁₆H₂₀N₃O [M+H]⁺ = 270.2, found 270.1.

### Step 2

**I-1** (120 mg, 0.45 mmol) and trifluoroacetic acid (2 mL) were added to a 5 mL microwave tube, and the mixture was heated to 90°C and stirred for 8 hours. The reaction mixture was cooled to room temperature and concentrated under reduced pressure to obtain a trifluoroacetate containing intermediate **I,** which was directly used in the next reaction step. ESI-MS calculated for C₈H₁₂N₃ [M+H]⁺ = 150.1, found 150.1.

### Intermediate O

### Synthetic route:

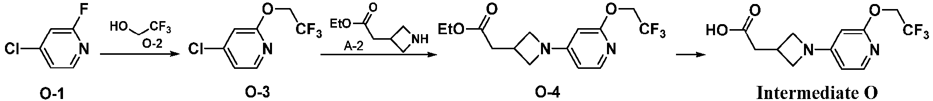

### Step 1

**O-1** (2.0 g, 15.2 mmol) was dissolved in DMF (20 mL), then **O-2** (1.52 g, 15.2 mmol) and sodium hydride (60%, 1.82 g, 45.6 mmol) were sequentially added thereto, and the mixture was heated to 60°C and stirred for 18 hours. The reaction mixture was cooled, then slowly added with saturated ammonium chloride solution (50 mL), and extracted with ethyl acetate (70 mL × 3). The organic phases were combined, washed with saturated brine (150 mL), dried over anhydrous sodium sulfate, filtered, and concentrated under reduced pressure to obtain a crude product containing the target compound, which was purified by silica gel column chromatography (petroleum ether/ethyl acetate, 4/1, v/v) to obtain **O-3.** ¹H NMR (400 MHz, DMSO-*d*₆) δ 8.21 (d, *J* = 5.50 Hz, 1H), 7.26 (dd, *J* = 5.50, 1.76 Hz, 1H), 7.22 (d, *J* = 1.74 Hz, 1H), 5.09-4.96 (m, 2H).

### Step 2

**O-3** (650 mg, 3.07 mmol) was dissolved in DMSO (6 mL), then intermediate **A-2** (1.1 g, 3.07 mmol), TEA (1.86 g, 18.4 mmol), and cesium fluoride (700 mg, 4.02 mmol) were sequentially added thereto, and the mixture was heated to 100°C and stirred for 18 hours. The reaction mixture was cooled, then added with water (30 mL), and extracted with dichloromethane (30 mL × 3). The organic phases were combined, washed with saturated brine (100 mL), dried over anhydrous sodium sulfate, filtered, and concentrated under reduced pressure to obtain a crude product containing the target compound, which was purified by silica gel column chromatography (petroleum ether/ethyl acetate, 4/1, v/v) to obtain **O-4.** ¹H NMR (400 MHz, DMSO-*d*₆) δ 7.76 (d, *J* = 5.76 Hz, 1H), 6.14 (dd, *J* = 5.80, 1.98 Hz, 1H), 5.77 (d, *J* = 1.96 Hz, 1H), 4.93-4.84 (m, 2H), 4.10-4.01 (m, 4H), 3.61-3.56 (m, 2H), 3.05-2.97 (m, 1H), 2.74-2.68 (m, 2H), 1.20-1.16 (m, 3H). ESI-MS calculated for C₁₄H₁₅F₃N₂O₃ [M+H]⁺ = 319.1, found 319.1.

### Step 3

**O-4** (150 mg, 0.47 mmol) was dissolved in tetrahydrofuran (4 mL) and water (1 mL), then lithium hydroxide (29 mg, 0.71 mmol) was added thereto, and the mixture was heated to 60°C and stirred for 1 hour. The reaction mixture was cooled, then added with hydrochloric acid (1 mol/L) to adjust the pH to 7, and lyophilized with water to obtain a crude product containing intermediate **O,** which was directly used in the next reaction step. ¹H NMR (400 MHz, DMSO-*d*₆) δ 7.73 (d, *J =* 5.80 Hz, 1H), 6.10 (dd, *J =* 5.76, 1.98 Hz, 1H), 5.72 (d, *J =* 1.96 Hz, 1H), 4.92-4.84 (m, 2H), 4.01-3.96 (m, 2H), 3.54-3.50 (m, 2H), 2.98-2.88 (m, 1H), 2.35-2.32 (m, 2H). ESI-MS calculated for C₁₂H₁₄F₃N₂O₃ [M+H]⁺ = 291.1, found 291.1.

### Intermediate P

### Synthetic route:

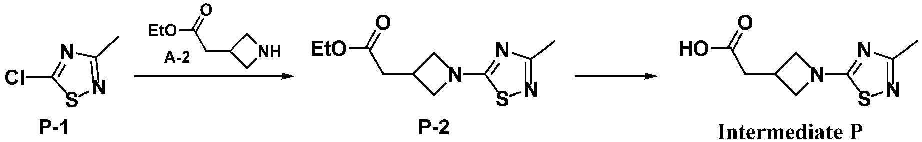

### Step 1

**P-1** (500 mg, 3.72 mmol) and A-2 (585 mg, 4.09 mmol) were dissolved in DMSO (5 mL), then TEA (1.50 g, 14.90 mmol) and cesium fluoride (152 mg, 3.72 mmol) were added thereto, and the mixture was heated to 100°C and stirred for 18 hours. The reaction mixture was added with saturated ammonium chloride solution (20 mL) and extracted with dichloromethane (20 mL × 3). The organic phases were combined, washed with saturated brine (50 mL), dried over anhydrous sodium sulfate, filtered, and concentrated under reduced pressure to obtain a crude product containing the target compound, which was purified by silica gel column chromatography (petroleum ether/ethyl acetate, 1/1, v/v) to obtain **P-2.** ¹H NMR (400 MHz, Chloroform-d) δ 4.35-4.27 (m, 2H), 4.19-4.12 (m, 2H), 3.90-3.82 (m, 2H), 3.30-3.17 (m, 1H), 2.73 (d, *J* = 7.84 Hz, 2H), 2.42 (s, 3H), 1.26 (t, *J* = 7.78 Hz, 3H). ESI-MS calculated for C₁₀H₁₆N₃O₂S [M+H]⁺ = 242.1, found 242.0.

### Step 2

**P-2** (200 mg, 0.83 mmol) was dissolved in methanol (2 mL) and water (1 mL), then lithium hydroxide monohydrate (35 mg, 0.83 mmol) was added thereto, and the mixture was stirred at room temperature for 3 hours. After the reaction was completed, the reaction mixture was added with dilute hydrochloric acid (1 mol/L) to adjust the pH to 5, concentrated under reduced pressure, and lyophilized to obtain a crude product containing intermediate **P,** which was directly used in the next reaction step. ¹H NMR (400 MHz, Chloroform-d) δ 4.39-4.29 (m, 2H), 3.97-3.86 (m, 2H), 3.35-3.22 (m, 1H), 2.78 (d, *J =* 8.00 Hz, 2H), 2.44 (s, 3H). ESI-MS calculated for C₈H₁₂N₃O₂S [M+H]⁺ = 214.1, found 214.0.

### Intermediate Q

### Synthetic route:

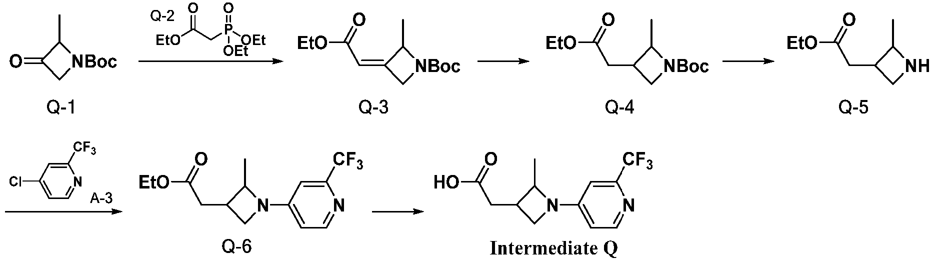

### Step 1

**Q-2** (726 mg, 3.24 mmol) was dissolved in tetrahydrofuran (20 mL), and the mixture was cooled to 0°C. Sodium hydride (60%, 130 mg, 3.24 mmol) was added thereto, and the mixture was stirred for 1 hour. **Q-1** (500 mg, 2.70 mmol) was then added thereto, and the mixture was stirred at room temperature for 18 hours. After the reaction was completed, the reaction mixture was quenched with saturated ammonium chloride solution (5 mL) and extracted with ethyl acetate (20 mL × 3). The organic phases were combined, washed with saturated brine (50 mL), dried over anhydrous sodium sulfate, filtered, and concentrated under reduced pressure to obtain a crude product containing the target compound, which was purified by silica gel column chromatography (petroleum ether/ethyl acetate, 4/1, v/v) to obtain **Q-3.** ¹H NMR (400 MHz, Chloroform-*d*) δ 5.69-5.63 (m, 1H), 5.06-5.01 (m, 0.3H), 4.81-4.76 (m, 0.7H), 4.68-4.50 (m, 2H), 4.15-4.03 (m, 2H), 1.40-1.36 (m, 12H), 1.24-1.15 (m, 3H).

### Step 2

**Q-3** (500 mg, 1.96 mmol) was dissolved in methanol (10 mL), and wet palladium on carbon (10%, 450 mg) was added thereto. The reaction system was replaced with hydrogen three times and stirred at room temperature for 1 hour. The reaction mixture was filtered, and the filtrate was concentrated under reduced pressure to obtain **Q-4.** ¹H NMR (400 MHz, Chloroform-d) δ 4.47-4.35 (m, 0.6H), 4.13 (q, *J =* 7.14 Hz, 2H), 4.06-3.87 (m, 1.4H), 3.52-3.48 (m, 1H), 3.02-2.87 (m, 0.6H), 2.61-2.53 (m, 2H), 2.45-2.40 (m, 0.4H) 1.44 (s, 9H), 1.40 (d, *J* = 6.20 Hz, 1.2H), 1.27-1.23 (m, 4.8H).

### Step 3

**Q-4** (250 mg, 0.97 mmol) was dissolved in dichloromethane (5 mL), then trifluoroacetic acid (3 mL) was added thereto, and the mixture was stirred at room temperature for 1 hour. The reaction mixture was concentrated under reduced pressure to obtain a crude product containing **Q-5,** which was directly used in the next reaction step. ESI-MS calculated for C₈H₁₆NO₂ [M+H]⁺ = 158.1, found 158.1.

### Step 4

The trifluoroacetate **of Q-5** (140 mg, 0.89 mmol) and **A-3** (173 mg, 0.95 mmol) were dissolved in dimethyl sulfoxide (5 mL), then TEA (385 mg, 3.80 mmol) and cesium fluoride (144 mg, 0.95 mmol) were sequentially added thereto, and the mixture was heated to 100°C and stirred for 18 hours. The reaction mixture was poured into water (20 mL) and extracted with dichloromethane (20 mL × 3). The organic phases were combined, washed with saturated brine (50 mL), dried over anhydrous sodium sulfate, filtered, and concentrated under reduced pressure to obtain a crude product containing the target compound, which was purified by silica gel column chromatography (petroleum ether/ethyl acetate, 2/3, v/v) to obtain **Q-6.** ¹H NMR (400 MHz, DMSO-*d*₆) δ 8.23-8.19 (m, 1H), 6.72 (s, 1H), 6.58 (d, *J* = 5.84 Hz, 1H), 4.53 (t, *J* = 7.24 Hz, 0.6H), 4.23-3.88 (m, 2.4H), 3.77-3.73 (m, 0.6H), 3.51-3.47 (m, 0.4H), 3.18-3.09 (m, 1H), 2.74-2.69 (m, 3H), 1.46 (d, *J* = 6.16 Hz, 1H), 1.31 (d, *J* = 6.52 Hz, 2H), 1.21-1.18 (m, 3H). ESI-MS calculated for C₁₄H₁₈F₃N₂O₂ [M+H]⁺ = 303.1, found 303.1.

### Step 5

**Q-6** (180 mg, 0.57 mmol) was dissolved in tetrahydrofuran (4 mL) and water (1 mL), then lithium hydroxide monohydrate (38 mg, 0.89 mmol) was added thereto, and the mixture was stirred at room temperature for 3 hours. After the reaction was completed, the reaction mixture was added with dilute hydrochloric acid (1 mol/L) to adjust the pH to 5, and lyophilized with water to obtain a crude product containing intermediate Q, which was directly used in the next reaction step. ESI-MS calculated for C₁₂H₁₄F₃N₂O₂ [M+H]⁺ = 275.1, found 275.0.

### Intermediate S

### Synthetic route:

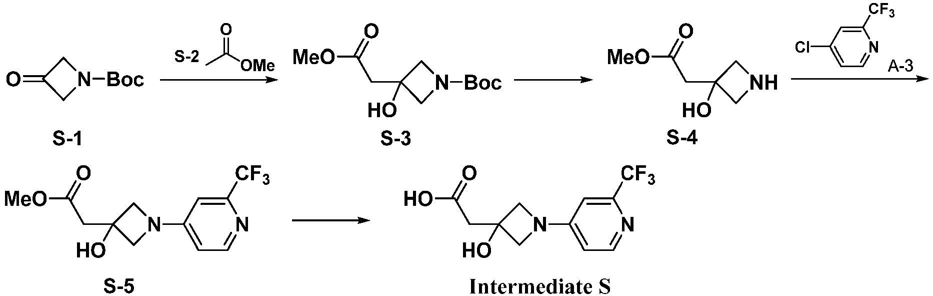

### Step 1

**S-2** (4.54 g, 61.3 mmol) was dissolved in tetrahydrofuran (120 mL), and the mixture was cooled to -78°C. A solution of LiHMDS in tetrahydrofuran (1.0 mol/L, 64.3 mL, 64.3 mmol) was slowly added dropwise thereto, and the mixture was stirred for 10 minutes. **S-1** (10 g, 58.4 mmol) was then added thereto, and the mixture was stirred for 15 minutes. The reaction system was warmed to 0°C, stirred for another 2 hours, added with ice water (100 mL), and extracted with ethyl acetate (100 mL × 2). The organic phases were combined, dried over anhydrous sodium sulfate, filtered, and concentrated under reduced pressure to obtain a crude product containing **S-3,** which was directly used in the next reaction step. ESI-MS calculated for C₁₁H₂₀NO₅ [M-56+H]⁺ = 190.1, found 190.1.

### Step 2

**S-3** (2.0 g, 8.15 mmol) was dissolved in dichloromethane (20 mL), then trifluoroacetic acid (20 mL) was added thereto, and the mixture was stirred at room temperature for 3 hours. The reaction mixture was concentrated under reduced pressure to obtain a trifluoroacetate of **S-4,** which was directly used in the next reaction step. ESI-MS calculated for C₆H₁₂NO₃ [M+H]⁺ = 146.1, found 146.2.

### Step 3

The trifluoroacetate of **S-4** (2.0 g, 13.8 mmol) and **A-3** (2.49 g, 13.8 mmol) were dissolved in dimethyl sulfoxide (10 mL), then TEA (5.58 g, 55.11 mmol) and cesium fluoride (2.1 g, 13.8 mmol) were sequentially added thereto, and the mixture was heated to 100°C and stirred for 18 hours. The reaction mixture was poured into water (80 mL) and extracted with dichloromethane (50 mL × 3). The organic phases were combined, washed with saturated brine (100 mL), dried over anhydrous sodium sulfate, filtered, and concentrated under reduced pressure to obtain a crude product containing the target compound, which was purified by silica gel column chromatography (petroleum ether/ethyl acetate, 3/7, v/v) to obtain **S-5.** ¹H NMR (400 MHz, DMSO-*d*₆) δ 8.22 (d, *J =* 5.68 Hz, 1H), 6.76 (d, *J =* 2.26 Hz, 1H), 6.62 -6.51 (m, 1H), 6.03 (s, 1H), 4.16-4.10 (m, 2H), 3.90-3.85 (m, 2H), 3.60 (s, 3H), 2.82 (s, 2H). ESI-MS calculated for C₁₂H₁₄F₃N₂O₃ [M+H]⁺ = 291.1, found 291.1.

### Step 4

**S-5** (130 mg, 0.45 mmol) was dissolved in tetrahydrofuran (2 mL) and water (0.2 mL), then lithium hydroxide monohydrate (28 mg, 0.68 mmol) was added thereto, and the mixture was stirred at room temperature for 2 hours. After the reaction was completed, the reaction mixture was added with dilute hydrochloric acid (1 mol/L) to adjust the pH to 5, and lyophilized with water to obtain a crude product containing intermediate S, which was directly used in the next reaction step. ESI-MS calculated for C₁₁H₁₂F₃N₂O₃ [M+H]⁺ = 277.1, found 277.0.

### Intermediate U

### Synthetic route:

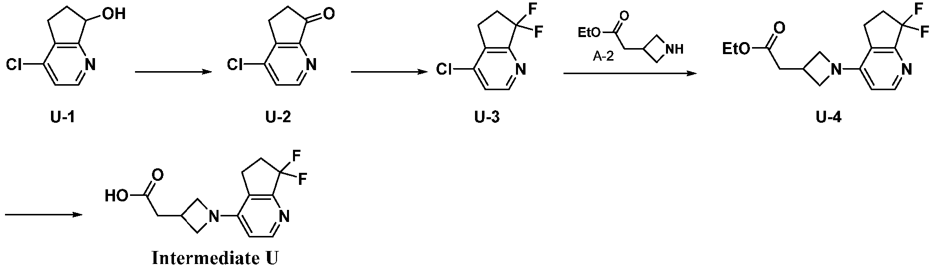

### Step 1

**U-1** (1.0 g, 5.90 mmol) and manganese dioxide (6.66 g, 76.65 mmol) were added to dichloromethane (15 mL), and the mixture was stirred at 25°C for 8 hours. The reaction mixture was filtered through diatomite, and the filtrate was concentrated under reduced pressure to obtain **U-2,** which was directly used in the next reaction step. ¹H NMR (400 MHz, Chloroform-*d*) δ 8.74-8.66 (m, 1H), 7.51 (d, *J =* 5.06 Hz, 1H), 3.24-3.17 (m, 2H), 2.88-2.80 (m, 2H). ESI-MS calculated for C₈H₇ClNO [M+H]⁺ = 168.0, found 168.0.

### Step 2

**U-2** (1.1 g, 6.56 mmol) was dissolved in dichloromethane (15 mL), then diethylaminosulfur trifluoride (3.7 g, 22.97 mmol) was added thereto at 0°C, and the mixture was warmed to room temperature and stirred for 18 hours. The reaction mixture was added with saturated sodium bicarbonate solution (20 mL) and extracted with dichloromethane (20 mL × 1). The organic phases were combined, dried over anhydrous sodium sulfate, filtered, and concentrated under reduced pressure to obtain a crude product containing the target compound, which was purified by silica gel column chromatography (petroleum ether/ethyl acetate, 7/3, v/v) to obtain **U-3.** ¹H NMR (400 MHz, Chloroform-*d*) δ 8.55 (d, *J =* 5.24 Hz, 1H), 7.39 (d, *J* = 5.22 Hz, 1H), 3.12-3.02 (m, 2H), 2.76-2.61 (m, 2H). ESI-MS calculated for C₈H₇ClF₂N [M+H]⁺ = 190.0, found 190.0.

### Step 3

Compounds **U-3** (300 mg, 1.58 mmol) and **A-2** (249 mg, 1.74 mmol) were dissolved in DMSO (10 mL), then TEA (641 mg, 6.33 mmol) and cesium fluoride (240 mg, 1.58 mmol) were added thereto, and the mixture was heated to 70°C and stirred for 18 hours. The reaction mixture was cooled to room temperature, then added with saturated ammonium chloride solution (50 mL), and extracted with ethyl acetate (40 mL × 3). The organic phases were combined, washed with saturated brine (100 mL), dried over anhydrous sodium sulfate, filtered, and concentrated under reduced pressure to obtain a crude product containing the target compound, which was purified by silica gel column chromatography (petroleum ether/ethyl acetate, 3/7, v/v) to obtain **U-4.** ¹H NMR (400 MHz, Chloroform-*d*) δ 8.24 (d, *J =* 5.58 Hz, 1H), 6.15 (d, *J =* 5.60 Hz, 1H), 4.36-4.30 (m, 2H), 4.16 (q, *J =* 7.12 Hz, 2H), 3.88-3.81 (m, 2H), 3.20-3.09 (m, 1H), 3.03-2.96 (m, 2H), 2.71 (d, *J* = 7.78 Hz, 2H), 2.62-2.48 (m, 2H), 1.27 (t, *J* = 7.06 Hz, 3H). ESI-MS calculated for C₁₅H₁₉F₂N₂O₂ [M+H]⁺ = 297.1, found 297.1.

### Step 4

**U-4** (70 mg, 0.24 mmol) was dissolved in tetrahydrofuran (2 mL) and water (0.4 mL), then lithium hydroxide monohydrate (10 mg, 0.24 mmol) was added thereto, and the mixture was stirred at room temperature for 3 hours. After the reaction was completed, the reaction mixture was added with dilute hydrochloric acid (1 mol/L) to adjust the pH to 5, and lyophilized with water to obtain a crude product containing intermediate **U,** which was directly used in the next reaction step. ESI-MS calculated for C₁₃H₁₅F₂N₂O₂ [M+H]⁺ = 269.1, found 269.1.

### Intermediate V

### Synthetic route:

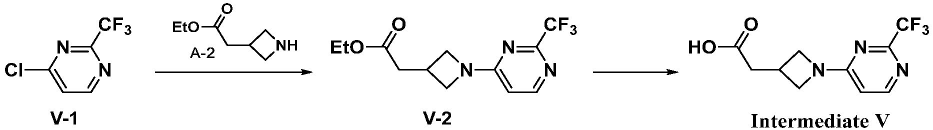

### Step 1

Compounds **V-1** (100 mg, 0.55 mmol) and **A-2** (157 mg, 0.55 mmol) were dissolved in DMSO (5 mL), then TEA (221 mg, 2.19 mmol) and cesium fluoride (80 mg, 0.55 mmol) were added thereto, and the mixture was heated to 100°C and stirred for 18 hours. The reaction mixture was cooled to room temperature, then added with saturated ammonium chloride solution (20 mL), and extracted with ethyl acetate (15 mL × 3). The organic phases were combined, washed with saturated brine (30 mL), dried over anhydrous sodium sulfate, filtered, and concentrated under reduced pressure to obtain a crude product containing the target compound, which was purified by silica gel column chromatography (petroleum ether/ethyl acetate, 2/3, v/v) to obtain **V-2.** ¹H NMR (400 MHz, Chloroform-d) δ 8.24 (d, *J* = 5.70 Hz, 1H), 6.23 (d, *J* = 5.86 Hz, 1H), 4.35-4.27 (m, 2H), 4.19-4.12 (m, 2H), 3.97-3.72 (m, 2H), 3.14-3.22 (m, 1H), 2.72 (d, *J* = 7.76 Hz, 2H), 1.28-1.24 (m, 3H). ESI-MS calculated for C₁₂H₁₅F₃N₃O₂ [M+H]⁺ = 290.3, found 290.2.

### Step 2

**V-2** (80 mg, 0.28 mmol) was dissolved in tetrahydrofuran (2 mL) and water (0.4 mL), then lithium hydroxide monohydrate (18 mg, 0.41 mmol) was added thereto, and the mixture was stirred at room temperature for 2 hours. After the reaction was completed, the reaction mixture was added with dilute hydrochloric acid (1 mol/L) to adjust the pH to 5, and lyophilized with water to obtain a crude product containing intermediate **V,** which was directly used in the next reaction step. ESI-MS calculated for C₁₀H₁₁F₃N₃O₂ [M+H]⁺ = 262.1, found 262.0.

### Intermediate X

### Synthetic route:

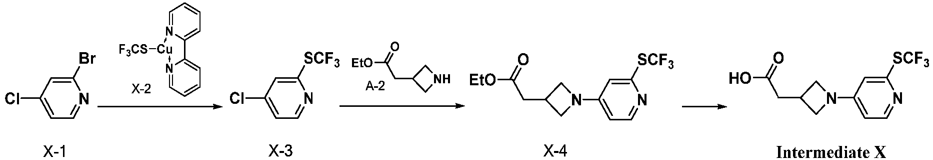

### Step 1

**X-1** (900 mg, 4.68 mmol) was dissolved in 1,4-dioxane (40 mL), then **X-2** (1.8 g, 5.61 mmol) was added thereto, and the mixture was heated to 90°C and stirred for 18 hours. The reaction mixture was cooled to room temperature, diluted with water (50 mL), and extracted with ethyl acetate (50 mL × 3). The organic phases were combined, dried over anhydrous sodium sulfate, filtered, and concentrated under reduced pressure to obtain a crude product containing the target compound, which was purified by silica gel column chromatography (petroleum ether/ethyl acetate, 1/0, v/v) to obtain **X-3.** ¹H NMR (400 MHz, Chloroform-*d*) δ 8.52 (d, *J* = 5.34 Hz, 1H), 7.58 (d, *J* = 1.82 Hz, 1H), 7.32 (dd, *J* = 5.30, 1.84 Hz, 1H).

### Step 2

**X-3** (100 mg, 0.47 mmol) and **A-2** (201 mg, 0.70 mmol) were dissolved in DMSO (3 mL), then TEA (142 mg, 1.40 mmol) and cesium fluoride (71 mg, 0.47 mmol) were added thereto, and the mixture was heated to 100°C and stirred for 18 hours. The reaction mixture was cooled to room temperature, then added with saturated ammonium chloride solution (10 mL), and extracted with dichloromethane (15 mL × 3). The organic phases were combined, washed with saturated brine (30 mL), dried over anhydrous sodium sulfate, filtered, and concentrated under reduced pressure to obtain a crude product containing the target compound, which was purified by silica gel column chromatography (petroleum ether/ethyl acetate, 7/3, v/v) to obtain **X-4.** ESI-MS calculated for C₁₃H₁₄F₃N₂O₂S [M+H]⁺ = 321.1, found 321.1.

### Step 3

**X-4** (150 mg, 2.50 mmol) was dissolved in tetrahydrofuran (3 mL) and water (1 mL), then lithium hydroxide monohydrate (30 mg, 0.70 mmol) was added thereto, and the mixture was heated to 40°C and stirred for 2 hours. After the reaction was completed, the reaction mixture was added with dilute hydrochloric acid (1 mol/L) to adjust the pH to 5, and lyophilized with water to obtain a crude product containing intermediate **X,** which was directly used in the next reaction step. ESI-MS calculated for C₁₁H₁₂F₃N₂O₂S [M+H]⁺ = 293.1, found 293.1.

### Intermediate AA

### Synthetic route:

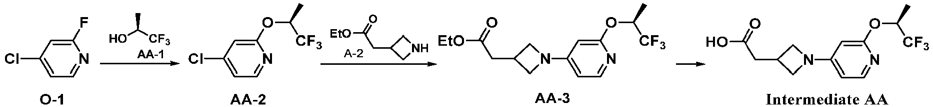

### Step 1

**AA-1** (1.4 g, 12.2 mmol) was dissolved in DMF (30 mL), and the mixture was cooled to 0°C. Sodium hydride (60%, 910 mg, 22.8 mmol) was added thereto, and the mixture was stirred for 30 minutes. **O-1** (2.0 g, 15.2 mmol) was then added thereto, and the mixture was stirred at room temperature for 18 hours. After the reaction was completed, the reaction mixture was quenched with saturated ammonium chloride solution (150 mL) and extracted with ethyl acetate (150 mL × 3). The organic phases were combined, washed with saturated brine (300 mL), dried over anhydrous sodium sulfate, filtered, and concentrated under reduced pressure to obtain a crude product containing the target compound, which was purified by silica gel column chromatography (petroleum ether/ethyl acetate, 19/1, v/v) to obtain **AA-2.** ¹H NMR (400 MHz, DMSO-*d*₆) δ 8.21 (d, *J =* 5.50 Hz, 1H), 7.25 (dd, *J =* 5.54, 1.78 Hz, 1H), 7.16 (d, *J* = 1.80 Hz, 1H), 5.95-5.86 (m, 1H), 1.45 (d, *J =* 6.68 Hz, 3H).

### Step 2

**AA-2** (400 mg, 1.77 mmol) and **A-2** (254 mg, 1.77 mmol) were dissolved in DMSO (15 mL), then TEA (1.08 g, 10.6 mmol) and cesium fluoride (404 mg, 2.66 mmol) were added thereto, and the mixture was heated to 100°C and stirred for 10 hours. The reaction mixture was cooled to room temperature, then added with saturated ammonium chloride solution (50 mL), and extracted with ethyl acetate (50 mL × 3). The organic phases were combined, washed with saturated brine (100 mL), dried over anhydrous sodium sulfate, filtered, and concentrated under reduced pressure to obtain a crude product containing the target compound, which was purified by silica gel column chromatography (petroleum ether/ethyl acetate, 3/17, v/v) to obtain **AA-3.** ¹H NMR (400 MHz, Chloroform-*d*) δ 7.75 (d, *J* = 5.74 Hz, 1H), 6.12 (dd, *J =* 5.82, 1.98 Hz, 1H), 5.87-5.80 (m, 1H), 5.73 (d, *J =* 1.96 Hz, 1H), 4.10-4.01 (m, 4H), 3.62-3.54 (m, 2H), 3.07-2.97 (m, 1H), 2.70 (d, *J* = 7.70 Hz, 2H), 1.38 (d, *J* = 6.54 Hz, 3H), 1.18 (t, *J* = 7.10 Hz, 3H). ESI-MS calculated for C₁₅H₂₀F₃N₂O₃ [M+H]⁺= 333.1, found 333.1.

### Step 3

**AA-3** (350 mg, 1.05 mmol) was dissolved in tetrahydrofuran (2 mL) and water (0.4 mL), then lithium hydroxide monohydrate (66 mg, 1.58 mmol) was added thereto, and the mixture was stirred at room temperature for 2 hours. After the reaction was completed, the reaction mixture was added with dilute hydrochloric acid (1 mol/L) to adjust the pH to 5, and filtered. The resulting solid was washed with water (3 mL) and dried to obtain intermediate **AA,** which was directly used in the next reaction step. ESI-MS calculated for C₁₃H₁₆F₃N₂O₃ [M+H]⁺ = 305.1, found 305.1.

### Intermediate AB

### Synthetic route:

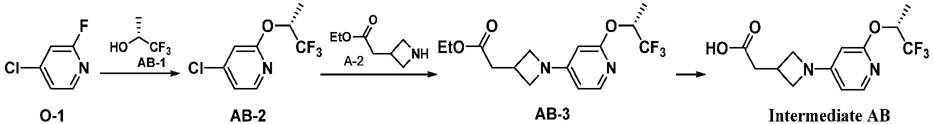

### Step 1

**AB-1** (208 mg, 1.82 mmol) was dissolved in DMF (4 mL), and the mixture was cooled to 0°C. Sodium hydride (60%, 137 mg, 3.43 mmol) was added thereto, and the mixture was stirred for 30 minutes. **O-1** (300 mg, 2.28 mmol) was then added thereto, and the mixture was stirred at room temperature for 18 hours. After the reaction was completed, the reaction mixture was quenched with saturated ammonium chloride solution (30 mL) and extracted with ethyl acetate (30 mL × 3). The organic phases were combined, washed with saturated brine (100 mL), dried over anhydrous sodium sulfate, filtered, and concentrated under reduced pressure to obtain a crude product containing the target compound, which was purified by silica gel column chromatography (petroleum ether/ethyl acetate, 19/1, v/v) to obtain **AB-2.** ¹H NMR (400 MHz, DMSO-*d*₆) δ 8.21 (d, *J* = 5.50 Hz, 1H), 7.25 (dd, *J* = 5.58, 1.76 Hz, 1H), 7.16 (d, *J* = 1.74 Hz, 1H), 5.93-5.85 (m, 1H), 1.45 (d, *J =* 6.68 Hz, 3H).

### Step 2

**AB-2** (200 mg, 0.89 mmol) and **A-2** (317 mg, 2.22 mmol) were dissolved in DMSO (5 mL), then TEA (538 mg, 5.32 mmol) and cesium fluoride (202 mg, 1.33 mmol) were added thereto, and the mixture was heated to 100°C and stirred for 10 hours. The reaction mixture was cooled to room temperature, then added with saturated ammonium chloride solution (20 mL), and extracted with ethyl acetate (20 mL × 3). The organic phases were combined, washed with saturated brine (50 mL), dried over anhydrous sodium sulfate, filtered, and concentrated under reduced pressure to obtain a crude product containing the target compound, which was purified by silica gel column chromatography (petroleum ether/ethyl acetate, 3/17, v/v) to obtain **AB-3.** ESI-MS calculated for C₁₅H₂₀F₃N₂O₃ [M+H]⁺ = 333.1, found 333.0.

### Step 3

**AB-3** (100 mg, 0.30 mmol) was dissolved in tetrahydrofuran (2 mL) and water (0.4 mL), then lithium hydroxide monohydrate (10 mg, 0.45 mmol) was added thereto, and the mixture was stirred at room temperature for 2 hours. After the reaction was completed, the reaction mixture was added with dilute hydrochloric acid (1 mol/L) to adjust the pH to 5, and filtered. The resulting solid was washed with water (3 mL) and dried to obtain intermediate **AB,** which was directly used in the next reaction step. ESI-MS calculated for C₁₃H₁₆F₃N₂O₃ [M+H]⁺ = 305.1, found 305.0.

### Intermediate AI

### Synthetic route:

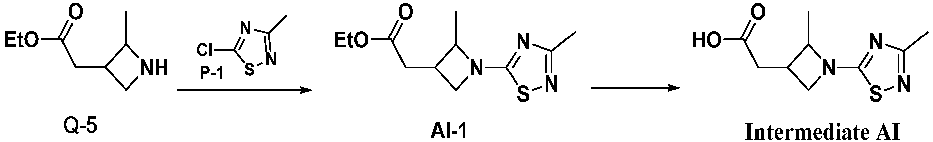

### Step 1

**Q-5** (109 mg, 0.69 mmol) and **P-1** (112 mg, 0.83 mmol) were dissolved in DMSO (5 mL), then TEA (280 mg, 2.77 mmol) and cesium fluoride (105 mg, 0.69 mmol) were added thereto, and the mixture was heated to 70°C and stirred for 18 hours. The reaction mixture was added with saturated ammonium chloride solution (20 mL) and extracted with ethyl acetate (20 mL × 3). The organic phases were combined, washed with saturated brine (50 mL), dried over anhydrous sodium sulfate, filtered, and concentrated under reduced pressure to obtain a crude product containing the target compound, which was purified by silica gel column chromatography (petroleum ether/ethyl acetate, 4/1, v/v) to obtain **AI-1.** ESI-MS calculated for C₁₁H₁₈N₃O₂S [M+H]⁺ = 256.1, found 256.1.

### Step 2

**AI-1** (90 mg, 0.35 mmol) was dissolved in tetrahydrofuran (2 mL) and water (1 mL), then lithium hydroxide monohydrate (18 mg, 0.42 mmol) was added thereto, and the mixture was stirred at room temperature for 3 hours. After the reaction was completed, the reaction mixture was added with dilute hydrochloric acid (1 mol/L) to adjust the pH to 5, concentrated under reduced pressure, and lyophilized to obtain a crude product containing intermediate **AI,** which was directly used in the next reaction step. ESI-MS calculated for C₉H₁₄N₃O₂S [M+H]⁺ = 228.1, found 228.0.

### Intermediate AK

### Synthetic route:

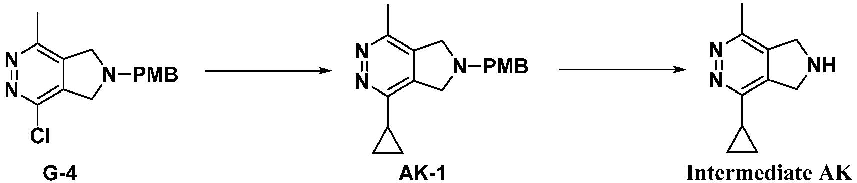

### Step 1

**G-4** (500 mg, 1.73 mmol), cyclopropylboronic acid (296 mg, 3.45 mmol), potassium carbonate (596 mg, 4.31 mmol), and [1,1'-bis(diphenylphosphino)ferrocene]dichloropalladium(II) (253 mg, 0.35 mmol) were added to 1,4-dioxane (6 mL), and the mixture was heated to 100°C under nitrogen atmosphere and stirred for 12 hours. The reaction mixture was cooled to room temperature, filtered, diluted with water (30 mL), and extracted with ethyl acetate (30 mL × 3). The organic phases were combined, washed with saturated brine (50 mL), dried over anhydrous sodium sulfate, filtered, and concentrated under reduced pressure to obtain a crude product containing the target compound, which was purified by silica gel column chromatography (petroleum ether/ethyl acetate, 2/3, v/v) to obtain **AK-1.** ¹H NMR (400 MHz, DMSO-*d*₆) δ 7.30 (d, *J* = 8.60 Hz, 2H), 6.92 (d, *J* = 8.62 Hz, 2H), 4.03-4.00 (m, 2H), 3.91-3.88 (m, 2H), 3.85 (s, 2H), 3.75 (s, 3H), 2.44 (s, 3H), 1.99-1.95 (m, 1H), 1.12-1.08 (m, 2H), 1.00-0.95 (m, 2H). ESI-MS calculated for C₁₈H₂₂N₃O [M+H]⁺ = 296.2, found 296.1.

### Step 2

**AK-1** (300 mg, 1.02 mmol) and trifluoroacetic acid (4 mL) were added to a microwave tube, and the mixture was heated to 90°C and stirred for 10 hours. The reaction mixture was cooled to room temperature and concentrated under reduced pressure to obtain a trifluoroacetate containing intermediate **AK,** which was directly used in the next reaction step. ESI-MS calculated for C₁₀H₁₄N₃ [M+H]⁺ = 176.1, found 176.0.

### Intermediate AO

### Synthetic route:

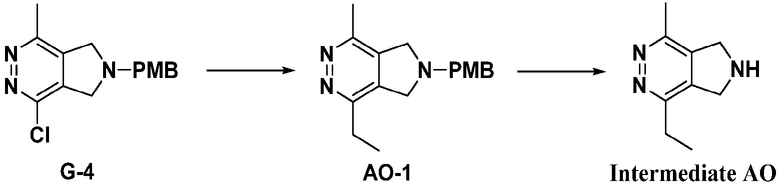

### Step 1

**G-4** (200 mg, 0.69 mmol), methanesulfonato(tricyclohexylphosphine)(2'-amino-1,1'-biphenyl-2-yl)palladium(II) (45 mg, 0.07 mmol), potassium phosphate (513 mg, 2.42 mmol), and ethylboric acid (153 mg, 2.07 mmol) were added to toluene (5 mL) and water (1 mL), and the mixture was heated to 100°C under nitrogen atmosphere and stirred for 12 hours. The reaction mixture was cooled to room temoerature. diluted with water (20 mL), and extracted with ethyl acetate (20 mL × 3). The organic phases were combined, dried over anhydrous sodium sulfate, and concentrated under reduced pressure to obtain a crude product containing the target compound, which was purified by silica gel column chromatography (petroleum ether/ethyl acetate, 2/3, v/v) to obtain **AO-1.** ¹H NMR (400 MHz, DMSO-*d*₆) δ 7.29 (d, *J =* 8.54 Hz, 2H), 6.92 (d, *J =* 8.52 Hz, 2H), 3.96-3.85 (m, 6H), 3.75 (s, 3H), 2.80 (q, *J =* 7.58 Hz, 2H), 2.47 (s, 3H), 1.20 (t, *J* = 7.58 Hz, 3H). ESI-MS calculated for C₁₇H₂₂N₃O [M+H]⁺ = 284.2, found 284.1.

### Step 2

**AO-1** (150 mg, 0.53 mmol) and trifluoroacetic acid (4 mL) were added to a microwave tube, and the mixture was heated to 90°C and stirred for 10 hours. The reaction mixture was cooled to room temperature and concentrated under reduced pressure to obtain a trifluoroacetate containing intermediate **AO,** which was directly used in the next reaction step. ESI-MS calculated for C₉H₁₄N₃ [M+H]⁺ = 164.1, found 164.0.

### Intermediate AP

### Synthetic route:

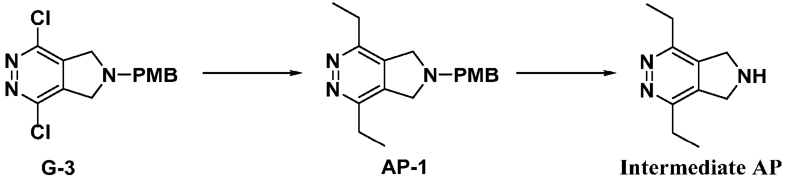

### Step 1

**G-3** (200 mg, 0.64 mmol), methanesulfonato(tricyclohexylphosphine)(2'-amino-1,1'-biphenyl-2-yl)palladium(II) (42 mg, 0.06 mmol), potassium phosphate (479 mg, 2.26 mmol), and ethylboric acid (285 mg, 3.87 mmol) were added to toluene (5 mL) and water (1 mL), and the mixture was heated to 100°C under nitrogen atmosphere and stirred for 18 hours. The reaction mixture was cooled to room temperature, diluted with water (20 mL), and extracted with ethyl acetate (20 mL × 3). The organic phases were combined, dried over anhydrous sodium sulfate, and concentrated under reduced pressure to obtain a crude product containing the target compound, which was purified by silica gel column chromatography (dichloromethane/methanol, 9/1, v/v) to obtain **AP-1.** ¹H NMR (400 MHz, DMSO-*d*₆) δ 7.31 (d, *J* = 8.60 Hz. 2H) 6.91 (d, *J* = 8.62 Hz. 2H) 3.95 (s, 3H) 3.85 (s, 2H), 3.75 (s, 4H), 2.80 (q, *J* = 7.58 Hz, 4H), 1.21 (t, *J* = 7.60 Hz, 6H). ESI-MS calculated for C₁₈H₂₄N₃O [M+H]⁺ = 298.2, found 298.1.

### Step 2

**AP-1** (150 mg, 0.50 mmol) and trifluoroacetic acid (4 mL) were added to a microwave tube, and the mixture was heated to 90°C and stirred for 10 hours. The reaction mixture was cooled to room temperature and concentrated under reduced pressure to obtain a trifluoroacetate containing intermediate **AP,** which was directly used in the next reaction step. ESI-MS calculated for C₁₀H₁₆N₃ [M+H]⁺ = 178.1, found 178.0.

### Intermediate AR

### Synthetic route:

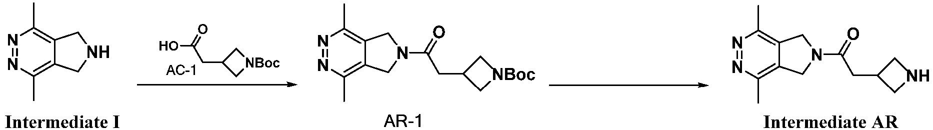

### Step 1

Intermediate **I** (800 mg, 5.36 mmol) and intermediate **AC-1** (1.15 g, 5.36 mmol) were dissolved in DMF (10 mL), then DIEA (2.08 g, 16.1 mmol) was added thereto, and the mixture was stirred at room temperature for 30 minutes. HATU (3.06 g, 8.04 mmol) was then added thereto, and the mixture was stirred at room temperature for another 1 hour. After the reaction was completed, the reaction mixture was diluted with water (100 mL) and extracted with ethyl acetate (80 mL × 3). The organic phases were combined, washed with saturated brine (150 mL), dried over anhydrous sodium sulfate, filtered, and concentrated under reduced pressure to obtain a crude product containing the target compound, which was purified by silica gel column chromatography (dichloromethane/methanol, 4/1, v/v) to obtain **AR-1.** ESI-MS calculated for C₁₈H₂₇N₄O₃ [M+H]⁺ = 347.3, found 347.2.

### Step 2

**AR-1** (1.00 g, 2.89 mmol) was dissolved in dichloromethane (6 mL), then trifluoroacetic acid (2 mL) was added thereto, and the mixture was stirred at room temperature for 3 hours. The reaction mixture was concentrated under reduced pressure to obtain a crude product containing intermediate **AR,** which was directly used in the next reaction step. ESI-MS calculated for C₁₃H₁₉N₄O [M+H]⁺ = 247.2, found 247.1.

### Preparation and synthesis of products

### Example 12

### Synthetic route:

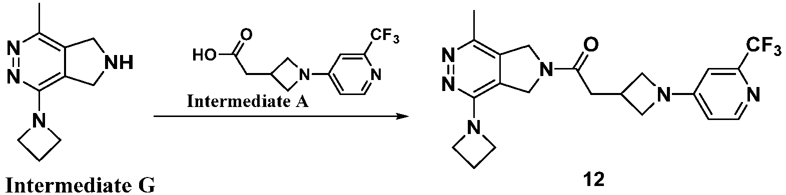

### Step 1

Intermediate **G** (50 mg, 0.26 mmol), HATU (149.9 mg, 0.39 mmol), DIEA (0.13 mL, 0.79 mmol), and intermediate **A** (136.8 mg, 0.53 mmol) were added to tetrahydrofuran (2 mL), and the mixture was stirred at room temperature for 3 hours. The reaction mixture was filtered and concentrated under reduced pressure to obtain a crude product containing the target compound, which was purified by preparative high performance liquid chromatography (chromatographic column: Waters-Xbridge-C18-10 µm-19 * 250 mm, mobile phase: acetonitrile-10 mmol/L ammonium bicarbonate aqueous solution, gradient: 20 to 50%, retention time: 9 minutes) to obtain compound **12.** ¹H NMR (400 MHz, DMSO-*d*₆) δ 8.21 (d, *J* = 5.66 Hz, 1H), 6.74-6.70 (m, 1H), 6.56-6.51 (m, 1H), 4.88-4.84 (m, 1H), 4.81-4.77 (m, 1H), 4.66-4.61 (m, 1H), 4.58-4.55 (m, 1H), 4.19-4.14 (m, 2H), 4.13-4.08 (m, 4H), 3.73-3.65 (m, 2H), 3.17-3.06 (m, 1H), 2.89-2.80 (m, 2H), 2.40 (s, 3H), 2.38-2.29 (m, 2H). ESI-MS calculated for C₂₁H₂₄F₃N₆O [M+H]⁺ = 433.2, found 433.2.

### Example 24

### Synthetic route:

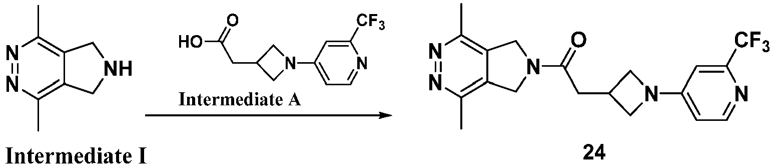

### Step 1

The trifluoroacetate of intermediate **I** (100 mg, 0.67 mmol) and intermediate **A** (262 mg, 1.01 mmol) were dissolved in tetrahydrofuran (4 mL), then DIEA (260 mg, 2.01 mmol) and HATU (382 mg, 1.01 mmol) were added thereto, and the mixture was stirred at room temperature for 3 hours. The reaction mixture was concentrated under reduced pressure to obtain a crude product containing the target compound, which was purified by preparative high performance liquid chromatography (chromatographic column: Waters-Xbridge C18 10 µm, 19 * 250 mm, mobile phase: acetonitrile-10 mmol/L ammonium bicarbonate aqueous solution, gradient: 17 to 47%, retention time: 9 minutes) to obtain compound **24.** ¹H NMR (400 MHz, DMSO-*d*₆) δ 8.21 (d, *J =* 5.66 Hz, 1H), 6.72 (d, *J =* 2.24 Hz, 1H), 6.54 (dd, *J =* 5.66, 2.24 Hz, 1H), 4.95-4.90 (m, 2H), 4.76-4.69 (m, 2H), 4.22-4.15 (m, 2H), 3.75-3.68 (m, 2H), 3.17-3.10 (m, 1H), 2.90-2.82 (m, 2H), 2.57-2.52 (m, 6H). ESI-MS calculated for C₁₉H₂₁F₃N₅O [M+H]⁺ = 392.2, found 392.2.

### Example 47

### Synthetic route:

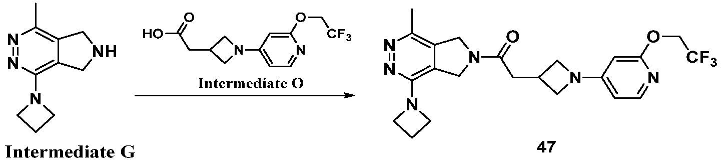

### Step 1

Intermediate **G** (30 mg, 0.16 mmol) and intermediate **O** (45 mg, 0.16 mmol) were dissolved in DMF (5 mL), then DIEA (122 mg, 0.95 mmol) was added thereto, and the mixture was stirred at room temperature for 30 minutes. HATU (72 mg, 0.19 mmol) was then added thereto, and the mixture was stirred at room temperature for another 1 hour. The reaction mixture was concentrated under reduced pressure to obtain a crude product containing the target compound, which was purified by preparative high performance liquid chromatography (chromatographic column: Waters-Xbridge-C18, 10 µm, 19 * 250 mm, mobile phase: acetonitrile-10 mmol/L ammonium bicarbonate aqueous solution, gradient: 32 to 62%, retention time: 9 minutes) to obtain compound **47.** ¹H NMR (400 MHz, DMSO-*d*₆) δ 7.76 (d, *J* = 5.78 Hz, -4.06 1H), 6.17-6.13 3.65-3.56 5.79 (t, *J* = 1.86 - Hz, 1H), 4.94-4.77 - (m, 4H), 4.64-4.56 (m, 2H), 4.14-4.06 (m,6H), 3.65-3.56 (m, 2H) 3.13-3.02 (m, 1H), 2.85-2.82 (m, 2H), 2.40 (s, 3H), 2.41-2.33 (m, 2H). ESI-MS calculated for C₂₂H₂₆F₃N₆O₂ [M+H]⁺ = 463.2, found 463.2.

### Example 50

### Synthetic route:

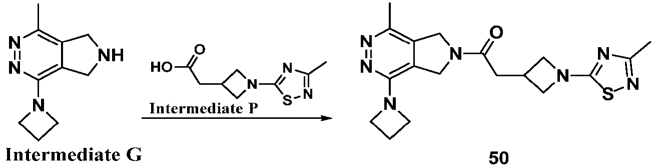

### Step 1

Intermediate **G** (30 mg, 0.16 mmol) and intermediate **P** (41 mg, 0.19 mmol) were dissolved in DMF (5 mL), then DIEA (62 mg, 0.47 mmol) was added thereto, and the mixture was stirred at room temperature for 30 minutes. HATU (90 mg, 0.24 mmol) was then added thereto, and the mixture was stirred at room temperature for another 2 hours. The reaction mixture was concentrated under reduced pressure to obtain a crude product containing the target compound, which was purified by preparative high performance liquid chromatography (chromatographic column: Waters-Xbridge-C18, 10 µm, 19 * 250 mm, mobile phase: acetonitrile-10 mmol/L ammonium bicarbonate aqueous solution, gradient: 9 to 39%, retention time: 9 minutes) to obtain compound **50.** ¹H NMR (400 MHz, DMSO-*d*₆) δ 4.85-4.74 (m, 2H), 4.65-4.51 (m, 2H), 4.25-4.20 (m, 2H), 4.15-4.07 (m, 4H), 3.83-3.76 (m, 2H), 3.25-3.15 (m, 1H), 2.92-2.84 (m, 2H), 2.40 (s, 3H), 2.38-2.31 (m, 2H), 2.28 (s, 3H). ESI-MS calculated for C₁₈H₂₄N₇OS [M+H]⁺ = 386.2, found 386.2.

### Example 51

### Synthetic route:

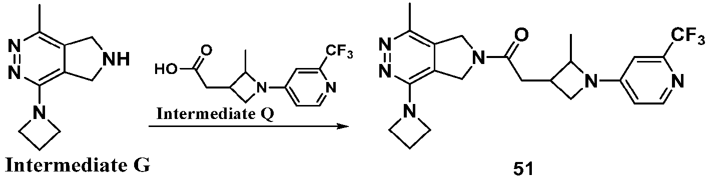

### Step 1

Intermediate **G** (30 mg, 0.16 mmol) and intermediate **Q** (43 mg, 0.11 mmol) were dissolved in DMF (3 mL), then DIEA (122 mg, 0.95 mmol) was added thereto, and the mixture was stirred at room temperature for 30 minutes. HATU (72 mg, 0.19 mmol) was then added thereto, and the mixture was stirred at room temperature for another 2 hours. The reaction mixture was concentrated under reduced pressure to obtain a crude product containing the target compound, which was purified by preparative high performance liquid chromatography (chromatographic column: Waters-Xbridge-C18, 10 µm, 19 * 250 mm, mobile phase: acetonitrile-10 mmol/L ammonium bicarbonate aqueous solution, gradient: 22 to 52%, retention time: 9 minutes) to obtain compound **51.** ¹H NMR (400 MHz, DMSO-*d*₆) δ 8.20 (d, *J = 5.70* Hz, 1H), 6.70 (s, 1H), 6.58 (d, *J* = 5.64 Hz, 1H), 4.92-4.84 (m, 2H), 4.66-4.51 (m, 3H), 4.15-4.02 (m, 4H), 4.01-3.96 (m, 1H), 3.76-3.71 (m, 1H), 3.24-3.20 (m, 1H), 2.89-2.77 (m, 2H), 2.42 (s, 3H), 2.39-2.34 (m, 2H), 1.37-1.34 (m, 3H). ESI-MS calculated for C₂₂H₂₆F₃N₆O [M+H]⁺ = 447.2, found 447.1.

### Example 56

### Synthetic route:

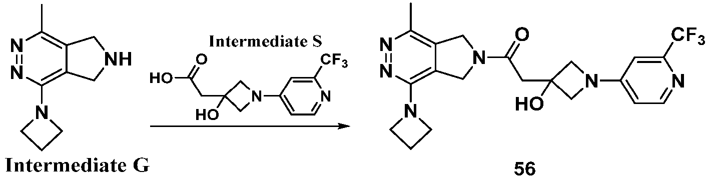

### Step 1

Intermediate **T** (20 mg, 0.11 mmol) and intermediate **S** (29 mg, 0.11 mmol) were dissolved in DMF (3 mL), then DIEA (55 mg, 0.42 mmol) was added thereto, and the mixture was stirred at room temperature for 30 minutes. HATU (48 mg, 0.13 mmol) was then added thereto, and the mixture was stirred at room temperature for another 1 hour. The reaction mixture was concentrated under reduced pressure to obtain a crude product containing the target compound, which was purified by preparative high performance liquid chromatography (chromatographic column: Waters-Xbridge-C18, 10 µm, 19 * 250 mm, mobile phase: acetonitrile-10 mmol/L ammonium bicarbonate aqueous solution, gradient: 17 to 47%, retention time: 9 minutes) to obtain compound **56.** ¹H NMR (400 MHz, DMSO-*d*₆) δ 8.22 (d, *J* = 5.68 Hz, 1H), 6.76 (t, *J =* 2.52 Hz, 1H), 6.58-6.56 (m, 1H), 5.93-5.90 (m, 1H), 4.91-4.82 (m, 2H), 4.65-4.57 (m, 2H), 4.19-4.15 (m, 2H), 4.13-4.09 (m, 4H), 3.90-3.87 (m, 2H), 2.92-2.88 (m, 2H), 2.41-2.39 (m, 3H), 2.36-2.32 (m, 2H). ESI-MS calculated for C₂₁H₂₄F₃N₆O₂ [M+H]⁺ = 449.2, found 449.1.

### Example 67

### Synthetic route:

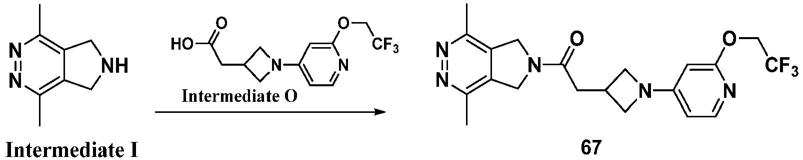

### Step 1

The trifluoroacetate of intermediate **I** (80 mg, 0.54 mmol) and intermediate **O** (156 mg, 0.54 mmol) were dissolved in DMF (5 mL), then DIEA (209 mg, 1.62 mmol) was added thereto, and the mixture was stirred at room temperature for 30 minutes. HATU (265 mg, 0.70 mmol) was then added thereto, and the mixture was stirred at room temperature for another 1 hour. The reaction mixture was concentrated under reduced pressure to obtain a crude product containing the target compound, which was purified by preparative high performance liquid chromatography (chromatographic column: Waters-Xbridge-C18, 10 µm, 19 * 250 mm, mobile phase: acetonitrile-10 mmol/L ammonium bicarbonate aqueous solution, gradient: 26 to 56%, retention time: 9 minutes) to obtain compound **67.** ¹H NMR (400 MHz, DMSO-*d*₆) δ 7.76 (d, *J* = 5.78 Hz, 1H), 6.15 (dd, *J* = 5.82, 1.94 Hz, 1H), 5.78 (d, *J* = 1.94 Hz, 1H), 5.01-4.77 (m, 4H), 4.71-4.68 (m, 2H), 4.11-4.06 (m, 2H), 3.63-3.58 (m, 2H), 3.13-3.05 (m, 1H), 2.84 (d, *J* = 7.62 Hz, 2H), 2.55-2.53 (m, 6H). ESI-MS calculated for C₂₀H₂₃F₃N₅O₂ [M+H]⁺ = 422.2, found 422.2.

### Example 68

### Synthetic route:

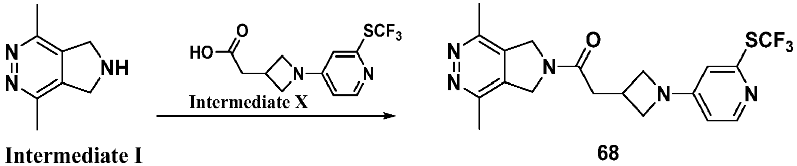

### Step 1

The trifluoroacetate of intermediate **I** (54 mg, 0.36 mmol) and intermediate **X** (70 mg, 0.24 mmol) were dissolved in DMF (5 mL), then DIEA (93 mg, 0.72 mmol) was added thereto, and the mixture was stirred at room temperature for 30 minutes. HATU (128 mg, 0.34 mmol) was then added thereto, and the mixture was stirred at room temperature for another 1 hour. The reaction mixture was concentrated under reduced pressure to obtain a crude product containing the target compound, which was purified by preparative high performance liquid chromatography (chromatographic column: Waters-Xbridge-C18, 10 µm, 19 * 250 mm, mobile phase: acetonitrile-10 mmol/L ammonium bicarbonate aqueous solution, gradient: 25 to 55%, retention time: 9 minutes) to obtain compound **68.** ¹H NMR (400 MHz, DMSO-*d*₆) δ 8.10 (d, *J* = 5.72 Hz, 1H), 6.59 (d, *J* = 2.18 Hz, 1H), 6.40 (dd, *J* = 5.76, 2.18 Hz, 1H), 4.93-4.91 (m, 2H), 4.70-4.67 (m, 2H), 4.17-4.11 (m, 2H), 3.69-3.65 (m, 2H), 3.15-3.09 (m, 1H), 2.86 (d, *J =* 7.68 Hz, 2H), 2.55-2.53 (m, 6H). ESI-MS calculated for C₁₉H₂₁F₃N₅OS [M+H]⁺ = 424.1, found 424.1.

### Example 89

### Synthetic route:

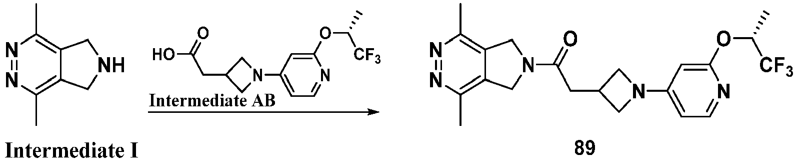

### Step 1

The trifluoroacetate of intermediate **I** (49 mg, 0.33 mmol) and intermediate **AB** (100 mg, 0.33 mmol) were dissolved in DMF (5 mL), then DIEA (127 mg, 0.99 mmol) was added thereto, and the mixture was stirred at room temperature for 30 minutes. HATU (175 mg, 0.46 mmol) was then added thereto, and the mixture was stirred at room temperature for another 1 hour. The reaction mixture was concentrated under reduced pressure to obtain a crude product containing the target compound, which was purified by preparative high performance liquid chromatography (chromatographic column: Waters-Xbridge-C18, 10 µm, 19 * 250 mm, mobile phase: acetonitrile-10 mmol/L ammonium bicarbonate aqueous solution, gradient: 35 to 65%, retention time: 11 minutes) to obtain compound **89.** ¹H NMR (400 MHz, DMSO-*d*₆) δ 7.75 (d, *J* = 5.80 Hz, 1H), 6.15-6.12 (m, 1H), 5.87-5.78 (m, 1H), 5.75-5.73 (m, 1H), 4.93-4.90 (m, 2H), 4.71-4.68 (m, 2H), 4.10-4.06 (m, 2H), 3.64-3.58 (m, 2H), 3.12-3.05 (m, 1H), 2.85-2.82 (m, 2H), 2.55-2.53 (m, 6H), 1.38 (d, *J* = 6.50 Hz, 3H). ESI-MS calculated for C₂₁H₂₅F₃N₅O₂ [M+H]⁺ = 436.2, found 436.2.

### Example 98

### Synthetic route:

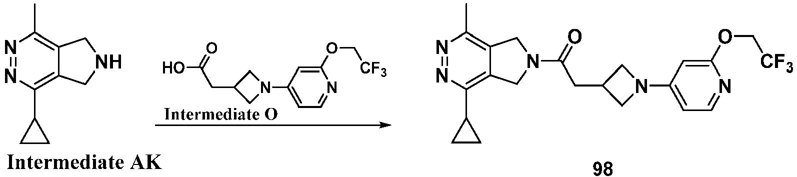

### Step 1

Intermediate **AK** (73 mg, 0.41 mmol) and intermediate **O** (80 mg, 0.28 mmol) were dissolved in DMF (5 mL), then DIEA (142 mg, 1.10 mmol) was added thereto, and the mixture was stirred at room temperature for 30 minutes. HATU (136 mg, 0.36 mmol) was then added thereto, and the mixture was stirred at room temperature for another 1 hour. The reaction mixture was concentrated under reduced pressure to obtain a crude product containing the target compound, which was purified by preparative high performance liquid chromatography (chromatographic column: Waters-Xbridge-C18, 10 µm, 19 * 250 mm, mobile phase: acetonitrile-10 mmol/L ammonium bicarbonate aqueous solution, gradient: 35 to 65%, retention time: 9 minutes) to obtain compound **98.** ¹H NMR (400 MHz, DMSO-*d*₆) δ 7.77 (d, *J* = 5.76 Hz, 1H), 6.16 (dd, *J* = 5.80, 1.96 Hz, 1H), 5.79 (d, *J* = 1.94 Hz, 1H), 5.06-5.03 (m, 1H), 4.95-4.86 (m, 3H), 4.79-4.69 (m, 2H), 4.09 (t, *J =* 8.04 Hz, 2H), 3.65-3.58 (m, 2H), 3.14-3.05 (m, 1H), 2.88-2.83 (m, 2H), 2.53 (s, 3H), 2.11-2.01 (m, 1H), 1.18-1.13 (m,2H), 1.09-1.04 (m, 2H). ESI-MS calculated for C₂₂H₂₅F₃N₅O₂ [M+H]⁺ = 448.2, found 448.2.

### Example 110

### Synthetic route:

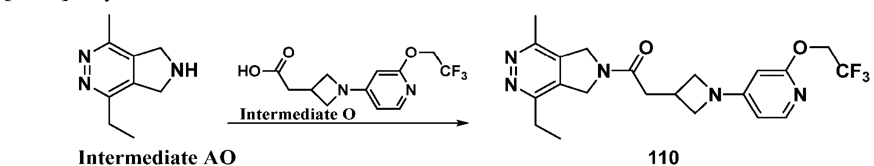

### Step 1

Intermediate **AO** (59 mg, 0.36 mmol) and intermediate **O** (70 mg, 0.24 mmol) were dissolved in DMF (5 mL), then DIEA (94 mg, 0.72 mmol) was added thereto, and the mixture was stirred at room temperature for 30 minutes. HATU (119 mg, 0.31 mmol) was then added thereto, and the mixture was stirred at room temperature for another 1 hour. The reaction mixture was concentrated under reduced pressure to obtain a crude product containing the target compound, which was purified by preparative high performance liquid chromatography (chromatographic column: Waters-Xbridge-C18, 10 µm, 19 * 250 mm, mobile phase: acetonitrile-10 mmol/L ammonium bicarbonate aqueous solution, gradient: 31 to 61%, retention time: 9 minutes) to obtain compound **110.** ¹H NMR (400 MHz, DMSO-*d*₆) δ 7.76 (d, *J =* 5.84 Hz, 1H), 6.15 (dd, *J* = 5.80, 1.96 Hz, 1H), 5.78 (d, *J* = 1.94 Hz, 1H), 5.02-4.85 (m, 4H), 4.74-4.67 (m, 2H), 4.11-4.05 (m, 2H), 3.64-3.57 (m, 2H), 3.12-3.04 (m, 1H), 2.90-2.82 (m, 4H), 2.55 (s, 3H), 1.26 (t, *J* = 7.54 Hz, 3H). ESI-MS calculated for C₂₁H₂₅F₃N₅O₂ [M+H]⁺ = 436.2, found 436.2.

### Example 111

### Synthetic route:

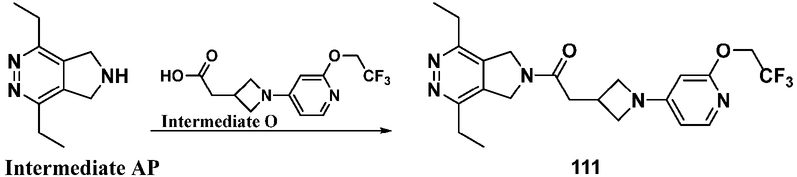

### Step 1

Intermediate **AP** (70 mg, 0.39 mmol) and intermediate **O** (115 mg, 0.39 mmol) were dissolved in DMF (5 mL), then DIEA (153 mg, 1.18 mmol) was added thereto, and the mixture was stirred at room temperature for 30 minutes. HATU (195 mg, 0.51 mmol) was then added thereto, and the mixture was stirred at room temperature for another 1 hour. The reaction mixture was concentrated under reduced pressure to obtain a crude product containing the target compound, which was purified by preparative high performance liquid chromatography (chromatographic column: Waters-Xbridge-C18, 10 µm, 19 * 250 mm, mobile phase: acetonitrile-10 mmol/L ammonium bicarbonate aqueous solution, gradient: 34 to 64%, retention time: 9 minutes) to obtain compound **111.** ¹H NMR (400 MHz, DMSO-*d*₆) δ 7.76 (d, *J =* 5.78 Hz, 1H), 6.15 (dd, *J =* 5.82, 1.94 Hz, 1H), 5.78 (d, *J =* 1.92 Hz, 1H), 4.97-4.83 (m, 4H), 4.73-4.70 (m, 2H), 4.10-4.06 (m, 2H), 3.64-3.57 (m, 2H), 3.13-3.05 (m, 1H), 2.91-2.83 (m, 6H), 1.27 (t, *J* = 7.54 Hz, 6H). ESI-MS calculated for C₂₂H₂₇F₃N₅O₂ [M+H]⁺ = 450.2, found 450.2.

### Example 116

### Synthetic route:

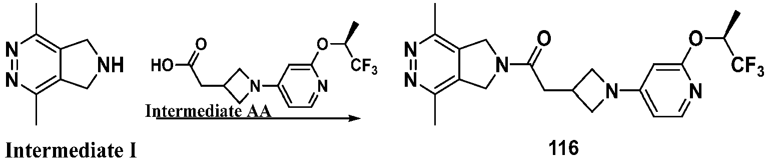

### Step 1

Intermediate **I** (66 mg, 0.44 mmol) and intermediate **AA** (90 mg, 0.30 mmol) were dissolved in DMF (5 mL), then DIEA (115 mg, 0.89 mmol) was added thereto, and the mixture was stirred at room temperature for 30 minutes. HATU (169 mg, 0.44 mmol) was then added thereto, and the mixture was stirred at room temperature for another 1 hour. The reaction mixture was concentrated under reduced pressure to obtain a crude product containing the target compound, which was purified by preparative high performance liquid chromatography (chromatographic column: Waters-Xbridge-C18, 10 µm, 19 * 250 mm, mobile phase: acetonitrile-10 mmol/L ammonium bicarbonate aqueous solution, gradient: 32 to 62%, retention time: 9 minutes) to obtain compound **116.** ¹H NMR (400 MHz, DMSO-*d*₆) δ 7.76 (d, *J* = 5.80 Hz, 1H), 6.13 (dd, *J* = 5.84, 2.00 Hz, 1H), 5.87-5.80 (m, 1H), 5.74 (d, *J* = 1.96 Hz, 1H), 4.93-4.90 (m, 2H), 4.71-4.68 (m, 2H), 4.12-4.03 (m, 2H), 3.64-3.55 (m, 2H), 3.14-3.03 (m, 1H), 2.86-2.82 (m, 2H), 2.58-2.52 (m, 6H), 1.38 (d, *J* = 6.50 Hz, 3H). ESI-MS calculated for C₂₁H₂₅F₃N₅O₂ [M+H]⁺ = 436.2, found 436.2.

### Example 123

### Synthetic route:

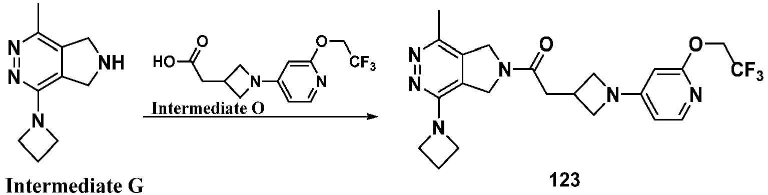

### Step 1

Intermediate **G** (33 mg, 0.17 mmol), HATU (78 mg, 0.20 mmol), DIEA (66 mg, 0.51 mmol), and intermediate **O** (50 mg, 0.17 mmol) were added to DMF (5 mL), and the mixture was stirred at room temperature for 3 hours. The reaction mixture was filtered and concentrated under reduced pressure to obtain a crude product containing the target compound, which was purified by preparative high performance liquid chromatography (chromatographic column: Waters-Xbridge-C18-10 µm-19 * 250 mm, mobile phase: acetonitrile-10 mmol/L ammonium bicarbonate aqueous solution, gradient: 30 to 60%, retention time: 10 minutes) to obtain compound **123.** ¹H NMR (400 MHz, DMSO-*d*₆) δ 7.76 (d, *J* = 5.78 Hz, 1H), 6.14 (dd, *J* = 5.78, 1.82 Hz, 1H), 5.78 (d, *J =* 1.884 Hz, 1H), 4.95-4.76 (m, 4H), 4.65-4.57 (m, 2H), 4.13-4.05 (m, 6H), 3.61-3.58 (m, 2H), 3.12-3.01 (m, 1H), 2.84-2.81 (m, 2H), 2.40-2.32 (m, 5H). ESI-MS calculated for C₂₂H₂₆F₃N₆O₂ [M+H]⁺ = 463.2, found 463.0.

### Example 124

### Synthetic route:

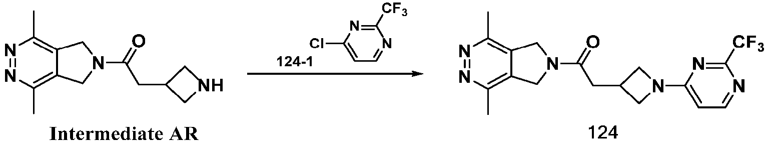

### Step 1

Intermediate **AR** (67 mg, 0.27 mmol), **124-1** (50 mg, 0.27 mmol), TEA (83 mg, 0.82 mmol), and cesium fluoride (62 mg, 0.42 mmol) were added to DMSO (5 mL), and the mixture was stirred at 100°C for 10 hours. The reaction mixture was cooled, diluted with water (50 mL), and extracted with ethyl acetate (50 mL × 3). The organic phases were combined, washed with saturated brine (100 mL), dried over anhydrous sodium sulfate, and concentrated under reduced pressure to obtain a crude product containing the target compound, which was purified by preparative high performance liquid chromatography (chromatographic column: Waters-Xbridge-C18-10 µm-19 * 250 mm, mobile phase: acetonitrile-10 mmol/L ammonium bicarbonate aqueous solution, gradient: 16 to 46%, retention time: 10 minutes) to obtain compound **124.** ¹H NMR (400 MHz, DMSO-*d*₆) δ 8.27 (d, *J* = 6.02 Hz, 1H), 6.59 (d, *J* = 6.02 Hz, 1H), 4.93-4.89 (m, 2H), 4.71-4.68 (m, 2H), 4.29-4.23 (m, 2H), 3.85-3.77 (m, 2H), 3.17-3.07 (m, 1H), 2.89-2.86 (m, 2H), 2.55-2.53 (m, 6H). ESI-MS calculated for C₁₈H₂₀F₃N₆O [M+H]⁺ = 393.2, found 393.2.

### Example 125

### Synthetic route:

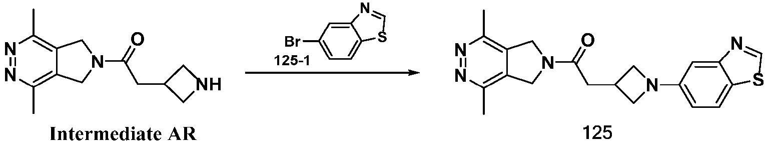

### Step 1

Intermediate **AR** (80 mg, 0.32 mmol), **125-1** (70 mg, 0.32 mmol), methanesulfonato(2-dicyclohexylphosphino-2',4',6'-tri-i-propyl-1,1'-biphenyl)(2'-amino-1,1'-biphenyl-2-yl)palladium(II) (28 mg, 0.03 mmol), and cesium carbonate (317 mg, 0.97 mmol) were added to 1,4-dioxane (5 mL), and the mixture was heated to 100°C under nitrogen atmosphere and stirred for 18 hours. The reaction mixture was cooled and concentrated under reduced pressure to obtain a crude product containing the target compound, which was purified by preparative high performance liquid chromatography (chromatographic column: Waters-Xbridge-C18-10 µm-19 * 250 mm, mobile phase: acetonitrile-10 mmol/L ammonium bicarbonate aqueous solution, gradient: 18 to 48%, retention time: 10 minutes) to obtain compound **125.** ¹H NMR (400 MHz, DMSO-*d*₆) δ 9.25 (s, 1H), 7.89 (d, *J =* 8.62 Hz, 1H), 7.02 (d, *J* = 2.18 Hz, 1H), 6.68 (dd, *J =* 8.62, 2.24 Hz, 1H), 4.94-4.92 (m, 2H), 4.72-4.69 (m, 2H), 4.10-4.05 (m, 2H), 3.60-3.56 (m, 2H), 3.16-3.03 (m, 1H), 2.88-2.84 (m, 2H), 2.55 (s, 3H), 2.53 (s, 3H). ESI-MS calculated for C₂₀H₂₂N₅OS [M+H]⁺ = 380.2, found 380.0.

### Example 126

### Synthetic route:

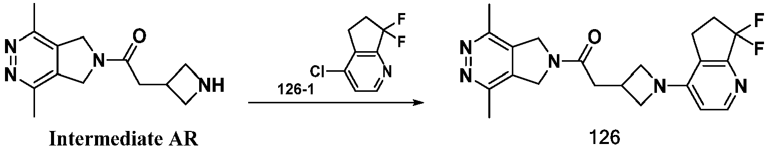

### Step 1

Intermediates **AR** (65 mg, 0.26 mmol), **126-1** (50 mg, 0.26 mmol), cesium fluoride (60 mg, 0.40 mmol), and TEA (80 mg, 0.79 mmol) were added to DMSO (5 mL). The reaction mixture was cooled, diluted with water (50 mL), and extracted with ethyl acetate (50 mL × 3). The organic phases were combined, washed with saturated brine (100 mL), dried over anhydrous sodium sulfate, and concentrated under reduced pressure to obtain a crude product containing the target compound, which was purified by preparative high performance liquid chromatography (chromatographic column: Waters-Xbridge-C18-10 µm-19 * 250 mm, mobile phase: acetonitrile-10 mmol/L ammonium bicarbonate aqueous solution, gradient: 15 to 45%, retention time: 10 minutes) to obtain compound **126.** ¹H NMR (400 MHz, DMSO-*d*₆) δ 8.11 (d, *J* = 5.50 Hz, 1H), 6.28 (d, *J =* 5.58 Hz, 1H), 4.94-4.91 (m, 2H), 4.70-4.67 (m, 2H), 4.32-4.28 (m, 2H), 3.86-3.82 (m, 2H), 3.09-2.98 (m, 3H), 2.87-2.84 (m, 2H), 2.56-2.53 (m, 8H). ESI-MS calculated for C₂₁H₂₄F₂N₅O [M+H]⁺ = 400.2, found 400.2.

### Example 127

### Synthetic route:

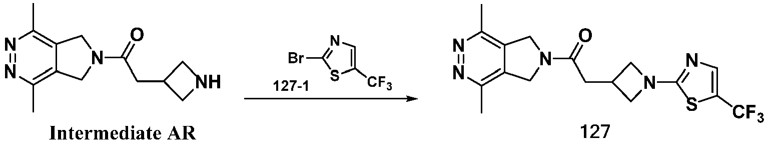

### Step 1

Intermediates **AR** (106 mg, 0.43 mmol), **127-1** (100 mg, 0.43 mmol), and cesium carbonate (351 mg, 1.08 mmol) were added to DMF (3 mL). The reaction mixture was cooled, diluted with water (50 mL), and extracted with ethyl acetate (50 mL × 3). The organic phases were combined, washed with saturated brine (100 mL), dried over anhydrous sodium sulfate, and concentrated under reduced pressure to obtain a crude product containing the target compound, which was purified by preparative high performance liquid chromatography (chromatographic column: Waters-Xbridge-C18-10 µm-19 * 250 mm, mobile phase: acetonitrile-10 mmol/L ammonium bicarbonate aqueous solution, gradient: 25 to 55%, retention time: 10 minutes) to obtain compound **127.** ¹H NMR (400 MHz, DMSO-*d*₆) δ 7.70 (s, 1H), 4.92-4.89 (m, 2H), 4.71-4.68 (m, 2H), 4.28-4.21 (m, 2H), 3.84-3.78 (m, 2H), 3.22-3.16 (m, 1H), 2.91-2.88 (m, 2H), 2.56-2.53 (m, 6H). ESI-MS calculated for C₁₇H₁₉F₃N₅OS [M+H]⁺ = 398.1, found 398.1.

### Example 128

### Synthetic route:

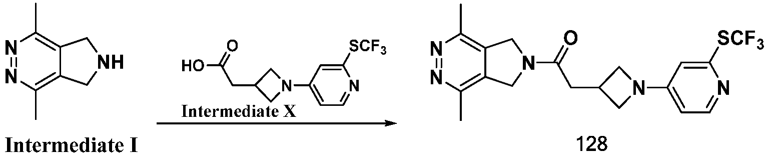

### Step 1

Intermediate **I** (26 mg, 0.17 mmol), HATU (78 mg, 0.20 mmol), DIEA (66 mg, 0.51 mmol), and intermediate **X** (50 mg, 0.17 mmol) were added to DMF (5 mL), and the mixture was stirred at room temperature for 3 hours. The reaction mixture was filtered and concentrated under reduced pressure to obtain a crude product containing the target compound, which was purified by preparative high performance liquid chromatography (chromatographic column: Waters-Xbridge-C18-10 µm-19 * 250 mm, mobile phase: acetonitrile-10 mmol/L ammonium bicarbonate aqueous solution, gradient: 20 to 50%, retention time: 10 minutes) to obtain compound **128.** ¹H NMR (400 MHz, DMSO-*d*₆) δ 8.10 (d, *J* = 5.72 Hz, 1H), 6.59 (d, *J* = 2.20 Hz, 1H), 6.40 (dd, *J* = 5.72, 2.22 Hz, 1H), 4.93-4.90 (m, 2H), 4.70-4.67 (m, 2H), 4.16-4.12 (m, 2H), 3.68-3.64 (m, 2H), 3.17-3.03 (m, 1H), 2.88-2.85 (m, 2H), 2.55-2.52 (m, 6H). ESI-MS calculated for C₁₉H₂₁F₃N₅OS [M+H]⁺ = 424.1, found 424.1.

### Example 129

### Synthetic route:

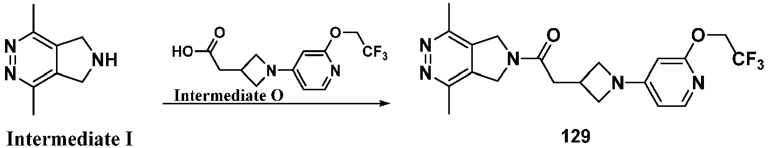

### Step 1

Intermediate **I** (26 mg, 0.17 mmol), HATU (78 mg, 0.20 mmol), DIEA (66 mg, 0.51 mmol), and intermediate **O** (50 mg, 0.17 mmol) were added to DMF (5 mL), and the mixture was stirred at room temperature for 3 hours. The reaction mixture was filtered and concentrated under reduced pressure to obtain a crude product containing the target compound, which was purified by preparative high performance liquid chromatography (chromatographic column: Waters-Xbridge-C18-10 µm-19 * 250 mm, mobile phase: acetonitrile-10 mmol/L ammonium bicarbonate aqueous solution, gradient: 37 to 47%, retention time: 10 minutes) to obtain compound **129.** ¹H NMR (400 MHz, DMSO-*d*₆) δ 7.76 (d, *J =* 5.80 Hz, 1H), 6.15 (dd, *J =* 5.78, 1.98 Hz, 1H), 5.78 (d, *J =* 1.96 Hz, 1H), 4.99-4.83 (m, 4H), 4.71-4.68 (m, 2H), 4.10-4.06 (m, 2H), 3.62-3.59 (m, 2H), 3.12-3.05 (m, 1H), 2.86-2.83 (m, 2H), 2.56-2.53 (m, 6H). ESI-MS calculated for C₂₀H₂₃F₃N₅O₂ [M+H]⁺ = 422.2, found 422.0.

### Example 130

### Synthetic route:

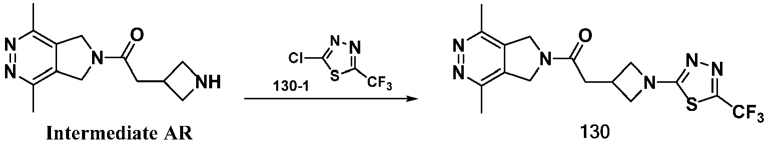

### Step 1

Intermediates **AR** (65 mg, 0.26 mmol), **130-1** (50 mg, 0.26 mmol), cesium fluoride (60 mg, 0.40 mmol), and TEA (80 mg, 0.79 mmol) were added to DMSO (5 mL). The reaction mixture was cooled, diluted with water (50 mL), and extracted with ethyl acetate (50 mL × 3). The organic phases were combined, washed with saturated brine (100 mL), dried over anhydrous sodium sulfate, and concentrated under reduced pressure to obtain a crude product containing the target compound, which was purified by preparative high performance liquid chromatography (chromatographic column: Waters-Xbridge-C18-10 µm-19 * 250 mm, mobile phase: acetonitrile-10 mmol/L ammonium bicarbonate aqueous solution, gradient: 18 to 45%, retention time: 9 minutes) to obtain compound **130.** ¹H NMR (400 MHz, DMSO-*d*₆) δ 4.93-4.89 (m, 2H), 4.72-4.68 (m, 2H), 4.38-4.32 (m, 2H), 3.96-3.88 (m, 2H), 3.29-3.18 (m, 1H), 2.92-2.88 (m, 2H), 2.55-2.52 (m, 6H). ESI-MS calculated for C₁₆H₁₈F₃N₆OS [M+H]⁺ = 399.1, found 399.1.

### Example 131

### Synthetic route:

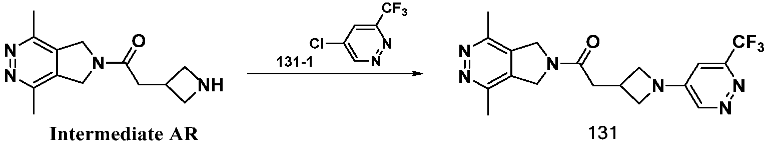

### Step 1

Intermediate **AR** (67 mg, 0.27 mmol), **131-1** (50 mg, 0.27 mmol), TEA (83 mg, 0.82 mmol), and cesium fluoride (42 mg, 0.27 mmol) were added to DMSO (5 mL), and the mixture was stirred at 70°C for 18 hours. The reaction mixture was cooled, diluted with water (50 mL), and extracted with ethyl acetate (50 mL × 3). The organic phases were combined, washed with saturated brine (100 mL), dried over anhydrous sodium sulfate, and concentrated under reduced pressure to obtain a crude product containing the target compound, which was purified by preparative high performance liquid chromatography (chromatographic column: Waters-Xbridge-C18-10 µm-19 * 250 mm, mobile phase: acetonitrile-10 mmol/L ammonium bicarbonate aqueous solution, gradient: 5 to 95%, retention time: 10 minutes) to obtain compound **131.** ¹H NMR (400 MHz, DMSO-*d*₆) δ 8.63 (d, *J* = 2.80 Hz, 1H), 6.91 (d, *J* = 2.80 Hz, 1H), 4.92-4.90 (m, 2H), 4.70-4.68 (m, 2H), 4.32-4.28 (m, 2H), 3.87-3.80 (m, 2H), 3.20-3.10 (m, 1H), 2.89-2.86 (m, 2H), 2.55-2.52 (m, 6H). ESI-MS calculated for C₁₈H₂₀F₃N₆O [M+H]⁺ = 393.2, found 392.9.

### Example 132

### Synthetic route:

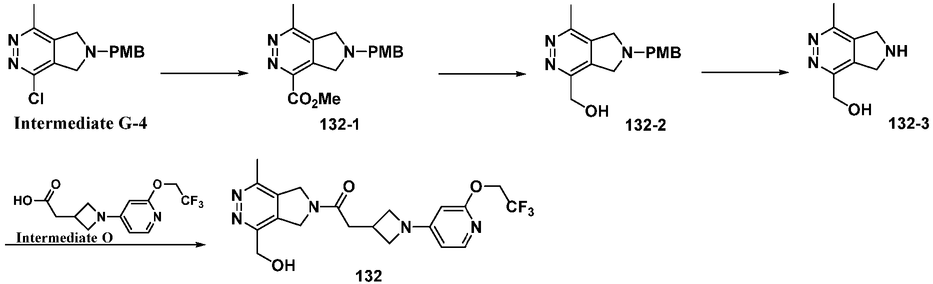

### Step 1

Intermediate **G-4** (500 mg, 1.60 mmol) was dissolved in methanol (20 mL), and [1,1'-bis(diphenylphosphino)ferrocene]dichloropalladium(II) dichloromethane complex (127 mg, 0.17 mmol) and TEA (524 mg, 5.18 mmol) were added thereto. The reaction system was replaced with carbon monoxide three times, then heated to 70°C, and stirred for 18 hours. The reaction mixture was filtered, and the filtrate was concentrated under reduced pressure and purified by silica gel column chromatography (petroleum ether/ethyl acetate, 1/4, v/v) to obtain **132-1.** ESI-MS calculated for C₁₇H₂₀N₃O₃ [M+H]⁺ =314.1, found 314.0.

### Step 2

**132-1** (200 mg, 0.64 mmol) was dissolved in tetrahydrofuran (10 mL), then lithium borohydride (14 mg, 0.64 mmol) was slowly added thereto at 0̊C, and the mixture was stirred at room temperature for 1 hour. The reaction mixture was quenched with water (30 mL) and extracted with dichloromethane (20 mL × 3). The organic phases were combined, dried over anhydrous sodium sulfate, filtered, concentrated under reduced pressure, and purified by silica gel column chromatography (petroleum ether/ethyl acetate, 1/4, v/v) to obtain **132-2.** ESI-MS calculated for C₁₆H₂₀N₃O₂ [M+H]⁺ = 286.2, found 286.2.

### Step 3

**132-2** (100 mg, 0.35 mmol) and trifluoroacetic acid (2 mL) were added to a 5 mL microwave tube, and the mixture was heated to 90°C and stirred for 8 hours. The reaction mixture was cooled to room temperature and concentrated under reduced pressure to obtain a crude product containing **132-3,** which was directly used in the next reaction step. ESI-MS calculated for C₈H₁₂N₃O [M+H]⁺ = 166.1, found 166.1.

### Step 4

**132-3** (56 mg, 0.34 mmol), HATU (98 mg, 0.26 mmol), DIEA (66 mg, 0.51 mmol), and intermediate **O** (50 mg, 0.17 mmol) were added to DMF (5 mL), and the mixture was stirred at room temperature for 3 hours. The reaction mixture was filtered and concentrated under reduced pressure to obtain a crude product containing the target compound, which was purified by preparative high performance liquid chromatography (chromatographic column: Waters-Xbridge-C18-10 µm-19 * 250 mm, mobile phase: acetonitrile-10 mmol/L ammonium bicarbonate aqueous solution, gradient: 5 to 95%, retention time: 10 minutes) to obtain compound **132.** ¹H NMR (400 MHz, MeOD-*d*₄) δ 7.74-7.71 (m, 1H), 6.14-6.12 (m, 1H), 5.82-5.75 (m, 1H), 5.12-5.10 (m, 1H), 4.97-4.89 (m, 4H), 4.82-4.78 (m, 1H), 4.74-4.68 (m, 2H), 4.19-4.14 (m, 2H), 3.73-3.64 (m, 2H), 3.26-3.08 (m, 1H), 2.95-2.91 (m, 2H), 2.65 (s, 3H). ESI-MS calculated for C₂₀H₂₃F₃N₅O₃ [M+H]⁺ = 438.2, found 437.9.

### Activity assay 1: Evaluation of PAM activity of compounds on M4 receptor

### Experimental purpose:

The activity of compounds on M4 receptor was determined by FLIPR CALCIUM 6 ASSAY KIT using a stable transfected cell line expressing human M4 receptor (M4-Gα15-CHO).

### Experimental materials:

| **Reagents and consumables** | **Supplier** | **Catalog number** |
|---|---|---|
| F-12 medium | Hyclone | SH30026.01 |
| FLIPR CALCIUM 6 ASSAY KIT | Molecul Device | R8191 |
| Fetal bovine serum | AusGeneX | FBS500-S |
| Hygromycin B | Innochem | H8080-1g |
| Geneticin (G418) | Gibco | 10131035 |
| 0.25% trypsin | Gibco | 25200072 |
| Phosphate buffered saline (PBS) | Innochem | B46663 |
| Hanks' balanced salt solution (HBSS) | Gibco | 14025-092 |
| Dimethyl sulfoxide (DMSO) | Sigma | D4540 |
| 384-well loading plate | Nunc | 264573 |
| 96-well polypropylene plate | Biosen | P-0.36-BSA-96 |
| 384-well cell plate | Corning | 3764 |
| 2-(4-(2-Hydroxyethyl)piperazine)ethanesulfonic acid (HEPES) | Santa Cruz | SC-29097A |
| Sodium hydroxide (NaOH) | Innochem | A36865 |
| Probenecid | MCE | HY-B0545 |
| Acetylcholine | MCE | HY-B0282 |
| Pipette tip | Molecular Devices | 9000-0763 |

### Experimental instruments:

| **Instruments and equipment** | **Supplier** | **Catalog number** |
|---|---|---|
| Biosafety cabinet | ECSO | AC2-6S1-TC |
| Carbon dioxide cell incubator | ESCO | CCI-170B-8 |
| Inverted microscope | Olympus | CKX53 |
| Microplate low-speed centrifuge | Xiangzhi | TD5B |
| FLIPR^{®} Penta | Molecul Device | 5.0 |

### Cell treatment:

1. This study uses a CHO cell line stably expressing M4-Gα15 receptor, which expresses human CHRM4 and GNA15 genes, respectively.
2. Cell treatment: M4-Gα15-CHO cells were cultured in F-12 medium containing 10% fetal bovine serum, 0.4 mg/mL hygromycin B, and 0.8 mg/mL geneticin G418, with a culture temperature of 37°C and a CO₂ concentration of 5%. The old media was removed and the cells were washed once with phosphate buffered saline. 1 mL of trypsin solution was then added, and the mixture was incubated at 37°C for approximately 2 minutes. After the cells were detached from the dish, approximately 5 mL of complete media pre-warmed at 37°C was added. The cell suspension was gently pipetted to separate the aggregated cells. The cell suspension was transferred to a sterile centrifuge tube and centrifuged at 1000 rpm for 5 minutes.

### Experimental procedure:

1. Cell plating: M4-Gα15-CHO cells were digested and collected, then resuspended and counted, and seeded into a 384-well cell plate at a density of 1.2 × 10⁴ cells/25 µL/well. The cell plate was then incubated in a 5% CO₂ incubator at 37°C for approximately 20 hours.
2. Preparation of loading buffer according to the instructions of FLIPR CALCIUM 6 ASSAY KIT after 24 hours: Component A was freeze-thawed to room temperature, and diluted with assay buffer and probenecid solution to obtain a loading buffer, which was kept at room temperature for later use.
3. The medium was removed from the cell plate, and 35 µL of loading buffer was quickly added to each well. After centrifugation, the cell plate was incubated at 37°C in the dark for 120 minutes.
4. A working solution of the compound to be tested was prepared, 5 µL of which was transferred to the corresponding cell wells, and incubated at 37°C in the dark for 30 minutes.
5. 30 nM acetylcholine agonist working solution was prepared and transferred to a 384-well loading plate at 20 µL/well.
6. The cell plate, the loading plate, and the pipette tip were placed at the corresponding positions of the FLIPR instrument. 10 µL of the agonist diluted in step 5 was added to the experimental wells using FLIPR, and data were collected at a wavelength of 515 nm to 575 nm.
7. The signal value was plotted against compound concentration. Curve fitting and EC₅₀ calculation were performed by nonlinear regression method using GraphPad Prism software.

### Preparation of assay buffer and loading buffer:

1. 0.5 mol/L 2-(4-(2-hydroxyethyl)piperazine)ethanesulfonic acid buffer was mixed with Hanks' balanced salt solution (pH 7.4) at a volume ratio of 1:24 to prepare an assay buffer. 10 mL of assay buffer was added to a vial of CALCIUM 6 ASSAY KIT Component A in the form of lyophilized powder, and aliquoted and stored at -20°C.
2. Probenecid powder was dissolved in 1 mol/L sodium hydroxide solution to a final concentration of 250 mol/L. The assay buffer, freeze-thawed Component A, and probenecid solution were mixed at a volume ratio of 44:5:1 to prepare a loading buffer.

### Experimental results:

| **Compound** | **EC₅₀ (µM)** |
|---|---|
| Example 12 | 0.35 |
| Example 24 | 0.387 |
| Example 47 | 0.25 |
| Example 50 | 1.78 |
| Example 51 | 1.37 |
| Example 67 | 0.39 |
| Example 68 | 0.48 |
| Example 89 | 0.29 |
| Example 98 | 1.13 |
| Example 110 | 0.90 |
| Example 116 | 0.58 |

### Experimental conclusion:

The experimental samples (compounds) were prepared from the corresponding examples, and the results are shown in the table above. The compounds of the present disclosure show agonistic activity on the M4 receptor in this experimental system.

### Activity assay 2: Evaluation of pharmacokinetic properties of compounds in vivo in mice

### Experimental purpose:

The compounds obtained in the examples of the present disclosure were evaluated for their pharmacokinetic properties *in vivo* in CD-1 mice.

### Experimental materials:

| **Material** | **Supplier** | **Catalog number** |
|---|---|---|
| CD-1 mice (male, 20 to 40 g, 6 to 9 weeks old) | Beijing Vital River Laboratory Animal Technology Co., Ltd. | M-LOT20220246 |
| EDTA2K anticoagulant tube | Jiangsu Kangjian Medical Apparatus Co., Ltd. | 20020301 |

### Experimental procedure:

The compounds were tested for their pharmacokinetic characteristics in rodents after intravenous injection and oral administration following standard protocols. In the experiment, the candidate compound was prepared into a clear solution or suspension with a specified vehicle, and administered to three mice via single-dose intravenous injection and oral administration, respectively. The vehicle for both intravenous injection and oral administration was 5% dimethyl sulfoxide + 95% (10% polyethylene glycol (15)-hydroxystearate in water). Whole blood samples within 24 hours were collected into a commercial EDTA2K anticoagulant tube, followed by centrifugation to obtain the upper plasma sample, and acetonitrile solution containing internal standard was added to precipitate proteins. After centrifugation, the supernatant was mixed with an equal volume of water, and after another centrifugation, the supernatant was taken for injection. Plasma concentration was quantitatively analyzed using LCMS/MS analysis method, and pharmacokinetic parameters were calculated.

### Experimental method:

| Test sample | Dose |
|---|---|
| Example 12 | Intravenous injection: 1.0 mg/kg |
| | Oral administration: 2.0 mg/kg |
| Example 67 | Intravenous injection: 1.0 mg/kg |
| | Oral administration: 2.0 mg/kg |
| Example 89 | Intravenous injection: 1.0 mg/kg |
| | Oral administration: 2.0 mg/kg |

### Experimental results:

| Test sample | Peak concentration Cₘₐₓ (ng/mL) | Half-life T_{1/2}, po (hr) | Volume of distribution at steady state Vdₛₛ (L/kg) | Clearance CL (mL/min/kg) | Area under the curve AUC₀₋ₗₐₛₜ, po (hr*ng/mL) | Bioavailability F (%) |
|---|---|---|---|---|---|---|
| Example 12 | 614 | 1.13 | 2.30 | 17.1 | 1693 | 86.6 |
| Example 67 | 827 | 4.78 | 1.53 | 3.32 | 8039 | 82.7 |
| Example 89 | 345 | 4.65 | 3.72 | 7.65 | 3474 | 80.6 |

### Experimental conclusion:

The test samples were prepared from the corresponding examples. The results show that the compounds of the present disclosure exhibit good pharmacokinetic properties.

### Activity assay 3: Evaluation of pharmacokinetic properties of compounds in vivo in rats

### Experimental purpose:

The compounds obtained in the examples of the present disclosure were evaluated for their pharmacokinetic properties *in vivo* in SD rats.

### Experimental materials:

| **Material** | **Supplier** | **Catalog number** |
|---|---|---|
| SD rats (male, 150 to 400 g, 6 to 9 weeks old) | Beijing Vital River Laboratory Animal Technology Co., Ltd. | R-LOT20230032 |
| EDTA2K anticoagulant tube | Jiangsu Kangjian Medical Apparatus Co., Ltd. | 20020301 |

### Experimental procedure:

The compounds were tested for their pharmacokinetic characteristics in rodents after intravenous injection and oral administration following standard protocols. In the experiment, the candidate compound was prepared into a clear solution or suspension with a specified vehicle, and administered to three rats via single-dose intravenous injection and oral administration, respectively. The vehicle for intravenous injection was 5% dimethyl sulfoxide + 95% (10% polyethylene glycol (15)-hydroxystearate in water). The vehicle for oral administration was 5% dimethyl sulfoxide + 95% (10% polyethylene glycol (15)-hydroxystearate in water) or 0.5% methyl cellulose + 0.2% Tween 80 + 99.3% water. Whole blood samples within 24 hours were collected into a commercial EDTA2K anticoagulant tube, followed by centrifugation to obtain the upper plasma sample, and acetonitrile solution containing internal standard was added to precipitate proteins. After centrifugation, the supernatant was mixed with an equal volume of water, and after another centrifugation, the supernatant was taken for injection. Plasma concentration was quantitatively analyzed using LCMS/MS analysis method, and pharmacokinetic parameters were calculated.

The test samples were prepared from the corresponding examples. Some of the compounds of the present disclosure have a bioavailability of 80% or more, a clearance of 16 mL/min/kg or less, a half-life (T_{1/2}) of 2 to 10 hours, and an area under the curve (AUC₀₋ₗₐₛₜ) of 5000 to 50000 hr*ng/mL. The compounds of the present disclosure exhibit good pharmacokinetic properties.

### Activity assay 4: Evaluation of pharmacokinetic properties of compounds in vivo in dogs

### Experimental purpose:

The compounds obtained in the examples of the present disclosure were evaluated for their pharmacokinetic properties *in vivo* in Beagles.

### Experimental materials:

| **Material** | **Supplier** | **Catalog number** |
|---|---|---|
| Beagles (male, 5 to 12 kg, ≥ 5 months old) | Jiangsu Masi Biotechnology Co., Ltd. | Lot_NJ_D20230206_M30F10 |
| EDTA2K anticoagulant tube | Jiangsu Kangjian Medical Apparatus Co., Ltd. | 20020301 |

### Experimental procedure:

The compounds were tested for their pharmacokinetic characteristics in Beagles after intravenous injection and oral administration following standard protocols. In the experiment, the candidate compound was prepared into a clear solution or suspension with a specified vehicle, and administered to two Beagles via single-dose intravenous injection and oral administration, respectively. The vehicle for intravenous injection was 5% dimethyl sulfoxide + 95% (10% polyethylene glycol (15)-hydroxystearate in water). The vehicle for oral administration was 0.5% methyl cellulose + 0.2% Tween 80 + 99.3% water. Whole blood samples within 24 hours were collected into a commercial EDTA2K anticoagulant tube, followed by centrifugation to obtain the upper plasma sample, and acetonitrile solution containing internal standard was added to precipitate proteins. After centrifugation, the supernatant was mixed with an equal volume of water, and after another centrifugation, the supernatant was taken for injection. Plasma concentration was quantitatively analyzed using LCMS/MS analysis method, and pharmacokinetic parameters were calculated.

The test samples were prepared from the corresponding examples. Some of the compounds of the present disclosure have a bioavailability of 80% or more, a clearance of 16 mL/min/kg or less, a half-life (T_{1/2}) of 2 to 10 hours, and an area under the curve (AUC₀₋ₗₐₛₜ) of 2000 to 20000 hr*ng/mL. The compounds of the present disclosure exhibit good pharmacokinetic properties.

### Activity assay 5: Efficacy evaluation of compounds in prepulse inhibition model in mice

### Experimental purpose:

The compounds obtained in the examples of the present disclosure were evaluated for their efficacy in a prepulse inhibition model in C57 mice.

### Experimental materials:

| **Material** | **Supplier** | **Catalog number or model number** |
|---|---|---|
| C57BL/6J mice (male, 20 to 22 g, 6 to 9 weeks old) | Zhejiang Vital River Laboratory Animal Technology Co., Ltd. | 1820230403129018 |
| Prepulse inhibition test chamber | Shanghai Xinruan Information Technology Co., Ltd. | XR-XZ208-8 |

### Experimental procedure:

Animals were randomly divided into four groups of 10 each based on body weight prior to administration, with group 1 being the vehicle control group, group 2 being the model group, and groups 3 and 4 being the administration groups. The animals in groups 1 and 2 were intragastrically administered with vehicle C. The animals in group 3 were intragastrically administered with the compound of the present disclosure at a dose of 5 mg/kg. The animals in group 4 were intragastrically administered with the compound of the present disclosure at a dose of 10 mg/kg. 30 minutes after the administration, the animals in group 1 were intraperitoneally injected with vehicle B (saline), and the animals in groups 2, 3, and 4 were intraperitoneally injected with Dizocilpine (MK-801) at a dose of 0.3 mg/kg. 30 minutes after the injection, the animals were placed in a prepulse inhibition test chamber to start the experiment, and their peak of response was recorded by the instrument. First, the background noise of the prepulse inhibition test chamber parameters was set to 67 dB, and the animals were gently placed in a restraining cage and allowed to adapt for 10 minutes. Then, pre-test parameters were set: the animals were subjected to five random stimuli at 120 dB within a 5-minute period. Formal test parameters were set: at the beginning of the experiment, the animals were subjected to ten shock stimuli: five stimuli at 120 dB and five stimuli at 81+120 dB, with an average interval of 7 to 21 seconds between each stimulus by selecting a random function. The background noise of 67 dB was maintained throughout the experiment. Percentage of prepulse inhibition in animals = 1 - [(peak of response during stimulation at 81+120 dB) / peak of response during stimulation at 120 dB] × 100%.

The test samples were prepared from the corresponding examples. The percentage of prepulse inhibition in mice was observed to be approximately 40 to 80%. Some of the compounds of the present disclosure can effectively reverse the damage caused by MK-801 to the function of prepulse inhibition in mice, and exhibit good *in vivo* efficacy in the prepulse inhibition model in C57 mice.

### Activity assay 6: Efficacy evaluation of compounds in forced swimming model in mice

### Experimental purpose:

The compounds obtained in the examples of the present disclosure were evaluated for their efficacy in a forced swimming model in C57 mice.

### Experimental materials:

| **Material** | **Supplier** | **Catalog number or model number** |
|---|---|---|
| C57BL/6J mice (male, 20 to 25 g, 6 to 8 weeks old) | Zhejiang Vital River Laboratory Animal Technology Co., Ltd. | 1820230403129018 |
| Forced swimming test frame and analysis software | Vistrack | XR-VT |

### Experimental procedure:

Animals were randomly divided into four groups of 8 each based on body weight prior to administration, with group 1 being the vehicle control group and groups 2 to 4 being the administration groups. The animals in group 1 were intragastrically administered with vehicle C. The animals in groups 2 to 4 were intragastrically administered with the compound of the present disclosure at a dose of 2 mg/kg, 5 mg/kg, and 10 mg/kg, respectively. After administration, the animals were returned to their original feeding cages. After 1 hour, the animals were gently taken out of their feeding cages, soothed for 1 to 3 minutes until they were not stressed, and individually placed in a glass cylinder of 40 cm in height and 13 cm in diameter containing water at a depth of 26 cm and maintained at 23°C. Then, the immobility time of the animals within a 4-minute period of forced swimming was automatically recorded using analysis software.

The test samples were prepared from the corresponding examples. The immobility time of the mice was observed to be approximately 50 to 150 seconds. Some of the compounds of the present disclosure can significantly reduce the immobility time of the mice during forced swimming, and exhibit good antidepressant efficacy in the forced swimming model in C57 mice.

### Activity assay 7: Efficacy evaluation of compounds in tail suspension model in mice

### Experimental purpose:

The compounds obtained in the examples of the present disclosure were evaluated for their efficacy in a tail suspension model in C57 mice.

### Experimental materials:

| **Material** | **Supplier** | **Catalog number or model number** |
|---|---|---|
| C57BL/6J mice (male, 20 to 25 g, 6 to 8 weeks old) | Zhejiang Vital River Laboratory Animal Technology Co., Ltd. | |
| Tail suspension test frame and analysis software | Vistrack | XR-VT |

### Experimental procedure:

Animals were randomly divided into two groups of 8 each based on body weight prior to administration, with group 1 being the vehicle control group and group 2 being the administration group. The animals in group 1 were intragastrically administered with vehicle C. The animals in group 2 were intragastrically administered with the compound of the present disclosure at a dose of 5 mg/kg. After administration, the animals were returned to their original feeding cages. After 1 hour, the animals were gently taken out of their feeding cages, soothed for 1 to 3 minutes until they were not stressed, wrapped with medical tape around the tail tip at 1/3, and suspended in a tail suspension box. Then, the immobility time of the animals within a 4-minute period was automatically recorded using analysis software.

The test samples were prepared from the corresponding examples. The immobility time of the mice was observed to be 150 seconds or less. Some of the compounds of the present disclosure exhibit good antidepressant efficacy in the tail suspension model in C57 mice.

## Claims

1. A compound of formula I or a pharmaceutically acceptable salt thereof, 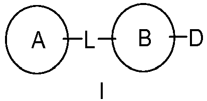
wherein 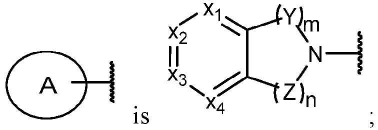
m is 1;
n is 1;
X₂ and X₃ are N;
X₁ and X₄ are independently -CR¹-;
Y and Z are independently -(CR²R³)ᵣ-; r is 1;
each R¹ is independently hydrogen, halogen, C₁-C₆ alkyl, C₁-C₆ alkoxy, 3- to 7-membered cycloalkyl, 3- to 7-membered heterocycloalkyl, or -NR¹⁻¹R¹⁻²; wherein the C₁-C₆ alkyl and C₁-C₆ alkoxy are optionally and independently substituted by 1, 2, 3, or 4 R^{a};
R¹⁻¹ and R¹⁻² are independently hydrogen or C₁-C₆ alkyl; or R¹⁻¹ and R¹⁻² together with the atom to which they are attached form a 3- to 7-membered heterocycloalkyl group;
each R^{a} is independently hydroxyl, C₁-C₆ alkoxy, or NR¹⁻⁴R¹⁻⁵;
R¹⁻⁴ and R¹⁻⁵ are independently hydrogen or C₁-C₃ alkyl;
R² and R³ are independently hydrogen;
L is 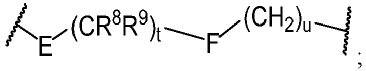 t is 0;
u is 1;
E is carbonyl;
F is a chemical bond;
B is 4- to 6-membered heterocycloalkyl; wherein the 4- to 6-membered heterocycloalkyl is optionally and independently substituted by 1, 2, or 3 Rⁱ;
each Rⁱ is independently hydrogen, halogen, hydroxyl, or C₁-C₃ alkyl;
D is 5- to 12-membered heteroaryl; wherein the 5- to 12-membered heteroaryl is optionally and independently substituted by 1, 2, or 3 R^{j};
each R^{j} is independently H, halogen, C₁-C₃ alkyl, OR¹²⁻³, or SR¹²⁻⁴; wherein the C₁-C₃ alkyl is optionally and independently substituted by 1, 2, or 3 R^{k};
R¹²⁻³ and R¹²⁻⁴ are independently C₁-C₃ alkyl; wherein the C₁-C₃ alkyl is optionally and independently substituted by 1, 2, or 3 R^{k-1};
each R^{k} is independently halogen or C₁-C₃ alkyl;
each R^{k-1} is independently halogen or C₁-C₃ alkyl;
the heteroatom in the 3- to 7-membered heterocycloalkyl, 4- to 6-membered heterocycloalkyl, and 5- to 12-membered heteroaryl is one or more kinds of N, O, and S, and the number of heteroatoms is 1 to 3.

2. The compound of formula I or the pharmaceutically acceptable salt thereof according to claim 1, which satisfies one or more of the following conditions;
(1) in R¹, the halogen is F, Cl, Br, or I, such as F or Cl; for another example, Cl;
(2) in R¹, the C₁-C₆ alkyl is C₁-C₃ alkyl, such as methyl, ethyl, n-propyl, or isopropyl; for another example, methyl, ethyl, or isopropyl;
(3) in R¹, the C₁-C₆ alkoxy is C₁-C₃ alkoxy, such as methoxy, ethoxy, n-propoxy, or isopropoxy; for another example, methoxy or ethoxy;
(4) in R¹, the 3- to 7-membered cycloalkyl is 3- to 6-membered cycloalkyl, such as cyclopropyl, cyclobutyl, or cyclopentyl; for another example, cyclopropyl;
(5) in R¹, the 3- to 7-membered heterocycloalkyl is 3- to 6-membered heterocycloalkyl; the heteroatom in the 3- to 6-membered heterocycloalkyl is preferably N or O; the number of heteroatoms in the 3- to 6-membered heterocycloalkyl is preferably 1 or 2; the 3- to 6-membered heterocycloalkyl is more preferably 4-membered heterocycloalkyl, such as 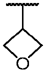 or 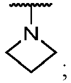
(6) in R^{a}, the C₁-C₆ alkoxy is C₁-C₃ alkoxy, such as methoxy, ethoxy, n-propoxy, or isopropoxy; for another example, methoxy;
(7) in R¹⁻¹ and R¹⁻², the C₁-C₆ alkyl is C₁-C₃ alkyl, such as methyl, ethyl, n-propyl, or isopropyl; for another example, methyl or ethyl, preferably methyl;
(8) in R¹⁻¹ and R¹⁻², the 3- to 7-membered heterocycloalkyl is 3- to 6-membered heterocycloalkyl; the heteroatom in the 3- to 6-membered heterocycloalkyl is preferably N or O; the number of heteroatoms in the 3- to 6-membered heterocycloalkyl is preferably 1 or 2; the 3- to 6-membered heterocycloalkyl is, for example, 4-membered heterocycloalkyl; for another example, 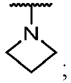
(9) in R¹⁻⁴ and R¹⁻⁵, the C₁-C₃ alkyl is methyl, ethyl, n-propyl, or isopropyl, such as methyl, ethyl, or n-propyl; for another example, methyl;
(10) in B, the 4- to 6-membered heterocycloalkyl is 4-membered heterocycloalkyl, 5-membered heterocycloalkyl, or 6-membered heterocycloalkyl; the heteroatom in the 4-membered heterocycloalkyl, 5-membered heterocycloalkyl, and 6-membered heterocycloalkyl is preferably N; the number of heteroatoms in the 4- to 6-membered heterocycloalkyl is preferably 1 or 2; for example, the 4- to 6-membered heterocycloalkyl is 4-membered azacycloalkyl or 5-membered azacycloalkyl; the 4-membered azacycloalkyl may be 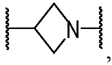 and the 5-membered azacycloalkyl may be 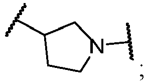
(11) in Rⁱ, the halogen is F, Cl, Br, or I, such as F or Cl; for another example, F;
(12) in Rⁱ, the C₁-C₃ alkyl is methyl, ethyl, n-propyl, or isopropyl, such as methyl;
(13) in R^{j}, the halogen is F, Cl, Br, or I, such as F or Cl; for another example, F;
(14) in R^{j}, the C₁-C₃ alkyl is methyl, ethyl, n-propyl, or isopropyl, such as methyl, ethyl, or isopropyl; for another example, methyl;
(15) in R¹²⁻³ and R¹²⁻⁴, the C₁-C₃ alkyl is methyl, ethyl, n-propyl, or isopropyl, such as methyl or ethyl;
(16) in R^{k}, the halogen is F, Cl, Br, or I, such as F or Cl; for another example, F;
(17) in R^{k}, the C₁-C₃ alkyl is methyl, ethyl, n-propyl, or isopropyl;
(18) in R^{k-1}, the halogen is F, Cl, Br, or I, such as F or Cl; for another example, F;
(19) in R^{k-1}, the C₁-C₃ alkyl is methyl, ethyl, n-propyl, or isopropyl, such as methyl.

3. The compound of formula I or the pharmaceutically acceptable salt thereof according to claim 1, which satisfies one or more of the following conditions;
(1) each R¹ is independently C₁-C₆ alkyl, 3- to 7-membered cycloalkyl, or -NR¹⁻¹R¹⁻²; wherein the C₁-C₆ alkyl is optionally and independently substituted by 1, 2, 3, or 4 R^{a}; for example, each R¹ is independently hydrogen, C₁-C₆ alkyl, 3- to 7-membered cycloalkyl, or-NR¹⁻¹R¹⁻²; preferably, each R¹ is independently C₁-C₆ alkyl;
(2) each R^{a} is independently hydroxyl, C₁-C₃ alkoxy, or NR¹⁻⁴R¹⁻⁵, such as hydroxyl;
(3) R¹⁻¹ and R¹⁻² together with the atom to which they are attached form a 3- to 7-membered heterocycloalkyl group;
(4) each R¹ is independently hydrogen, hydroxyl, or C₁-C₃ alkyl, such as hydrogen;
(5) each R^{j} is independently H, halogen, C₁-C₃ alkyl, OR¹²⁻³, or SR¹²⁻⁴; wherein the C₁-C₃ alkyl is optionally and independently substituted by 1, 2, or 3 R^{k}; for example, each R^{j} is independently H, halogen, C₁-C₃ alkyl, OR¹²⁻³, or SR¹²⁻⁴; for another example, each R^{j} is independently C₁-C₃ alkyl, -OR¹²⁻³, or -SR¹²⁻⁴; preferably, each R^{j} is independently -OR¹²⁻³; further preferably, R^{j} is halogen or C₁-C₃ alkyl;
(6) each R^{k} is independently halogen;
(7) each R^{k-1} is independently halogen;
(8) D is 5- to 6-membered heteroaryl; wherein the 5- to 6-membered heteroaryl is optionally and independently substituted by 1, 2, or 3 R^{j}; the 5- to 6-membered heteroaryl is, for example, 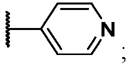
(9) the heteroatom in the 3- to 7-membered heterocycloalkyl, 4- to 6-membered heterocycloalkyl, 5- to 6-membered heteroaryl, and 5- to 12-membered heteroaryl is selected from one, two, or three kinds of N, O, or S, and the number of heteroatoms is 1, 2, or 3; for example, the heteroatom in the 3- to 7-membered heterocycloalkyl, 4- to 6-membered heterocycloalkyl, and 5- to 12-membered heteroaryl is N, O, or S, and the number of heteroatoms is 1 to 3.

4. The compound of formula I or the pharmaceutically acceptable salt thereof according to any one of claims 1 to 3, wherein the compound of formula I is a compound of formula I-3; 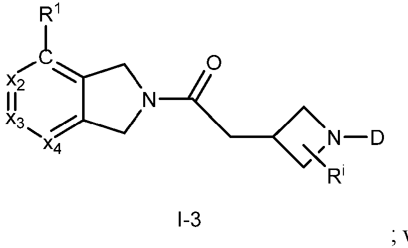 wherein R¹, Rⁱ, X₂, X₃, X₄, and D are as described in any one of claims 1 to 3.

5. The compound of formula I or the pharmaceutically acceptable salt thereof according to claim 1, wherein the compound of formula I is a compound of formula I-1-4; 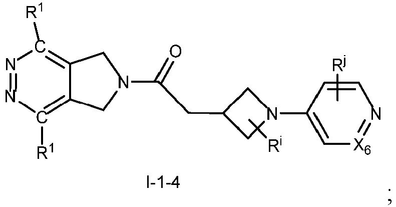
X₆ is C or N;
wherein R¹, Rⁱ, and R^{j} are as described in claim 1.

6. The compound of formula I or the pharmaceutically acceptable salt thereof according to claim 1, which satisfies one or more of the following conditions;
(1) in B, the heteroatom in the 4- to 6-membered heterocycloalkyl is N, and the number of heteroatoms is 1; for example, 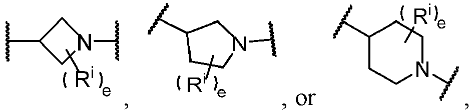 wherein e is independently 0, 1, 2, or 3; for another example, 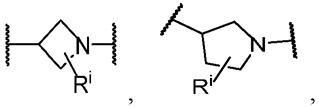 or 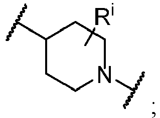
(2) in D, the 5- to 12-membered heteroaryl is 5- to 6-membered heteroaryl, phenyl-fused 5- to 6-membered heteroaryl, or 5- to 7-membered cycloalkyl-fused phenyl;
preferably, the compound of formula I satisfies one or more of the following conditions:
(a) D is 6-membered heteroaryl, wherein the heteroatom in the 6-membered heteroaryl is N, and the number of heteroatoms is 1 or 2; for example, 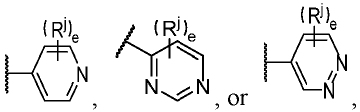 wherein e is independently 0, 1, 2, or 3; for another example, 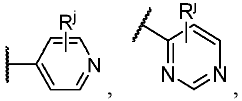 or 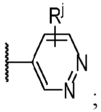
(b) D is 5-membered heteroaryl, wherein the heteroatom in the 5-membered heteroaryl is one or more kinds of N, S, and O, and the number of heteroatoms is 1, 2, or 3; for example, 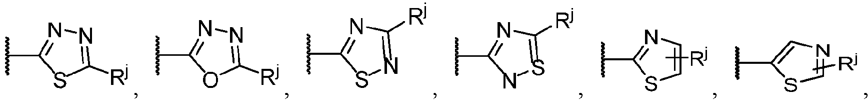 or 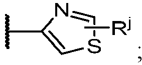 for another example, the heteroatom in the 5-membered heteroaryl is N and/or S, the number of heteroatoms is 1, 2, or 3; for yet another example, the heteroatom in the 5-membered heteroaryl is N or S, and the number of heteroatoms is 1, 2, or 3; preferably, 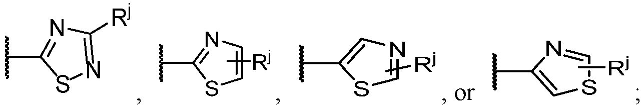
(c) D is 6-membered heteroaryl-fused 5-membered cycloalkyl, wherein the heteroatom in the 6-membered heteroaryl is N, and the number of heteroatoms is 1; for example, 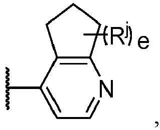 wherein e is independently 0, 1, 2, or 3; for another example, 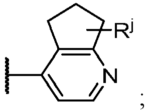
(d) D is phenyl-fused 5-membered heteroaryl, wherein the heteroatom in the 5-membered heteroaryl is N and/or S, and the number of heteroatoms is 2; for example, 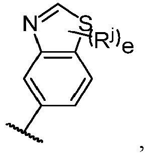 wherein e is independently 0, 1, 2, or 3; for another example,

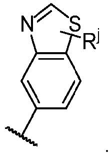

7. The compound of formula I or the pharmaceutically acceptable salt thereof according to claim 1, wherein the compound of formula I is any one of the following schemes:
scheme 1:
wherein 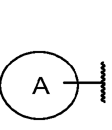 is 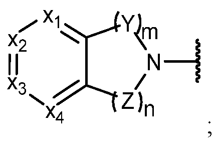
m is 1;
n is 1;
X₂ and X₃ are N;
X₁ and X₄ are independently -CR¹-;
Y and Z are independently -(CR²R³)ᵣ-; r is 1;
each R¹ is independently C₁-C₆ alkyl, 3- to 7-membered cycloalkyl, or -NR¹⁻¹R¹⁻²; wherein the C₁-C₆ alkyl is optionally and independently substituted by 1, 2, 3, or 4 R^{a}; R¹⁻¹ and R¹⁻² together with the atom to which they are attached form a 3- to 7-membered heterocycloalkyl group;
each R^{a} is independently hydroxyl;
R² and R³ are independently hydrogen;
L is 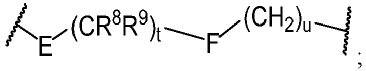 t is 0;
u is 1;
E is carbonyl;
F is a chemical bond;
B is 4- to 6-membered heterocycloalkyl; wherein the 4- to 6-membered heterocycloalkyl is optionally and independently substituted by 1, 2, or 3 Rⁱ;
each Rⁱ is independently hydrogen, hydroxyl, or C₁-C₃ alkyl;
D is 5- to 12-membered heteroaryl; wherein the 5- to 12-membered heteroaryl is optionally and independently substituted by 1, 2, or 3 R^{j};
each R^{j} is independently H, halogen, C₁-C₃ alkyl, OR¹²⁻³, or SR¹²⁻⁴; wherein the C₁-C₃ alkyl is optionally and independently substituted by 1, 2, or 3 R^{k};
R¹²⁻³ and R¹²⁻⁴ are independently C₁-C₃ alkyl; wherein the C₁-C₃ alkyl is optionally and independently substituted by 1, 2, or 3 R^{k-1};
each R^{k} is independently halogen;
each R^{k-1} is independently halogen;
the heteroatom in the 3- to 7-membered heterocycloalkyl, 4- to 6-membered heterocycloalkyl, and 5- to 12-membered heteroaryl is one or more kinds of N, O, and S, and the number of heteroatoms is 1 to 3; 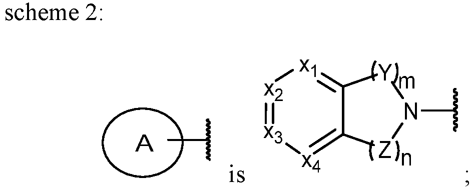
wherein
m is 1;
n is 1;
X₂ and X₃ are N;
X₁ and X₄ are independently -CR¹-;
Y and Z are independently -(CR²R³)ᵣ-; r is 1;
each R¹ is independently C₁-C₆ alkyl, 3- to 7-membered cycloalkyl, or -NR¹⁻¹R¹⁻²; wherein the C₁-C₆ alkyl is optionally and independently substituted by 1, 2, 3, or 4 R^{a}; R¹⁻¹ and R¹⁻² together with the atom to which they are attached form a 3- to 7-membered heterocycloalkyl group; the heteroatom in the 3- to 7-membered heterocycloalkyl group is selected from one, two, or three kinds of N, O, and S, and the number of heteroatoms is 1, 2, or 3;
each R^{a} is independently hydroxyl;
R² and R³ are independently hydrogen;
L is 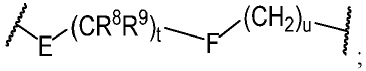
t is 0;
u is 1;
E is carbonyl;
F is a chemical bond;
B is 4- to 6-membered heterocycloalkyl; wherein the 4- to 6-membered heterocycloalkyl is optionally and independently substituted by 1, 2, or 3 Rⁱ; the heteroatom in the 4- to 6-membered heterocycloalkyl is selected from one, two, or three kinds of N, O, and S, and the number of heteroatoms is 1, 2, or 3;
each Rⁱ is independently hydrogen, hydroxyl, or C₁-C₃ alkyl;
D is 5- to 6-membered heteroaryl; wherein the 5- to 6-membered heteroaryl is optionally and independently substituted by 1, 2, or 3 R^{j}; the heteroatom in the 5- to 6-membered heteroaryl is selected from one, two, or three kinds of N, O, and S, and the number of heteroatoms is 1, 2, or 3;
each R^{j} is independently H, halogen, C₁-C₃ alkyl, OR¹²⁻³, or SR¹²⁻⁴; wherein the C₁-C₃ alkyl is optionally and independently substituted by 1, 2, or 3 R^{k};
R¹²⁻³ and R¹²⁻⁴ are independently C₁-C₃ alkyl; wherein the C₁-C₃ alkyl is optionally and independently substituted by 1, 2, or 3 R^{k-1};
each R^{k} is independently halogen;
each R^{k-1} is independently halogen.

8. The compound of formula I or the pharmaceutically acceptable salt thereof according to claim 1, which satisfies one or more of the following conditions:
(1) each R¹ is independently methyl, 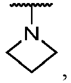 ethyl, 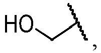 or 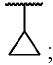 preferably, each R¹ is independently methyl, 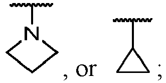
(2) 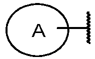 is 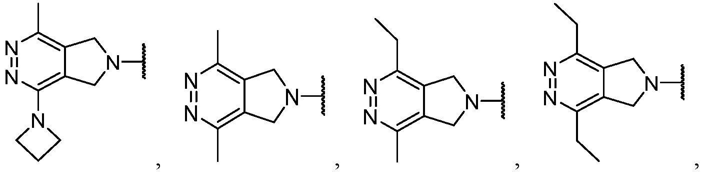 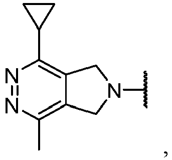 or 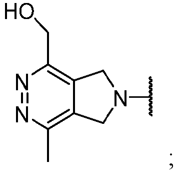 for example, 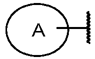 is 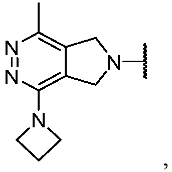 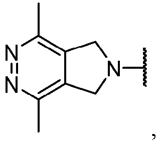 or 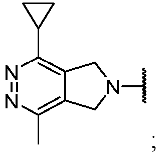
(3) L is 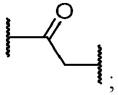 preferably, L is 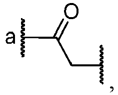 wherein 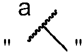 represents the site connected to A;
(4) B is 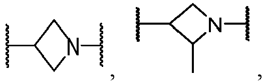 or 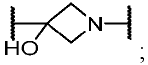 preferably, B is 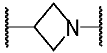 or 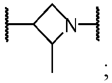
(5) D is 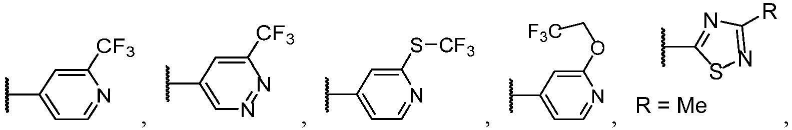 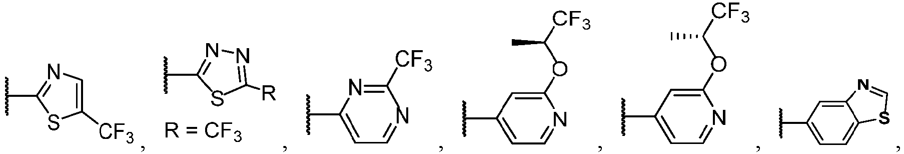 or 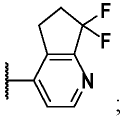 for example, D is 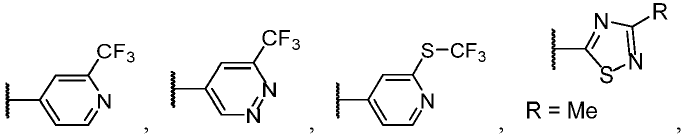 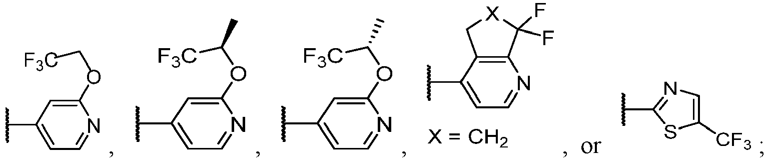 preferably, D is

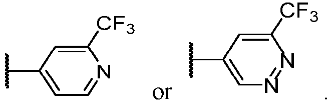

9. The compound of formula I or the pharmaceutically acceptable salt thereof according to claim 1, which is any one of the following compounds:

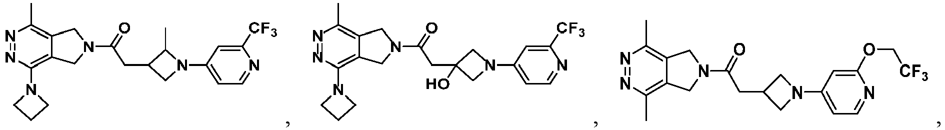

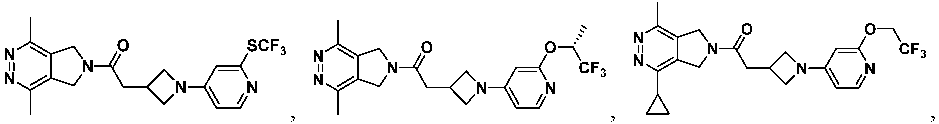

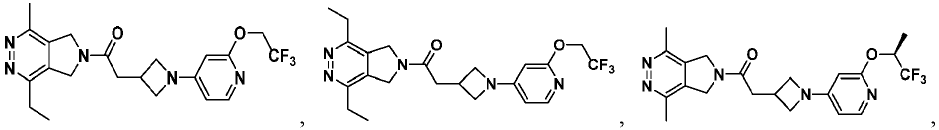

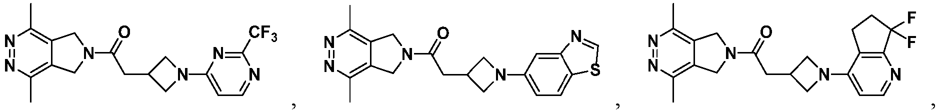

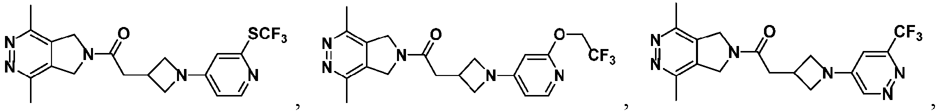

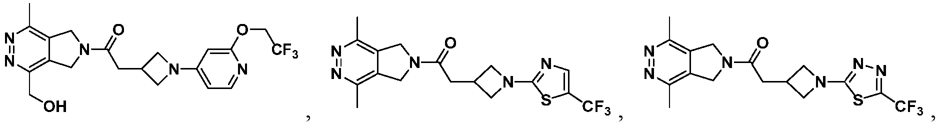

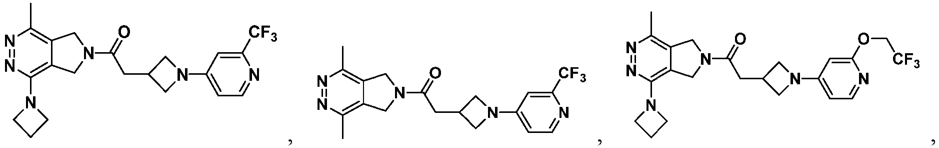

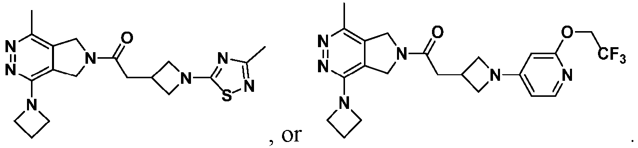

10. The present disclosure provides a preparation method for the compound of formula I or the pharmaceutically acceptable salt thereof according to any one of claims 1 to 9, wherein the preparation method is any one of the following schemes:
scheme (a): in an organic solvent, in the presence of a catalyst, performing a condensation reaction between a compound of formula II and a compound of formula III as follows to obtain the compound of formula I; 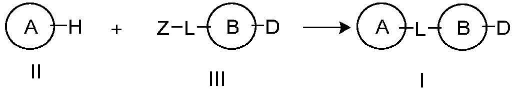
scheme (D): in an organic solvent, in the presence of a catalyst, performing a cyclization reaction between a compound of formula VIII and a compound of formula VIIII as follows to obtain the compound of formula I; 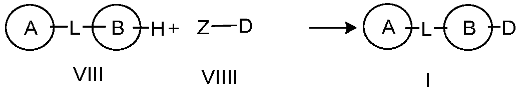
wherein Z is halogen, TsO-, hydroxyl, methoxy, ethoxy, n-propoxy, or isopropoxy; the halogen is, for example, chlorine or bromine; preferably, Z is hydroxyl, methoxy, ethoxy, n-propoxy, or isopropoxy;
A, L, B, and D are as described in any one of claims 1 to 9.

11. A pharmaceutical composition comprising a therapeutically effective amount of substance A and a pharmaceutical excipient; the substance A is the compound of formula I or the pharmaceutically acceptable salt thereof according to any one of claims 1 to 9.

12. A use of substance A in the manufacture of a positive allosteric modulator of a muscarinic receptor; the substance A is the compound of formula I or the pharmaceutically acceptable salt thereof according to any one of claims 1 to 9, or the pharmaceutical composition according to claim 11.

13. A use of substance A in the manufacture of a medicament for treating and/or preventing a muscarinic receptor-mediated disease; the substance A is the compound of formula I or the pharmaceutically acceptable salt thereof according to any one of claims 1 to 9, or the pharmaceutical composition according to claim 11; preferably, the disease is Parkinson's disease, Alzheimer's disease, Huntington's disease, schizophrenia, or drug addiction.
